(19)

Europäisches
Patentamt
European
Patent Office
Office européen
des brevets

(11) **EP 3 875 478 A1**

(12) **EUROPEAN PATENT APPLICATION**

(43) Date of publication:
**08.09.2021 Bulletin 2021/36**

(21) Application number: **20382160.8**

(22) Date of filing: **05.03.2020**

(51) Int Cl.:
***C07K 14/725*** (2006.01)          ***C07K 14/705*** (2006.01)
***C07K 14/735*** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Canvax Biotech, S.L.**
**14014 Córdoba (ES)**

(72) Inventors:
• **Paz-Rojas, Elier**
  **14004 Córdoba (ES)**

• **Garcia-Maceira, Fe Isabel**
  **14004 Córdoba (ES)**
• **Luna-Guerrero, Verónica**
  **14013 Córdoba (ES)**
• **Montero-Peñalvo, Gracia**
  **14004 Córdoba (ES)**
• **Garcia-Maceira, Tania**
  **14004 Córdoba (ES)**
• **Gomez-Melero, Sara**
  **11407 Jerez de la Frontera (ES)**

(74) Representative: **Carlos Hernando, Borja**
**Garrigues IP, S.L.P.**
**Hermosilla, 3**
**28001 Madrid (ES)**

(54) **NOVEL NON-IMMUNOGENIC CHIMERIC ANTIGEN RECEPTORS AND USES THEREOF**

(57)     The present invention is related to novel artificial T cell activating receptor known as chimeric antigen receptors (CARs) based on mutants of human high affinity Fc gamma receptor alpha chain (CD64) with 7 to about 25 fold reduced affinity for human IgG1, relative to affinity of wild type CD64 for human IgG1, linked to monomeric forms of intracellular stimulatory and co-stimulatory domains and where the ligand that binds the target antigen is a fusion protein between a target recognition domain (TRD) and a Fc gamma 1 or an equivalent Fc gamma. Such CARs may be useful for treating various diseases such as cancer, infectious diseases and autoimmune diseases among others.

**EP 3 875 478 A1**

## Description

### Field of the invention

The present invention is related to novel artificial T cell activating receptor known as chimeric antigen receptors (CARs) based on mutants of human high affinity Fc gamma receptor alpha chain (CD64) with 7 to about 25 fold reduced affinity for human IgG1, relative to affinity of wild type CD64 for human IgG1, linked to monomeric forms of intracellular stimulatory and co-stimulatory domains and where the ligand that binds the target antigen is a fusion protein between a target recognition domain (TRD) and a Fc gamma 1 or an equivalent Fc gamma. Such CARs may be useful for treating various diseases such as cancer, infectious diseases and autoimmune diseases among others.

### Background of the invention

Generation of a protective immune response against diseases like cancer and infections has been a major goal of immunology. Genetic modification of T lymphocytes and Natural Killers cells (NK) is one of the strategies to generate rapid and potent immune responses. For example, T lymphocytes may be genetically modified with a different T Cell Receptor (TCR) or with a chimeric receptor that both binds a target antigen through the extracellular region and also has intracellular domains for activation of the T lymphocyte and cytotoxicity of the target cell. Such chimeric receptors are collectively known as Chimeric Antigen Receptors or CARs. The first CAR that used single chain variable fragment derived from the antigen recognition domain of antibodies was described by Eshhar and collaborators in 1993 (Eshhar Z *et al,* 1993). This first generation of CARs used CD3zeta only as intracellular activation domain and although they were efficient in target cell cytotoxicity assays, they produced low amounts of gamma-interferon and T cell expansion mediated by antigen exposure was very limited and thus this first CAR generation had a low in vivo effect (Brocker & Karjalainen, 1995; Brocker, 2000).

The addition of one co-stimulatory domain such as the intracellular region of CD28, 4-1BB, ICOS, OX-40 or DAP10 has led to development of second-generation CARs where one of such domains is included along with CD3z stimulatory domain, whereas addition of two co-stimulatory domains, for example CD28 and 4-1BB, together with CD3z stimulatory domain is a third generation CAR. Clinical outcome in patients treated with second generation CARs has been impressive with over 70% initial complete responses in patients with refractory hematological tumors.

However, current CARs still need to be improved for both efficacy and toxicity. Efficacy should be improved because it is known that in vivo persistence is low in a significant proportion of patients but also it has been observed that tumor escape variants rapidly evolve making therapy ineffective. For example, dual tandem CARs targeting two different target proteins at the surface of tumor has been proposed to address the loss of one target antigen. Another potential solution is the use of a universal or switch CAR where T cell is modified with an invariant surface CAR where one or several ligands bind and such ligands also bind to a target protein within tumor cell surface. The first two attempts to develop universal CARs were made in 1994 by Smyth MJ and coworkers and by Kershaw MH and coworkers in 1996 (Smyth *et al,* 1994; Kershaw *et al,* 1996) using human CD64 or human FcERIa to modify cytotoxic T lymphocytes, respectively. Both CARs were of first generation and used the natural coupling of both CD64 or FcERIa to common gamma chain, a polypeptide that includes one ITAM in the intracellular region and triggers degranulation of lysosomes and cytotoxicity of T lymphocytes. Third attempt to develop a high affinity Fc receptor-based CAR was made in 2006 by Teng MWL and coworkers (Teng *et al,* 2006) and this was the first second generation CAR that included a high affinity Fc receptor along with intracellular CD28 and CD3z.

In 2006 Clemenceau *et al* (Clemenceau *et al,* 2006) developed another universal first generation-based CAR using CD16, one of the low affinity receptors for immunoglobulins. This CAR comprised CD16V158 fused to the full FcERI gamma including the two extracellular amino acids, the transmembrane and the full intracellular domain and thus by design it was a dimeric protein that was lentivirally transduced into T lymphocytes, but they did not assay, using for example Western blot, if the real protein was a dimer as predicted. Specific lysis was observed in vitro using rituximab, an antibody used as anti-CD20 drug, against a CD20 positive B-lymphoblastoid cell line. They used the V158 variant of CD16 as this has about 1,8 times more affinity for IgG and in particular it is known that patients treated with rituximab that carry this variant have a higher response to therapy (Cartron *et al,* 2002).

Previously it was demonstrated that two signals were needed at the same time for T lymphocyte expansion, that is, for example CD28 and CD3z either in parallel or in series were needed to support expansion and to secrete high amounts of cytokines (Alvarez-Vallina & Hawkins, 1996; Finney *et al,* 1998; Hombach *et al,* 2001). Also, in 2001 it was demonstrated that CD3z produced a more potent stimulation signal than FcERI gamma chain (Haynes *et al,* 2001) although this conclusion was made using a solid tumor as model and hematological tumors and solid tumor may behave differently as for example in solid tumors there is an immunosuppressive environment and probably more potent CARs are needed when compared with hematological ones.

European Patent EP3057986 granted to Campana D and Kudo K (Campana & Kudo, 2017), describes a second-generation CAR based on CD16V158 fused to the transmembrane of CD8 alpha, 4-1BB and CD3z that is an obvious evolution to a second-generation CAR of the first generation CD16V158-based CAR-T initially developed by Clemenceau (Clemenceau *et al*, 2006). Previous work of Campana group at St Jude Children's Research Hospital (Imai *et al,* 2004) had demonstrated that addition of 4-1BB to CD3z was superior than CD3z alone, so patent EP3057986B1 described such combination, that is they have made CD16V158-hingeCD8a-TMCD8a-4-1BB-CD3z. This system works and have been licensed to Unum Therapeutics where it is currently on clinical trials as ACTR087. But this system does not teach if it is possible to develop a CAR based on other Fc receptors, in particular for example if a CD64 with an affinity for IgG1 higher than that of CD16V158 is still useful or if is inhibited by human serum, the percentage of inhibition and the suitability to make a CAR. In addition, they have not optimized the design based on scFv to CD16 as they have designed a dimeric CAR using the dimeric hinge of CD8a but when they have tested in Western blot it was found that probably over 70 percent of CAR molecules are monomeric (see EP3057986B1 Figure 1C at page 45).

WO20171613333 filed by Unum Therapeutics describes that all Fc receptors share common properties and thus their findings related only to CD16 are extrapolated to all Fc receptors. For example, at page 23 they state "In some examples, the mutated ligand-binding domain of an Fc receptor can be derived from FcERI, FcERI/CD23, FcaRI/CD89, $Fc\alpha\mu R$ or FcRn. In some examples, the mutated extracellular ligand-binding domain of an Fc receptor can be derived from CD16A, CD16B, CD32A, CD32B, CD32C, CD64A, CD64B, CD64C, or a naturally-occurring polymorphism variant thereof as described herein (e.g. CD16A V158, CD16A F158, or any other example of a naturally occurring polymorphism presented in Table 1. In some examples, the mutated ligand-binding domain of an Fc receptor is derived from CD16, such as CD16A, and comprises one or more mutations relative to the amino acid sequence of its wild-type counterpart" whereas they claim "a chimeric receptor wherein the Fc gamma receptor is selected from a group consisting of CD16A, CD16B, CD64A, CD64B, CD64C, CD32A and CD32B and one or more mutations located in the D2 domain", that is, all the mutations previously known in state of art are claimed as belonging to this invention but without a classification of mutations for selection of those useful and those that are not useful for CAR development, that is, they do not teach specific examples outside CD16 that demonstrate suitability of other Fc receptors for CAR development as state of art described previously both a CD64-based CAR and a second generation FcERI-based CAR and their problems. Importantly, work of Kudo and Campana and further research at Unum Therapeutics does not teach if receptors with higher affinity for human IgG1 than that of CD16V158 are useful for the development of chimeric antigen receptors. This is highly relevant for the present invention because CD16V158 has a dissociation constant (Kd) for IgG1 of about 350nM while the Kd of native CD64 for IgG1 is about 10nM, that is, CD64 has about 35-fold higher affinity for IgG1 than the CD16V158 receptor variant that has the highest affinity among variants of CD16. Affinity is also relevant because it is known that patients with CD16V158 variant have a better response to Rituximab antibody than patients with CD16F158 but affinity difference between those two variants is about 1,8-fold, that is, very low differences in affinity of CD16 have a significative impact in clinical outcome of patients. But previous research does not teach if a further improvement of affinity will be useful or not because state of art teaches against using CD64 to develop CARs. That is, current state of art does not teach about the suitability of mutants of CD64 with a Kd between that of native CD64 and that of CD16V158 to develop chimeric antigen receptors.

In addition, current state of art describes a significative role of the hinge for CAR activity and toxicity (Guest *et al,* 2005; Qin *et al,* 2017). In another example, Ying Z et al (Ying *et al,* 2019) describe an anti-CD19 scFv-based CAR where the addition of just 15 amino acids in the hinge eliminates CRS and neurotoxicities of grades over than 1.

One important property of current CARs based on scFv is that most of them are dimeric by design. For example, US 7,446,190 describes a scFv-based CAR wherein the hinge, transmembrane and intracellular domain where derived from human CD28 and fused to human CD3z and in this case the hinge comprises the cysteine at position 141 according to Uniprot P10747 that homodimerizes CD28. This is one of the main differences between this invention and previous work of Finney HM *et al* (see Figure 1E of Finney *et al*, 1998) wherein also hinge, transmembrane and intracellular domain of human CD28 is fused to CD3z but in the work of Finney a *BamH*I restriction enzyme site is introduced for DNA cloning between hinge and transmembrane and thus as described, using the oligo T4058, four additional amino acids (L, D, P and K) are included between the Cysteine at 141 and the transmembrane region and thus it is lost the optimal orientation between the Cysteine at 141. A CAR based on FMC63 anti-CD19 clone that uses a hinge of human CD8 alpha of 45 amino acids and also the transmembrane of human CD8 alpha is also partially dimeric (see Figure S6B of Rodgers, 2016). In addition, those researchers have also demonstrated that a 12 amino acid long hinge based on modified IgG4 hinge with Serine at 228 changed to Proline for interchain disulfide bond stabilization is also partially dimeric and such dimeric CAR is slightly more active than monomeric one for maximum cell lysis and has a lower EC50 than the monomeric one, albeit interleukin-2 secretion was not different between the dimer and the monomer. But this state of art does not teach if those commonly used hinges and transmembrane domains may be useful for the development of CD64-based CARs.

Thus, it seems an oversimplification to assume that all Fc receptor behave in the same way when for example: (i) CD64 has a third domain called D3 that is not present in CD16 nor in FcERI alpha and although this D3 domain is positioned about 30 Angstroms away from the Fc interface and thus D3 domain of CD64 does not make contact directly with Fc and in fact a truncated soluble CD64 without D3 domain has high affinity for IgG with about 3 fold reduction in affinity (Asaoka *et al,* 2013) but domain D3 is important for making the receptor protruding more into the extracellular space than its two-domain Fc receptor counterparts and, thus, more available for binding; (ii) CD23 is a type II protein and there is no known CAR based on CD23 protein that have the amino terminal end inside of cells and the carboxy terminus outside of cells; (iii) IgE has an additional domain that is not present on IgG; (iv) proposed model for mode of binding of IgG1 to CD64 is different from that of CD16 because binding to CD16 seems to put variable regions of antibody away from membrane while binding to CD64 leave variable regions closer to the membrane of the immune cell (see for example, Kiyoshi et al, Nature 2015); (v) CD16 and CD64 share some IgG isotypes like human IgG1, human IgG3 and human IgG4 that bind to both receptors but there are isotypes like human IgG2 that bind to CD16 but not to CD64; (vi) each Fc receptor must be considered different because there are differences in affinities, in serum inhibition, in structure, in expression levels, in mode of binding, in types of proteins (ie class I or class II), in number of immunoglobulins that bind to each receptor and in amount of serum IgGs that may inhibit each receptor; (vii) current state of art does not teach if it is possible to develop a CD64-based CAR, in particular, if mutants of CD64 with higher affinity for IgG1 than that of the CD16F158 and thus with unknown response to potential serum inhibition that may compromise their utility for the development of CD64-based CAR. That is, the knowledge that all Fc receptors bind to immunoglobulins is not enough to assume that they behave in the same way for the development of a new kind of receptors like CARs where affinity, inhibition of activity by serum, structure of the receptor, expression levels in lymphocytes after transduction and binding mode must be tested experimentally to provide specific examples of suitability of each receptor for the development of novel CAR.

In the present invention we demonstrate for the first time that: (1) A CAR comprising the extracellular domain of CD64 and the transmembrane of CD8 alpha without hinge results in a better expression on the surface of primary T lymphocytes and more homogeneous expression across different patients than either a CAR containing a hinge of 14 amino acids of CD8 alpha and the same transmembrane of CD8 alpha or a CAR containing a hinge of 39 amino acids and the transmembrane domain of human CD28, the two most common hinge and transmembrane domains used today in CAR development; (2) CARs based on CD64 have better surface expression than equivalent CARs based on CD16; (3) unexpectedly cytotoxicity of native CD64-based CARs is not inhibited by serum using either protein targets like CD20 or protein targets like CD22 with a much lower density on the surface of tumoral cells than CD20; (4) cytokine secretion in short term coculture between T lymphocytes transduced with a CD64-based CAR and a target cell in the presence of both, a specific IgG1 antibody against CD20 at 5 micrograms per milliliter and 20 percent of human serum is inhibited up to a 85 percent for a native CD64 and proliferation is reduced about a 90 percent in the presence of human serum at 20 percent when compared with proliferation using fetal bovine serum and those results confirm that native CD64 is not useful alone to the development of CARs; (5) for either some already known CD64 mutations and for novel mutations of CD64 with low immunogenicity described for the first time in the present invention the cytokine secretion in short term coculture between T lymphocytes transduced with such mutated CD64-based CAR and a target cell in the presence of both, a specific IgG1 antibody against CD20 at 5 micrograms per milliliter and 20 percent of human serum is inhibited in a range of 60 to 25 percent for mutants of CD64 with a Kd for human IgG1 (huIgG1) between 70nM to about 180nM, that is, mutants of CD64 with affinities for huIgG1 about 5 to 1,4 fold higher than that of CD16V158 and thus, we demonstrate for the first time that reducing the affinity of CD64 for serum IgGs, to levels outside those demonstrated for CD16-based CARs, relieve serum inhibition and allows the development of CD64-based CARs; (6) CARs based on the above mutants of CD64 using a monomeric transmembrane of CD8 alpha without hinge have a higher proliferation rate if CD28 co-stimulatory domain is used instead of those with only 4-1BB co-stimulatory domain in the presence of 20 percent of human serum and thus CARs containing at least CD28 and CD3z are preferred in the methods of the present invention; (7) preferred CARs based on the above mutants of CD64 are disclosed and are based on mutations that generate non-immunogenic proteins, that is proteins that do not contain strong binding peptides for HLA I, that allow multiple infusions of engineered immune cells that express such chimeric antigen receptors.

**Brief description of the invention**

A chimeric antigen receptor (CAR) is a receptor to be expressed on the surface of immune cells, normally T lymphocytes or NK cells that comprises an extracellular domain with affinity for a target antigen expressed on the surface of a target cell, a linker and transmembrane region to anchor the protein to the surface of the immune cell and an intracellular region to transduce a signal inside the immune cell. Such signal comprises both, (a) the release of cytotoxic proteins like perforin and granzymes and (b) signaling for cytokine release and proliferation of immune cells. Intracellular domains of CARs normally comprise the intracellular region of CD3zeta fused to intracellular regions of proteins like CD28, 4-1BB or equivalents. To introduce a CAR inside immune cells several methods may be used like transduction by retroviruses or lentiviruses, transposon-mediated transformation or electroporation, being transduction the most widely used method to introduce a CAR into immune cells.

The present invention is based on the findings that, contrary to current state of art, it is possible to develop a CAR based on CD64. The authors of the present invention have found that: (a) CD64-based CARs have higher surface expression in human T lymphocytes when CD8 alpha transmembrane without hinge is used when compared with either CD8 alpha with a hinge or CD28 with hinge and such CAR has a higher surface expression than CD16-based CARs; (b) cytotoxicity is not useful as a method to discriminate between CARs with different serum inhibition levels while cytokine secretion and proliferation in the presence of human serum is useful to discriminate between those CARs; (c) mutants of CD64 with affinities for IgG1 between about 70nM to about 180nM have a serum inhibition level compatible with their potential use for the development of CD64-based CARs and CD28 intracellular co-stimulation domains outperforms 4-1BB-based CARs in cytokine secretion and proliferation level and thus, CARs containing at least CD28 and CD3z stimulation domains are preferred over CARs containing only 4-1BB and CD3z stimulation domains; (d) preferred mutants of CD64 with low inhibition by unrelated serum IgG are disclosed and are based on mutations that generate non-immunogenic proteins that allow multiple infusions of engineered immune cells that express such chimeric antigen receptors.

In a first embodiment the present invention is directed to a chimeric antigen receptor (CAR) that comprises: (a) the extracellular domain of a mutated high affinity Fc gamma receptor (mutant CD64); (b) a transmembrane domain; and (c) an intracellular signal transduction domain, wherein the mutated extracellular domain of the high affinity Fc gamma receptor of (a) has reduced affinity of from about 7 fold to about 25 fold for native Fc of human IgG1 relative to native extracellular domain of the high affinity Fc gamma receptor and in a cytokine secretion assay the interferon-gamma secretion of the coculture is at least 40% of interferon-gamma secretion relative to the same co-culture when the 10% of fetal bovine serum is substituted by 20% of human serum wherein such coculture is of about 24 h and comprises: (1) a lymphocyte that express a chimeric antigen receptor (CAR) that uses such mutated extracellular domain of the high affinity Fc gamma receptor; (2) a target cell that expresses on the cell surface an antigen specifically recognized by an antibody; (3) a specific antibody for the target antigen of (2) used in the coculture at a concentration up to 5 micrograms per milliliter and (4) human serum at a final concentration in the coculture of 20% in volume percent used as an inhibitor of the reaction of the lymphocyte of (1) with a target cell of (2) using an antibody of (3).

In a second embodiment the present invention is directed to a chimeric antigen receptor (CAR) according to the previous paragraph wherein the mutated extracellular domain of the high affinity Fc gamma receptor (CD64) comprises at least a mutation in the FG loop of the receptor that is located between Glycine at position 164 to Valine at position 183 according to CD64 described in SEQ. ID. NO. 1.

In a third embodiment the present invention is directed to a chimeric antigen receptor (CAR) according to second embodiment wherein the mutation of CD64 at FG loop is made by substitutions of amino acids at positions between Cysteine at 168 to Glycine at 180 and/or by an insertion of an amino acid between Cysteine at 168 and Lysine at 173.

In a fourth embodiment the present invention is directed to a chimeric antigen receptor (CAR) according to third embodiment wherein the mutated CD64 consist of CD64 mutated at FG loop selected from the amino acid sequences of SEQ. ID. NO. 2 to SEQ. ID. NO. 10.

In a fifth embodiment the present invention is directed to a chimeric antigen receptor (CAR) according to first embodiment wherein the mutated extracellular domain of the high affinity Fc gamma receptor (CD64) comprises at least a mutation in one of the amino acids that form part of the hydrophobic pocket that interacts with Leucine 235 of human IgG1.

In a sixth embodiment the present invention is directed to a chimeric antigen receptor (CAR) according to fifth embodiment wherein the extracellular domain of the high affinity Fc gamma receptor (CD64) mutated in at least one amino acid that form part of the hydrophobic pocket that interacts with Leucine 235 of human IgG1 includes at least a mutation of amino acids that are selected from a group consisting of W104, L105, A126, W127, K130, V132 and Y176.

In a seventh embodiment the present invention is directed to a chimeric antigen receptor (CAR) according to sixth embodiment wherein the mutated amino acid in the hydrophobic pocket of CD64 is K130.

In an eighth embodiment the present invention is directed to a chimeric antigen receptor (CAR) according to seventh embodiment wherein mutated K130 amino acid in the hydrophobic pocket of CD64 is substituted with either an amino acid with a short lateral group selected from Serine, Glycine or Alanine with SEQ. ID. NO. 14 to SEQ. ID. NO. 16 or preferentially, with a bulky amino acid selected from Y, F or W with SEQ. ID. NO. 11 to SEQ. ID. NO. 13.

In a ninth embodiment the present invention is directed to a chimeric antigen receptor (CAR) according to first to eighth embodiments wherein the Fc fusion protein that binds to the CAR is selected from either a human IgG1 or a human IgG4 that preferentially has a mutation at F234L for improved binding to human CD64 and those IgG recognize a target antigen on the surface of a target cell.

In a tenth embodiment the present invention is directed to a chimeric antigen receptor (CAR) according to first to eighth embodiments wherein the Fc fusion protein is selected from human Fc gamma 1 or human Fc gamma 4 mutated at F234L and the target recognition domain is selected from a group consisting of a scFv, an affibody and ankyrin, a fynomer, a nanobody, a ligand to a surface receptor or a scTCR.

In an eleventh embodiment the present invention is directed to a chimeric antigen receptor (CAR) according to embodiments first to eighth wherein such CAR has no hinge that joins the mutated CD64 alpha chain to the transmembrane and intracellular stimulation domain and such transmembrane domain is selected from the group consisting of human CD28, human CD32 or human CD8 alpha.

In a twelfth embodiment the present invention is directed to a chimeric antigen receptor (CAR) according to embodiment eleven wherein preferred monomeric transmembrane domain is selected from human CD8 alpha with either SEQ. ID. NO. 17 or SEQ. ID. NO. 18.

In a thirteenth embodiment the present invention is directed to a chimeric antigen receptor (CAR) according to eleventh and twelfth embodiments wherein such CAR has an intracellular co-stimulation domain selected from a group consisting of CD28, 4-1BB, OX-40, DAP10 or CD27.

In a fourteenth embodiment the present invention is directed to a chimeric antigen receptor (CAR) according to eleventh and twelfth embodiments wherein such CAR has a preferred intracellular co-stimulation domain derived from human CD28 with SEQ. ID. NO. 19.

In a fifteenth embodiment the present invention is directed to a chimeric antigen receptor (CAR) according to embodiments thirteenth or fourteenth wherein such CAR has an intracellular stimulation domain selected from a group consisting of: (a) CD28 intracellular co-stimulation domain fused to either the whole CD3z intracellular region with amino acid sequence SEQ. ID. NO. 20 or a truncated CD3z with amino acid sequence SEQ. ID. NO. 21; (b) CD28 intracellular co-stimulation domain fused to a second intracellular co-stimulation domain selected from 4-1BB, OX-40 or ICOS and to either the whole CD3z intracellular region or a truncated CD3z with two ITAMs as described in SEQ. ID. NO. 22 to SEQ. ID. NO. 27.

In a sixteenth embodiment the present invention is directed to a chimeric antigen receptor (CAR) wherein such CAR has the following structure CD64X-TMCD8-Z wherein: (a) CD64X is a human CD64 extracellular domain of SEQ. ID. NO. 1 that contains at least one mutation wherein such mutation is essentially non-immunogenic for HLA I, i.e. using NetMHC v4.0 in silico prediction tool peptides containing such mutations have only weak binding peptides as defined by peptides that are above 0,5 percent of rank for binding to each HLA I, such mutation is made by an insertion of a single amino acid between Cysteine at 168 and Lysine at 173 of SEQ. ID. NO. 1 and preferential amino acid insertion is selected from any of the following sequences: SEQ. ID. NO. 7 to SEQ. ID. NO. 10; (b) TMCD8 is the transmembrane domain derived from human CD8 alpha with either sequence SEQ. ID. NO 17 or sequence SEQ. ID. NO. 18; (c) Z is selected from: (i) Human CD28 intracellular co-stimulation domain fused with a linker preferentially of two Glycine amino acids (GG), used to reduce the HLA I immunogenicity of the joining peptide, to either full or truncated human CD3z intracellular stimulation domain as described in sequences SEQ. ID. NO. 20 and SEQ. ID. NO. 21; (ii) Human 4-1BB intracellular co-stimulation domain inserted between CD28 intracellular co-stimulation domain and CD3z intracellular stimulation domain as described in sequences SEQ. ID. NO. 22 and SEQ. ID. NO. 25; (iii) Human OX40 intracellular co-stimulation domain inserted between CD28 intracellular co-stimulation domain and CD3z intracellular stimulation domain as described in sequences SEQ. ID. NO. 23 and SEQ. ID. NO. 26 or (iv) Human ICOS intracellular co-stimulation domain inserted between CD28 intracellular co-stimulation domain and CD3z intracellular stimulation domain as described in sequences SEQ. ID. NO. 24 and SEQ. ID. NO. 27.

In a seventeenth embodiment the present invention is directed to the nucleic acid sequences encoding chimeric antigen receptors according to any preceding embodiments for engineering of an immune cell.

In a eighteenth embodiment the present invention is directed to vectors comprising any of the nucleic acid sequences of seventeenth embodiment wherein such vector is selected from the group consisting of a retroviral vector, a lentiviral vector or a transposon and this vector is used for transduction or transfection or electroporation of an immune cell selected from a group consisting of an autologous T lymphocyte, an allogenic T lymphocyte, an autologous primary NK cell, an allogenic primary NK cell or an allogenic NK cell line.

In a nineteenth embodiment the present invention is directed to an engineered immune cell that expresses at least one of the above chimeric antigen receptors of first to seventeenth embodiments for use in the treatment of a disease selected from a tumor, an infectious disease or an autoimmune disease wherein such treatment may comprise either a single infusion or multiple infusions of engineered immune cells that express the chimeric antigen receptors.

In a twentieth embodiment the present invention is directed to a pharmaceutical composition comprising an engineered immune cell according to the nineteenth embodiment and a pharmaceutically acceptable carrier or excipient.

In a twenty-first embodiment the present invention is directed to a chimeric antigen receptor (CAR) according to nineteenth embodiment wherein target antigen may be selected from a group that comprises, but is not limited to, for example BCMA, CAIX, CA125, CD5, CD6, CD19, CD20, CD21, CD22, CD23, CD27L, CD30, CD33, CD34 CD38, CD40, CD44, CD47, CD54, CD55, CD56, CD70, CD74, CD79a, CD79b, CD80, CD117, CD123, CD138, CD151, CD171, CD200, CEA, CEACAM5, cG250, CLL1, CS1, CTLA4, CXCR4, cytokeratin, EGFR, EpCAM, EphA2, EphB4, FOLR1, FRalpha, fibronectin, GD2, GD3, GPC3, GPRC5D, HER2, IGF-1R, IL13Ra2 (CD213a2), kappa light chain, Lewis Y, LIV, L1CAM, LGR5, LPAR, melanin, mesothelin, MUC1, MUC16, nectin, PSMA, TAG72 and 5T4 among others.

In a twenty-second embodiment the present invention is directed to a kit of parts that comprises: (a) an immune cell that expresses a chimeric antigen receptor according to any of first to sixteenth embodiments with an acceptable vehicle; (b) a composition comprising a Fc fusion protein or a human native IgG1 or a human IgG4 mutated at F234L which binds to the mutated CD64 of the CAR expressed on the surface of the immune cell of (a), together with an acceptable vehicle, and (c) instructions of use.

A still further object of the present invention is a method of treatment of a subject, including a human being, diagnosed with a tumor, an infectious disease or an autoimmune disease wherein such method of treatment may comprise treating said subject with a therapeutically effective quantity of engineered immune cells that express at least one of the above chimeric antigen receptors of the first to seventeenth embodiments and wherein such method comprises a single infusion or multiple infusions of said engineered immune cells.

**Brief description of the drawings**

The present invention is best understood with the following figures:

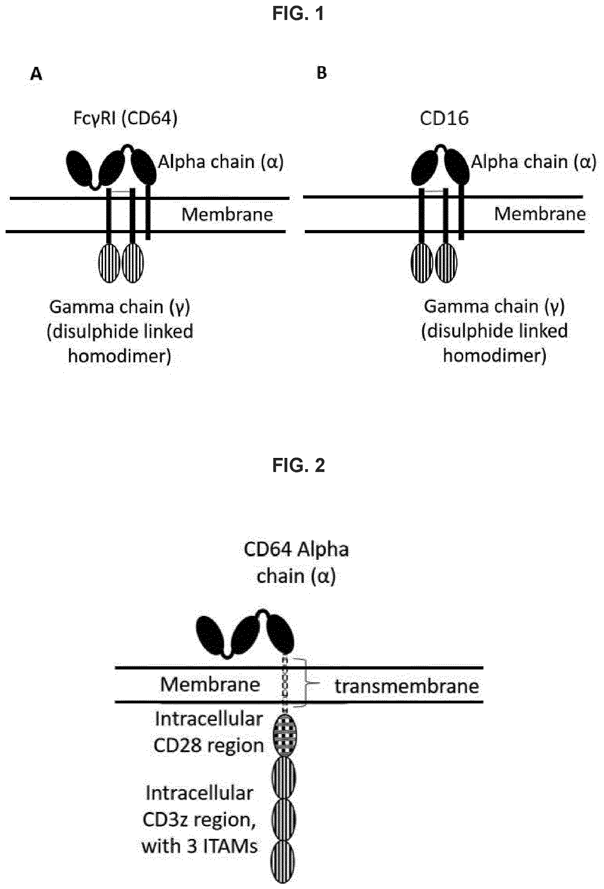

Figure 1: Structure of native human CD64 and CD16 receptors. Both receptors include a monomeric alpha chain that binds specifically to IgG1, IgG3 and IgG4 in CD64 and to IgG1, IgG2, IgG3 and IgG4 in CD16 and a shared invariant gamma chain that transduces signal inside the cell and contains an intracellular ITAM sequence per polypeptide for signal transduction. CD64 contains a third extracellular domain that is not present on CD16 nor on FcERI. Binding mode of IgG1 to CD64 is different than that of IgG1 to CD16 as Fab moiety is parallel with respect to membrane in IgG1 bound to CD64 while Fab is projected away membrane in IgG1 bound to CD16

Figure 2: Structure of a Universal CAR with extracellular region of CD64 and the intracellular region of a second generation based-CAR (2G-CAR) with CD28 and CD3z intracellular regions. As described in the present invention a monomeric CAR is preferred, that is, there is no hinge connecting CD64 extracellular region to transmembrane and thus, both extracellular region (CD64 alpha chain) and intracellular region (CD28-CD3z) are monomers.

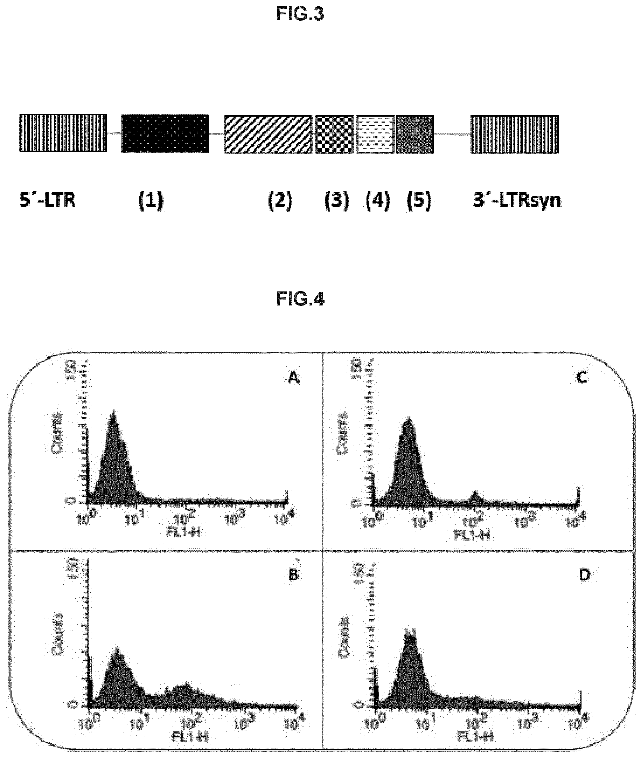

Figure 3: Structure of a lentiviral vector used for transduction of immune cells. (1) Promoter for CAR expression that may be selected from MCSV, EF1a, PGK or equivalent constitutive promoters or from TCRVB6.7 promoter of SEQ. ID. NO. 31; (2) Extracellular domain of CD64 CAR, a mutated form of SEQ. ID. No. 1; (3) Transmembrane region; (4) Intracellular co-stimulation domain selected from the intracellular region of 4-1BB, CD28, ICOS, OX-40, DAP10 or CD27 or combinations thereof; (5) Intracellular stimulation domain of either full or truncated CD3 zeta.

Figure 4: Comparison of surface expression on primary T lymphocytes of a CD64-based CAR-T with a CD16-based CAR having each TM of CD8 alpha and 4-1BB and CD3z intracellular domains. CAR based on CD64 has no hinge while CD16 has a 14 amino acids-long hinge. Non-transduced T lymphocytes were included as controls in (A) and (C). (B) is the histogram of a CAR with CD64 extracellular domain fused to the transmembrane of CD8 alpha with 4-1BB and CD3z intracellular domains and; (D) is the histogram of a CAR with CD16 extracellular domain fused to a 14 amino acids-long hinge of human CD8 alpha and transmembrane of CD8 alpha with 4-1BB and CD3z intracellular domains. The percentage of T lymphocytes that express the CD64-based CAR on their surface is 31,6 percent in (B) while the percentage of T lymphocytes that express the CD16-based CAR on their surface is 9,8 percent in (D) (see Table 1A).

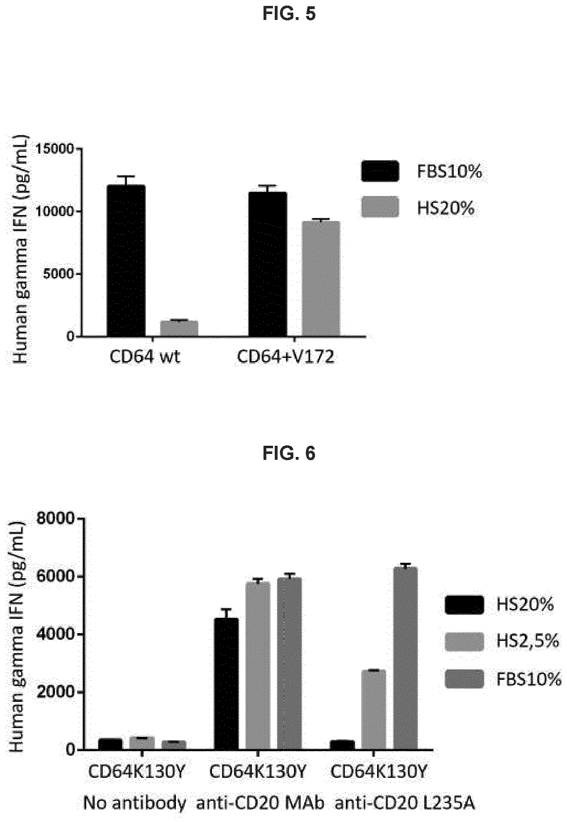

Figure 5: Cytokine secretion induced by a 24-hour coculture of either a native CD64-based CAR-T or a mutated CD64-based CAR-T (CD64+V172) with Raji cells in presence of either Fetal Bovine Serum at 10 percent (FBS10%) used as a non-inhibitory serum and Human Serum at 20 percent (HS20%) using an anti-CD20 antibody.

Figure 6: Cytokine secretion induced by a 24-hour coculture of a K130Y CD64-based CAR-T with Raji without antibody (left); a specific anti-CD20 antibody (center) and a specific anti-CD20 antibody mutated at Leucine 235 to Alanine (right). Fetal bovine serum at 10 percent (FBS10%) was used as a non-inhibitory serum and Human Serum at either 20 percent (HS20%) or 2,5 percent (HS2,5%) as inhibitory conditions.

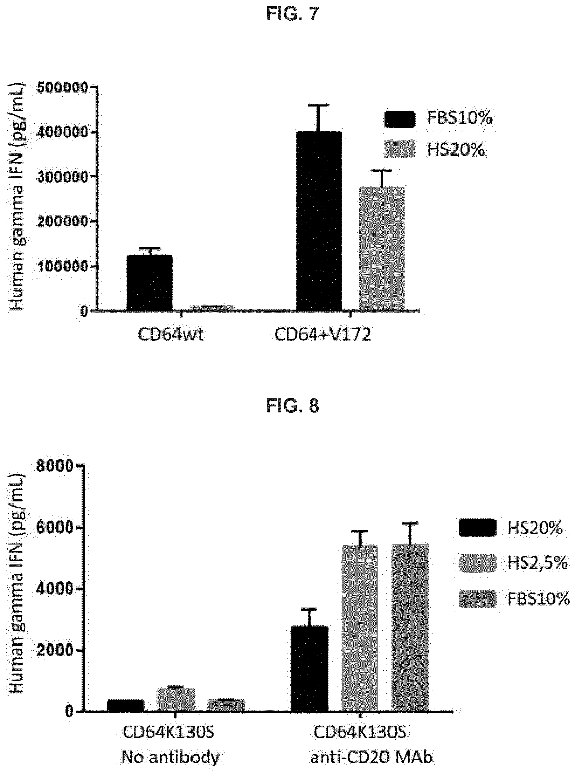

Figure 7: Cytokine secretion induced by a 72-hour coculture of either a native (wild type) CD64 or CD64+V172 based CAR-T with Raji as target cell and a specific anti-CD20 antibody in the presence of either FBS at 10 percent (FBS10%) or Human Serum at 20 percent (HS20%) used as inhibitor of binding of specific antibody to a CD64-based CAR-T.

Figure 8: Cytokine secretion induced by a 24-hour coculture of a K130S CD64-based CAR-T with Raji as target cell and: no antibody (left) and a specific anti-CD20 antibody (right). Fetal bovine serum at 10 percent (FBS10%) was used as a non-inhibitory serum and Human Serum at either 20 percent (HS20%) or 2,5 percent (HS2,5%) as inhibitory conditions.

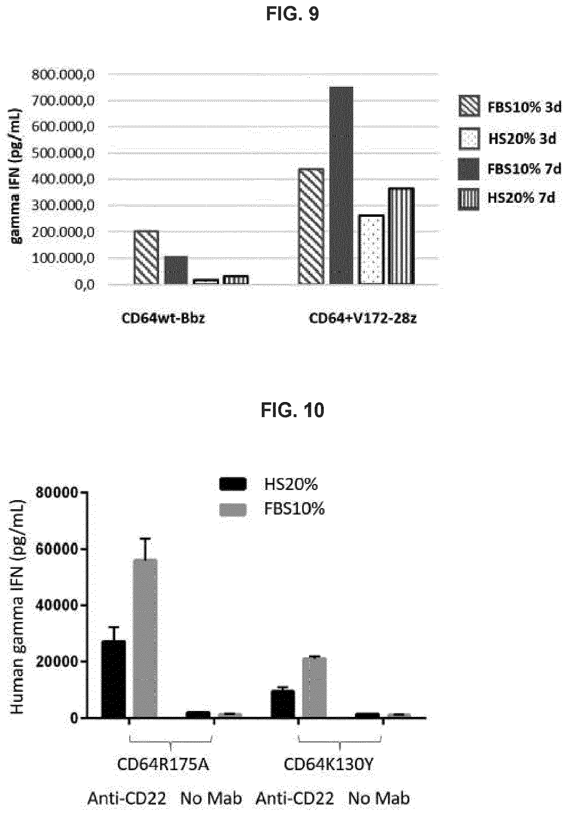

Figure 9: Cytokine secretion induced in a proliferation assay at 3 days and 7 days of coculture of either a wild-type (CD64wt-Bbz) or a mutated CD64-based (CD64+V172-28z) CAR-T, in the presence of Raji as target cell and using anti-CD20 antibody. Fetal bovine serum at 10 percent (FBS10%) was used as a non-inhibitory serum and Human Serum at 20 percent (HS20%) as inhibitor.

Figure 10: Cytokine secretion induced by a 24-hour coculture of either a K130Y or an R175A CD64-based CAR-T, lentiviral transduced into human primary T lymphocytes in the presence of Raji as target cell and: a specific anti-CD22 antibody (left) and no antibody (right). Fetal bovine serum at 10 percent (FBS10%) was used as a non-inhibitory serum and Human Serum at 20 percent (HS20%) as inhibitor.

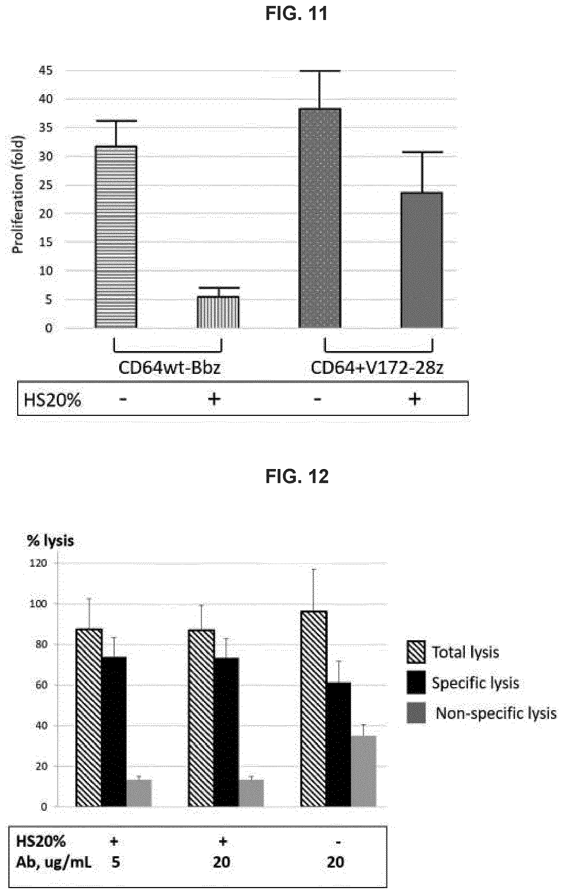

Figure 11: Proliferation assay of T lymphocytes after 7 days of coculture of either a wild-type (CD64wt-Bbz) or a mutated CD64-based (CD64+V172-28z) CAR-T in the presence of Raji as target cell and using anti-CD20 antibody. Fetal bovine serum at 10 percent (FBS10%) was used as a non-inhibitory serum and Human Serum at 20 percent (HS20%) as inhibitor.

Figure 12: Cytotoxicity in an assay of T lymphocytes after 24 hours of coculture of a wild-type CD64-based CAR-T, in the presence of Raji-FFluc as target cell and using anti-CD20 antibody. Fetal bovine serum at 10 percent (FBS10%) was used as a non-inhibitory serum and Human Serum at 20 percent (HS20%) as inhibitor.

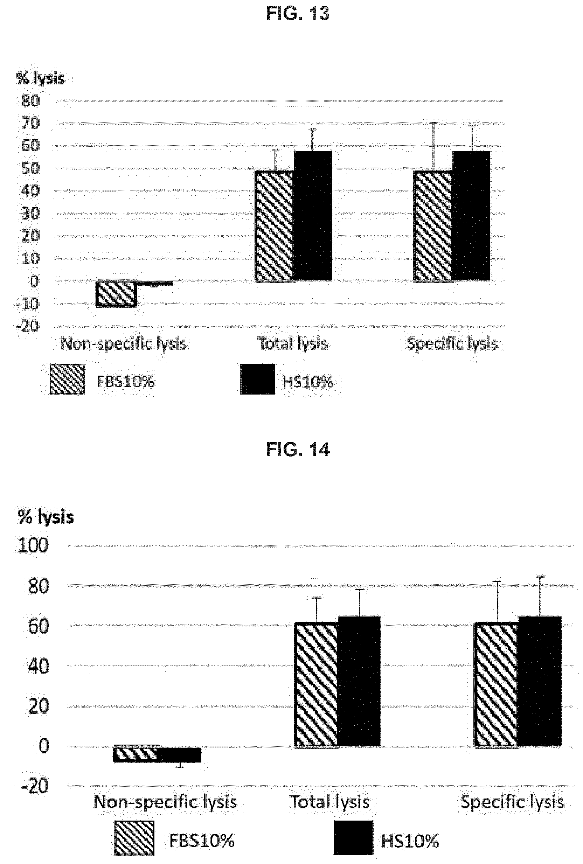

Figure 13: Cytotoxicity in an assay of T lymphocytes after 24 hours of coculture of a wild-type CD64-based CAR-T in the presence of Raji-FFluc as target cell and using anti-CD22 antibody. Fetal bovine serum at 10 percent (FBS10%) was used as a non-inhibitory serum and Human Serum at 20 percent (HS20%) as inhibitor.

Figure 14: Cytotoxicity in an assay of T lymphocytes after 24 hours of coculture of a R175A mutated CD64-based CAR-T, in the presence of Raji-FFluc as target cell and using anti-CD22 antibody. Fetal bovine serum at 10 percent (FBS10%) was used as a non-inhibitory serum and Human Serum at 10 percent (HS10%) as inhibitor.

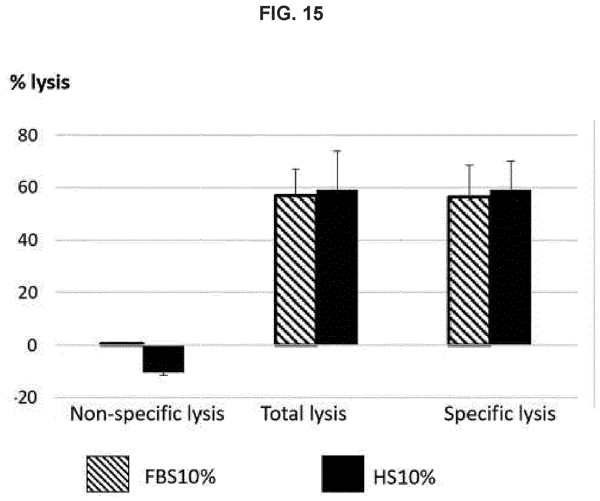

Figure 15: Cytotoxicity in an assay of T lymphocytes after 24 hours of coculture of a K130Y mutated CD64-based CAR-T, in the presence of Raji-FFluc as target cell and using anti-CD22 antibody at 1 microgram per milliliter. Fetal bovine serum at 10 percent (FBS10%) was used as a non-inhibitory serum and Human Serum at 10 percent (HS10%) as inhibitor.

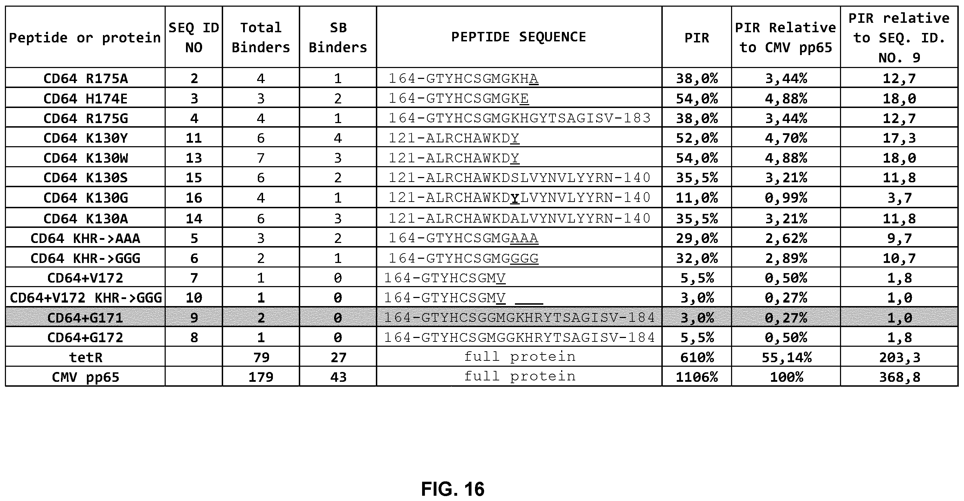

Figure 16: PIR values of CAR-Ts with preferred mutants of CD64 according to the present invention

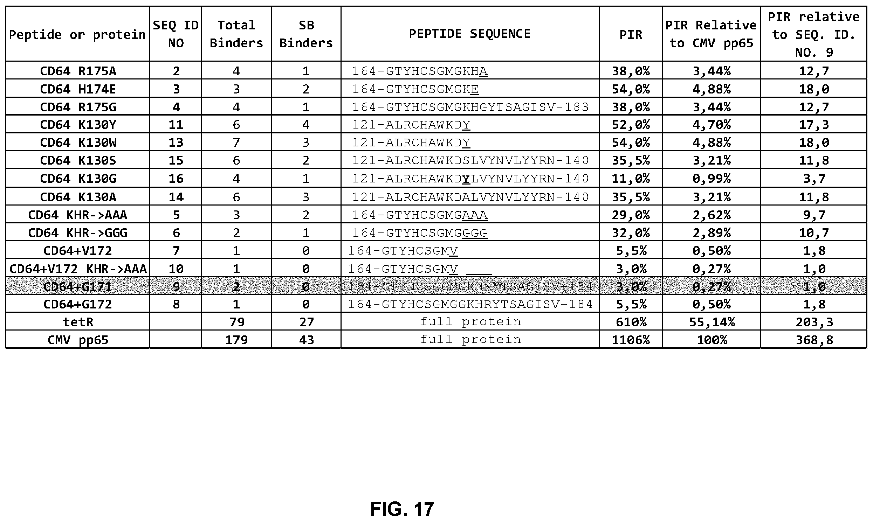

Figure 17: Classification of CD64 mutants by Potential Immunogenicity Rank (PIR).

**Detailed description of the invention**

Unless defined otherwise, all technical and scientific terms used herein have the same meaning as commonly understood by one of ordinary skill in the art to which the invention pertains. Although any methods and materials similar or equivalent to those described herein can be used in the practice for testing of the present invention, the preferred materials and methods are described herein. In describing and claiming the present invention, the following terminology will be used.

It is also to be understood that the terminology used herein is for the purpose of describing particular embodiments only, and is not intended to be limiting.

"High affinity Fc gamma receptor or CD64", refers to a molecule that occurs on the surface of cells and interact with immunoglobulins with high affinity, that is, the dissociation constant is about 10 nanomolar.

"CAR", are chimeric antigen receptors artificially introduced on the surface of T lymphocytes and Natural Killer (NK) cells by for example electroporation, adenoviral, retroviral or lentiviral transduction or using chemical compounds that facilitate introduction of exogenous genes into cells. Such CARs comprise at least: (1) an target recognition domain (TRD) that should be a receptor, a ligand, the variable region of an antibody or equivalent polypeptide, for example a scFv, a camelid single chain antibody, a fynomer, an ankyrin, a nanobody or any polypeptide with enough affinity for a target protein; (2) a transmembrane domain to join the extracellular antigen recognition domain to the cell membrane and sometimes using a linker or spacer between the TRD and the transmembrane region to avoid steric hindrance and (3) an intracellular signal transduction domain or intracellular stimulation domain that includes a main activation domain derived from intracellular domains of CD3z or fragments thereof that are polypeptides that contain intracellular domains with ITAM sequences and at least one co-stimulatory domain for optimal signal transduction that may be selected from the intracellular domains of CD28, 4-1BB, ICOS, OX40, DAP10 or CD27 for example.

"Intracellular stimulation domain" is a domain derived from the intracellular region of CD3z that comprises either the whole intracellular domain of CD3z chain that have 3 ITAMs or a fragment of intracellular region of CD3z with 2 ITAMs.

"Intracellular co-stimulatory domain" is a domain derived from the intracellular region of co-stimulatory receptors expressed on the surface of T lymphocytes or NK cells. Such intracellular coo-stimulation domain may be derived from CD28, 4-1BB, OX40, ICOS, CD27 or DAP10 for example.

"First generation CARs or 1G-CARs", are chimeric antigen receptors were the ICD comprises only CD3z.

"Second generation CARs or 2G-CARs", are chimeric antigen receptors were the ICD comprises both CD3z and another co-stimulation domain selected for example from CD28, 4-1BB, OX40, ICOS, CD27 or DAP10. Examples of 2G-CARs may be CD28-CD3z, 4-1BB-CD3z, OX40-CD3z, ICOS-CD3z, CD27-CD3z or DAP10-CD3z.

"Third generation CARs or 3G-CARs", are chimeric antigen receptors were the ICD comprises both CD3z and two other co-stimulation domains selected for example from CD28, 4-1 BB, OX40, ICOS, CD27 and DAP10. Examples of 3G-CARs may be CD28-4-1BB-CD3z, CD28-DAP10-CD3z, CD28-CD27-CD3z, 4-1BB-ICOS-CD3z, 4-1BB-CD27-CD3z or DAP10-ICOS-CD3z.

"Universal CARs", are chimeric antigen receptors were the target recognition domain (TRD) is a polypeptide that does not directly recognizes the target antigen on the surface of a target cell, for example on the surface of a tumor cell, but such universal CAR has a Ligand Recognition Domain (LRD) instead of a TRD and such LRD recognizes a second soluble ligand that is a fusion polypeptide with a target recognition domain (TRD) that specifically binds to a target antigen on the surface of a target cell and has also a domain with affinity for the LRD in such a way that there is only specific target recognition in the presence of this ligand that acts as a bridge between the lymphocyte and the target protein on the surface of target cell.

"Monomeric and dimeric polypeptide", a polypeptide should exist as a single chain and thus this polypeptide is termed a monomer but also polypeptides may associate with other polypeptides and in this case the resulting protein is termed a dimer if there are two polypeptides, a trimer if there are three polypeptides and so on. In addition, dimer, trimer, tetramer or polypeptides may be composed of identical polypeptides and then they are termed homodimer or homotrimer if they are composed of two or three identical polypeptides. Other kind of dimeric, trimeric or multimeric proteins are composed of at least two different polypeptides and then they are called heterodimers, heterotrimers or heteromeric proteins. For a polypeptide to be associated into dimers, timers or multimers there must be interacting regions between subunits to promote and keep the association between different polypeptide chains. There are three kinds of interacting regions relevant for the methods of the present invention: (1) interaction between transmembrane regions, for example the interaction between high affinity Fc receptors and the common gamma chain is mediated by their respective transmembrane regions; (2) interactions mediated by a disulfide bond, for example the interaction between immunoglobulins heavy chains is mediated by a region of the immunoglobulin called hinge region that contain cysteines that binds each other and keeps the dimeric structure of the immunoglobulin; (3) interactions mediated by protein dimerization domains such as protein homodimerization domains or protein heterodimerization domains that though complementary hydrophobic or electrostatic interactions promote association of several polypeptides.

"Fc gamma fusion protein" is a polypeptide comprising at least the Fc gamma region of human IgG1 or IgG4 that comprises at least the hinge region, CH2 and CH3 domains of IgG1 or IgG4 fused at the carboxyl terminal end of a target recognition domain (TRD) that may be selected for example from a Fab, a scFv, a nanobody, an ankyrin, a fynomer or a scTcR in such way that the general structure of the fusion protein from amino to carboxyl terminal end is TRD-hinge-CH2-CH3 where hinge, CH2 and CH3 are the domains of IgG1 or IgG4.

The present invention describes CARs based on mutants of the high affinity Fc gamma receptor alpha chain (CD64) where such mutants have about 7 to 20 fold reduced affinity for both serum IgG and a specific IgG that binds a target antigen on the surface of a target cells but as a consequence of such reduced affinity this mutated CD64 is less sensitive to inhibition by unrelated IgG present on normal serum and thus more efficient CARs are developed using those mutants of CD64 with lower affinity for IgG than using wild type CD64. Preferred mutants of CD64 are described and are based on novel mutants with low inhibition by human serum that also are predicted non immunogenic when peptides are processed and presented into HLA I and such non immunogenic mutants of CD64 are especially useful for development of CAR-Ts that are going to be infused multiple times into patients. In addition, certain intracellular co-stimulatory domains that mitigate inhibition by human serum are disclosed. Such universal chimeric antigen receptors may be used for immunotherapy of several diseases like cancer, infectious diseases or autoimmune diseases.

Current state of art teaches that the development of a CD16-based CAR is possible but also that development of CD64-based CARs is not possible because they are inhibited by unrelated serum IgGs and thus it is currently unknown if mutated CD64 with affinities for IgGs between that of CD16V158 and that of native CD64 may be used to develop useful CARs.

The present invention is based on the following findings: (1) CARs based on CD64 have better surface expression than equivalent CARs based on CD16, a finding that underlines the fact that each Fc receptor is different ; (2) unexpectedly cytotoxicity of native CD64-based CARs is not inhibited by serum using either protein targets like CD20 or CD22 and this fact is unexplained as according to law of mass action the receptor must be fully occupied by serum IgGs at the normal concentrations of those IgGs found in serum; (3) cytokine secretion in a 24 hour coculture between T lymphocytes transduced with a CD64-based CAR and a target cell in the presence of both, a specific IgG1 antibody at 5 micrograms per milliliter and 20 percent of human serum is inhibited up to a 80 to 90 percent for a native CD64 to about a 25 percent for a mutant CD64 with a Kd for human IgG1 (huIgG1) of 180nM, that is a mutant CD64 with an affinity for huIgG1 about 1,9 fold higher than that of CD16V158; (4) the inhibition of activity of a mutated CD64 based CAR-T may be mitigated if the CAR comprises the CD28 intracellular co-stimulation domain; (5) CAR based on mutants of CD64 with very low predicted HLA I immunogenicity are described in the methods of the present invention and such CAR-Ts are particularly useful for treatment where multiple infusions of CAR-T are needed.

We describe in detail each of the above findings.

CARs based on CD64 have better surface expression than equivalent CARs based on CD16.

The process employed to produce a CAR include the production of a lentiviral supernatant and the transduction of T lymphocytes with such lentiviral supernatant.

To produce lentiviral supernatants 293T cells are plated for example in 175 square centimeters flasks, at 44.000 cells per square centimeter in 40 milliliters of DMEM containing 10 percent FBS medium, 24 hours before transfection and then transfected with 10 micrograms of a vector that has inserted the CAR and 30 micrograms of a 1:1:1 mix of pLP1:pLP2:VSVG plasmid vectors using transfection reagents like for example Canfast™ reagent, as recommended by manufacturer. After 48 hours supernatants are harvested and concentrated using Amicon centrifuge filters as recommended by manufacturer. Lentiviral titers are determined using cell lines like for example Jurkat. Cells are transduced, for example using 200.000 cells, with different dilutions of concentrated lentiviral supernatants and the percentage of positive cells is measured using specific antibodies and flow cytometry.

Titer is normally calculated using the following equation

$$\text{Titer (IU/mL)} = [\text{Amount of cells}]^* \ [\text{percent (+) cells}]^* \ [\text{dilution fold}]/[\text{volume (mL) of infection}]$$

where [amount of cells] is 200.000; the [percentage of positive cells] is the percent of positive cells at a dilution of supernatant that produces about a 10 percent transduction measured by flow cytometry; [dilution fold] is the number of times that a supernatant is diluted to produce about a 10 percent transduction and [volume of transduction] is fixed to 0,5mL.

Transduction of T lymphocytes is normally made using at least 200.000 primary T lymphocytes isolated from peripheral blood of healthy people using human Pan T Cell Isolation Kit from Miltenyi Biotec. T lymphocytes that are activated with for example anti-CD3/anti-CD28 Dynabeads from ThermoFisher at 3:1 bead to cell ratio during 24-48 hours in X-VIVO™ 15 medium containing 10 percent of FBS and 50 units per milliliter of human interleukin-2 and activated T cells are transduced by centrifugation in 24-well plates containing 200.000 T lymphocytes and concentrated lentiviral supernatant with 5 micrograms per milliliter of polybrene. 24 hours after transduction medium is exchanged and cells are cultured in X-VIVO™ 15 medium containing 50 units per milliliter of human interleukin-2.

Importance of nature of hinge in CAR development is already known in state of art but most of previous work has been done in scFv-based CARs. For example, Guest RD et al (see Guest RD et al, 2005) analyzed four different scFv-based CARs for CEA, NCAM, oncofetal antigen 5T4 and CD19 and they found that 5T4 and NCAM scFv-based CARs showed enhanced specific cytokine release and cytotoxicity only when possessing an extracellular spacer region while CEA and CD19 scFv-based CARs performed better cytokine release only in the absence of an extracellular spacer. In another work Qin L et al (see Qin L et al, 2017) analyzed the effect of incorporation of a hinge domain derived from either IgG4 CH3 domain or IgD in five different scFv-based CARs against CD19, mesothelin, PSCA, MUC1 and HER2 and they found that a hinge promotes expansion mainly of CD4 positive CAR T cells and that there was enhanced antitumor capacity for certain target antigens like mesothelin but not for CD19 with hinge incorporation.

Other investigators have also found that hinge optimization may be even avoid tonic signaling and cell exhaustion phenotype (Jonnalagadda M *et al,* 2015; Watanabe N *et al,* 2016) that found that a hinge based on human IgG1 CH2 plus CH3 domains binds to CD64 and promoted macrophage elimination but also tonic signaling, exhaustion and lower antitumoral effect but mutation of the hinge to eliminate binding to Fc-receptors or use of a IgG4 hinge was useful to improve antitumoral effect and tumor localization of CAR T cells.

In addition most of the hinges described to date are dimeric, for example hinge from CD8 alpha has a cysteine at position -2 relative to the start of transmembrane region (according to Uniprot P01732) while hinge from CD28 has a cysteine at position -12 relative to the start of transmembrane region according to Uniprot P10747 and CARs using both hinges and containing scFv are both dimeric when analyzed by Western blot, but it is not clear if a dimeric hinge is important for the development of efficient CARs.

Thus, for scFv-based CARs the hinge may: (a) reduce the distance to a target epitope; (b) ameliorate steric hindrance of target epitope; (c) increase the flexibility of the scFv for access to target epitope; or (d) dimerize the CAR. But today there is no rule for a selection of an optimized hinge and as stated by Jonnalagadda M et al 2015, "currently there is no general principle that can be applied to the optimal hinge/spacer/linker to use when designing a CAR and rather, the optimal sequence to use for a particular antigen will need to be empirically determined". All the above conclusions are valid for scFv-based CARs but for Fc receptors based CARs the problem is still more difficult because most of our knowledge about CAR design is based on scFv as binding molecules that only have one domain but for CD64, a large molecule with three domains where another large antibody ligand with at least four domains is going to be bound, current state of art teaches us about using a hinge in the same way it is used on most CAR-T based on scFv.

In the present invention we demonstrate that, a CAR based on CD64 without any hinge is expressed more efficiently than a CAR with about 15 amino acids, for example, the commonly used hinge and transmembrane domain from human CD8 alpha chain with a hinge of 15 amino acids that includes a cysteine for dimerization has a lower expression than a CAR without any hinge based on the same CD8 alpha, that is, a monomeric CD64-based CAR-T is more efficiently expressed on the cell surface of primary T lymphocytes than a dimeric CAR-T. Figure 4 shows a comparison of surface expression on primary T lymphocytes of a CD64-based CAR-T with a CD16-based CAR having each TM of CD8 alpha and 4-1BB and CD3z intracellular domains. CAR based on CD64 has no hinge while CD16 has a 14 amino acids-long hinge. Non-transduced T lymphocytes were included as controls in (A) and (C). (B) is the histogram of a CAR with CD64 extracellular domain fused to the transmembrane of CD8 alpha with 4-1BB and CD3z intracellular domains and; (D) is the histogram of a CAR with CD16 extracellular domain fused to a 14 amino acids-long hinge of human CD8 alpha and transmembrane of CD8 alpha with 4-1BB and CD3z intracellular domains. The percentage of T lymphocytes that express the CD64-based CAR on their surface is 31,6 percent in (B) while the percentage of T lymphocytes that express the CD16-based CAR on their surface is 9,8 percent in (D) (see Table 1A).

As a conclusion, using above conditions, we determined that to produce at least a 20 percent of transduction of human primary T lymphocytes, it is needed at least a 10 to 100-fold concentration of lentiviral supernatants, except for lentivirus that express eGFP that may be used unconcentrated to reach about a 20-30 percent of eGFP positive T lymphocytes. As a rule, for a CD64-based CAR a 10 to 30-fold concentration of lentiviral supernatant is needed while upon replacing CD64 for CD16 as the extracellular domain, the concentration fold of lentiviral supernatant is increased to about 100-fold for an equivalent percent of transduction. This difference is highly relevant to produce CARs as the cost of lentiviral supernatant is about 30 to 40 percent of total cost of CAR production, thus, reducing up to 3-fold the amount of lentiviral supernatant may reduce the production cost of CARs at least a 20 percent. Reducing CAR production costs may be important to reduce the final price of the product as those customized products have a high price that may compromise the access of patients to this highly effective therapy.

Cytotoxicity of a CD64 based CAR is not inhibited in the presence of human serum.

Concentration of IgG1 in human normal serum is between 5 to 9 milligrams per milliliter and human CD64 has a dissociation constant (Kd) of about 10 nanomolar and thus using an average of 7 mg per mL as the free antibody concentration in serum, then 99,98 percent of CD64 molecules must be occupied at any time, that is, there are only available 2 molecules for each 10.000 molecules. When considering that about 200 molecules are needed for cytotoxicity and 2.000 molecules are needed for cytokine induction in T lymphocytes (see for example Watanabe K et al, 2015), then a CAR based on CD64 with about 1 million molecules of CD64 is needed per lymphocyte to be active, while about 10 million molecules are needed for proliferation if all CAR molecules are available for cell-to-cell contact. As the number of CAR molecules per cell is well below 100.000 molecules per cell, probably between 10.000-60.000 molecules per cell, then CD64 must considered not useful for the development of a CAR. Unexpectedly, we have found that cytotoxicity of CD64 based CARs is almost unaffected by human serum concentrations up to 20%, the maximal amount we have tested in vitro. Using a 20% of human serum it is expected to have a 99,90 percent of CD64 molecules bound to unrelated serum IgG and thus, only 10 molecules are available for every 10.000 molecules and using the above numbers about 200.000 molecules per cell are needed for cytotoxicity while 2 million molecules per cell are needed for proliferation and those figures are about 10 to 100 fold higher than the real number of CAR molecules per cell. Thus, current state of art teaches against using CD64 as a basis for a CAR development and this is true from both the theoretical basis and from lab research. But unexpectedly we have found that total cytotoxicity using Raji, a CD20 positive human lymphoma cell line, using rituximab (anti-CD20) antibody at 5 micrograms per milliliter and a native CD64 based CAR the difference in cytotoxicity has not statistical significance (p>0,05) when there is no human serum in the assay versus a 20 percent human serum during the assay. Those results are shown in Figure 12. Then we compared cytotoxicity using CD22 as a target antigen because this molecule has a lower surface expression than CD20, and thus, it should be considered a more stringent model antigen to test if cytotoxicity is inhibited by unrelated IgG present in human serum. Results are shown in Figure 13 and unexpectedly there was no inhibition of cytotoxicity by human serum for an antigen with low surface expression like CD22. That is, it is expected that a receptor like CD64 with a constant of dissociation (Kd) of about 10 nanomolar on the presence of about 1,4 milligrams per milliliter of unrelated IgG1, is saturated by this unrelated IgG1 and thus there are no free CD64 molecules to be bound to rituximab as about 99,9 percent of CD64 molecules are occupied. When just specific lysis is considered there were also no differences between cytotoxicity without human serum and cytotoxicity in the presence of 20 percent of human serum. This unexpected finding demonstrates that using cytotoxicity as a measure of efficacy of a CAR, it is possible to use native CD64 to develop a CAR. This finding refutes the theoretical predictions from the law of mass action that establishes relationships between Kd and all the reactants and thus for a Kd of 10nM of native CD64 and a free IgG1 concentration in serum of 7 mg per mL there is a very low fraction (about 0,02 percent, that is 2 molecules of every 10.000 molecules) of free CD64 on the surface of cells available to be bound by a specific antibody to lyse target cells. In addition, this finding is also in contradiction with to current experimental state of art where a CD64 based CAR-T developed about 25 years ago (Smyth MJ *et al*, 1994) is not considered a viable alternative to scFv-based CARs due to the high serum concentration of IgG that compete with specific antibodies and the surface of immune cells that express CD64 is almost fully occupied by blocking antibodies.

We have selected 5 micrograms per milliliter as the amount of specific antibody and 20% of human serum to be used to test inhibition of CD64 by unrelated antibodies based on current knowledge about therapy with monoclonal antibodies. For example, maintenance standard dose of rituximab is 375 milligrams per square meter using mainly two administration schedules: infusions every 3 months for two years and infusions every 2 months for 1-2 years and previous studies have proposed a presumptive active level of 25,000 nanograms per milliliter in anti-lymphoma treatment (Jager U *et al* 2012). In "in vitro" cell culture it is not possible to use 100 percent human serum as culture medium, so we used 20 percent human serum, that is, a five-fold lower concentration of serum immunoglobulins than that found in vivo and thus, if we assume that a serum level of 25 micrograms per milliliter is an optimal level then this amount should be equivalent to 5 micrograms per milliliter in vitro. The above assumptions imply that a steady serum levels of an antibody must be 25 micrograms per milliliter to have the same level of inhibition, and thus, if serum levels are for example 10 micrograms per milliliter then the inhibition should be best modulated using an in vitro level of about 2 micrograms per milliliter. In another example, if serum levels are about 50 micrograms per milliliter then in vitro equivalent levels, using 20 percent human serum as inhibitor, are 10 micrograms per milliliter. All above serum levels may be reached in vivo in patients, for example, just after antibody administration, serum levels may be well over 50 micrograms per milliliter while just before antibody administration the serum levels may be below 10 micrograms per milliliter and thus, in general the use of an antibody concentration in vitro between 2-10 micrograms per milliliter is a good in vitro model to estimate percent inhibition of CD64 by serum. We thus have selected this range, mainly 5 micrograms per milliliter in the presence of 20 percent human serum, to measure inhibition. The concentration of antibody of 2 micrograms per milliliter is a more stringent condition to test inhibition by serum while the use of an antibody concentration of 10 to 20 micrograms per milliliter is a less stringent condition to test inhibition but implies that higher doses of antibodies must be administered to patients to reach an steady serum level of about 50 to 100 micrograms per milliliter. In absence of human serum, a concentration of a specific antibody between 1 to 20 micrograms per milliliter is equivalent.

_Cytokine secretion after 24 hours of coincubation of a CD64-based CAR with a target cell and a specific antibody is highly inhibited in native CD64 in the presence of human serum and a combination of certain mutations of CD64 with selected intracellular co-stimulation domains help to reduce the inhibition of cytokine secretion induced by human serum to a level that support proliferation._

Clinical success of current CARs is strongly related to the ability of such immune engineered cells to proliferate when target cells expressing the antigen recognized by CAR are encountered, that is CAR activity is related by its cytotoxicity against tumoral cells but first generation of CARs bearing just CD3zeta were also cytotoxic but did not persist. CAR persistence is related to proliferation and cell division and cytokine secretion is normally used as a biomarker of potency and proliferation. Several cytokines like gamma-IFN, IL-2, TNF-alpha and GM-CSF may be used as biomarkers of proliferation of CARs. The most common assay is a short-term co-culture between CAR T cells and target cells and usually assays from overnight (about 16 to 20 hours) (Schneider D *et al*, 2017; Haynes NM *et al*, 2002) to 48 hours (Finney HM *et al*, 2004,) are used to measure cytokine secretion, being 24 hours the most common incubation time (Milone MC *et al*, 2009; Long AH *et al*, 2015; Zah E *et al*, 2016) but also some use a time frame from 24 to 48 hours as an equivalent co-culture time (Grada Z *et al*, 2013).

In the methods of the present invention we have used 24 hours as the co-incubation time using T lymphocytes without IL-2 and without anti-CD3/ anti-CD28 beads for the last 16 hours. Selection of a 24 hours coculture is because during a coculture between T lymphocytes that express a CAR on the cell surface and target cells that express on surface a target antigen there are several events that occur at the same time, that is cytotoxicity where T lymphocytes lyse target cells, cytokine secretion by T lymphocytes and proliferation of T lymphocytes. Of the above events, cytotoxicity occurs early and in fact many cytotoxicity assays use 2-24 hours, but 4 hours is considered a standard assay. Cytokine secretion should be measured between 16 hours to for example 72 hours but it is important to assure that viable target cells remain in the coculture at later time points to keep effector cells with stimulus. In addition, target cells may modulate the expression of the target antigen after contact with an antibody and thus, kinetic of target antigen down-modulation after contact with the specific antibody should be important. Finally, to facilitate contact between effector and target cells, a high concentration of cells is used and then it is important to keep enough nutrients to cells. Thus, we have selected 24 to 72 hours when Raji is used in the co-culture to minimize nutrient consumption, to minimize the impact of loss of target cells and to avoid the effect of target antigen down-modulation. For other cell lines like Nalm-6 that is extremely sensitive to cytotoxicity, 24 hours is a better time of co-culture to compare different CAR-T when a 1:1 effector to target ratio is used but in general test conditions must be optimized for each cell line.

The coculture used in the methods of the present invention to measure cytokine secretion was the following: (a) 50.000 effector T lymphocytes with at least a 20 percent expression of the CAR on the surface measured by flow cytometry (final secretion of cytokines was normalized by the percent of CAR positive cells) were added per well of a round bottom 96-well microplate; (b) 50.000 target cells that express the target antigen were added to each well; (c) a final concentration of a specific antibody between 1-5 micrograms per milliliter was added to each well except to the control wells that were used as negative controls; (d) human serum at 20 percent used as an inhibitor of interaction between CD64 and specific antibody or fetal bovine serum (FBS) at 10 percent used as a control without inhibitor; (e) X-Vivo 15 culture medium was added to a final 200 microliter per well.

Using this standard coculture and the standard 24h co-incubation time, we have found that a universal CAR based on CD64 extracellular region linked to transmembrane of CD8alpha, either intracellular region of 4-1BB and intracellular region of CD3zeta (Bbz) or intracellular region of CD28 and intracellular region of CD3zeta (28z), Raji as a CD20 positive target cell and rituximab (anti-CD20) as a target specific antibody induces a level of about 6.000 picograms per milliliter for Bbz to 20.000 picograms per milliliter for 28z of gamma interferon measured by ELISA and normalized to 50.000 positive CAR cells in absence of human serum as inhibitor. As we have observed that time from lentiviral transduction has an effect in cytokine secretion level, we have used a time from transduction between 9-14 days for cytokine assays, more preferably about 11 days, but more importantly we have compared only conditions with: (a) the same time after transduction because gamma interferon secretion may be up to 40 percent lower if T lymphocytes of 14 days of transduction are used when compared with T lymphocytes of 9 days of transduction and (b) the same percent of transduction of T lymphocytes that was adjusted to the lower level using non-transduced T lymphocytes because it is known that activation of T lymphocytes secrete cytokines that not only activate the same cell in an autocrine signaling but also there is a paracrine activation in nearby cells and thus, both the number of CAR positive cells and total number of cells are important in a coculture. A range of 15 to 35 percent of expression of CAR on the surface of primary immune cells was always used for cytokine production assays; (c) results should be compared only if lymphocytes from the same donor are used as there is strong variability between lymphocytes of different donors.

Table 1 shows gamma interferon secretion of a co-culture of two CD64-based CAR-T with Raji and anti-CD20 antibody either in absence of human serum or in the presence of human serum. Results show that 20 percent human serum inhibits cytokine secretion to a 90 percent when a CAR-T based on wild-type CD64 is used while inhibition of a mutated CD64 is just a 20 percent.

**Table 1:** secretion of cytokines after 24h of co-culture with Raji and anti-CD20 antibody with two different CAR-T based on CD64.

| Secretion of cytokines after 24h of co-culture with Raji and anti-CD20 antibody | | | | | | |
|---|---|---|---|---|---|---|
| CAR-T | | 10% FBS | | 20% human serum | | % inhibition 20%HS vs 10%FBS |
| Extracellular | Intracellular | Mean | SD | Mean | SD | |
| CD64 wild type | 4-1BB-CD3z | 12.021,2 | 793,2 | 1.167,4 | 172,7 | 90,3% |
| CD64 with V172 | CD28-CD3z trunc | 11.475,2 | 593,9 | 9.131,9 | 273,8 | 20,4% |

Then the same experiment was extended to 72 hours. Table 2 below shows a comparison of unrelated IgGs present in human serum on inhibition of gamma-interferon secretion in a co-culture of two CD64-based CAR-T with Raji in the presence or absence of anti-human CD20 antibody. Those results show that a CAR based on wild type (native) CD64 and using 4-1BB and CD3z as intracellular domain have about 3-fold lower gamma interferon secretion after 72 hours of co-culture than a CAR-T based on a mutated CD64 with CD28 and a truncated CD3z as intracellular domain. But when 20 percent human serum is used then, a CAR-T containing wild-type CD64 has a 93 percent inhibition and thus is not useful to develop a CAR-T.

**Table 2:** Secretion of cytokines after 72h of co-culture with Raji and anti-CD20 antibody

| Secretion of cytokines after 72h of co-culture with Raji and anti-CD20 antibody | | | | | | |
|---|---|---|---|---|---|---|
| CAR-T | | 10% FBS | | 20% human serum | | % inhibition |
| Extracellular | Intracellular | Mean | SD | Mean | SD | |
| CD64 wild type | 4-1BB-CD3z | 122.637,3 | 18.024,0 | 8.807,6 | 1.879,1 | 92,8% |
| CD64 with V172 | CD28-CD3z trunc | 399.103,6 | 60.138,6 | 273.229,4 | 41.162,0 | 31,5% |

These results indicate that using Raji as the target cell either a 24 hour or a 72hour co-culture is a valid method to discriminate between different CD64-based CAR-Ts.

The specificity and affinity of human Fc gamma receptors for different IgG subclasses have been determined previously (Bruhns P *et al,* 2009) and have been reviewed (van der Poel CE *et al,* 2011). In general, CD64 has been described as the receptor of high affinity for IgG1, IgG3 and IgG4 and the dissociation constant for IgG1 is about 10-15 nanomolar. In contrast CD16 V158 allele has a Kd of about 325-500 nanomolar while CD16 F158 allele has a Kd of about 555-850 nanomolar, that is, affinity of CD64 for IgG1 is about 32-fold higher than that of the CD16 allele with highest affinity for IgG1.

Also, structure-activity relationship between CD64 and bound IgG1 have been described in state of art. The crystal structure of CD64 was determined for the first time by Lu J et al (Lu J *et al,* 2011) at 2,65 Angstroms. They demonstrated that: (a) the D3 domain, that is present only on CD64 but not in CD16 nor in Fc epsilon receptor I (FcERI), is positioned away from the IgG binding site and does not contact with IgG although is needed for binding, probably to increase the distance from the cell membrane of the IgG bound to CD64 receptor; (b) The interdomain angle between D1 and D2 domains of low affinity IgG receptors (CD16 and Fc gamma receptor IIA) is of about 55-60 degrees while the interdomain angle between D1 and D2 domains of the high affinity immunoglobulin receptors (CD64 and FcERI) is of 35-39 degrees, that is, high affinity receptors are more closed than their low affinity counterparts; (c) identity at the amino acid level of D1 and D2 domains between FcERI and CD16 is 41,1 percent, between CD64 and FcERI is 39,8 percent and between CD64 and CD16 is 43,9 percent, that is only about 70 amino acids are identical between those receptors while about 110 amino acids are different; (d) the FG-loop of CD64 with the sequence SEQ. ID. NO. 84 171-MGKHRY-176 contains a single residue deletion highly conserved between species when compared with other Fc receptors like FcERI and CD16 and replacement of FG-loop of CD16 with that of CD64 results in 9-fold increase of affinity of the new CD16. Previously it was demonstrated that a transplant of the FG loop of FcERI to Fc gamma RII confers detectable IgE binding (Hulett MD *et al,* 1993). But this research does not teach us if an amino acid should be inserted into the FG loop of CD64 and the effect on affinity, serum inhibition level, expression level into primary T lymphocytes and immunogenicity of the resultant mutant.

For example, Lu J *et al* (see Lu J *et al,* 2015) described seven mutations in FG loop of CD64 that reduce affinity of IgG1 for CD64. The amino acids with SEQ. ID. NO. 84 171-MGKHRY-176 are involved into FG loop interaction with glycans of IgG and they describe seven mutations each with different effect on the affinity of CD64 for IgG. However, those authors do not measure for example the inhibition level of mutants of CD64 by human serum, expression level of the mutants of CD64 on the surface of primary T lymphocytes nor immunogenicity of the resultant mutants of CD64, that is, they do not demonstrate the suitability of such mutant for the development of a CD64-based CAR-T. It is important to note that all the mutants of CD64 described by those researchers have an affinity for IgG1 higher than that of CD16V158 allele for IgG1 and thus CAR developed using CD16 may not be used as a model for mutants of receptors of CD64 with higher affinity for IgG1 than that of CD16. A second-generation CAR based on a Fc receptor was described for the first time by Teng MWL *et al* in 2006 (Teng MWL *et al,* 2006) but they do not teach how to solve problems associated to each Fc receptor. In case of CD64 the main problems to be solved are how to mitigate inhibition by human serum, how to efficiently express this CD64 on the surface of primary T lymphocytes and this research should be preferentially be done without increasing the immunogenicity of the novel CAR-T generated. For FcERI the main problem is how to avoid binding of infused IgE to mast cells and basophils to avoid an anaphylactic shock due to polymerization of IgE.

In a further research of CD64, the crystal structure of CD64 bound to Fc was determined at 1,80 Angstrom resolution (Kiyoshi M *et al,* 2015) revealing a unique hydrophobic pocket of CD64 that accommodates Leucine at 235 of the IgG1 and explains the high affinity of the interaction. Surprisingly those authors found that glycans attached to Fc make little contribution to the interaction surface of CD64 to IgG and this conclusion is in contrast with the results of Lu J *et al,* 2015 described above. This contradiction illustrates the extreme complexity of working with macromolecular complex of glycoproteins and thus, that every mutation must be tested experimentally and not just theoretically predicted. For example, the amino acid Lysine at 145 of CD64 interacts with Glutamic Acid at 269 (E269) of IgG1 (see Figure 3c, page 5 of Kiyoshi M *et al,* 2015) and thus we predicted that the mutation K145D of CD64 may be used to create a repulsion between this amino acid and E269 of serum immunoglobulins while a mutant IgG1 with E269K should keep the affinity of interaction. But surprisingly, this K145D mutant of CD64 does not express on the surface of primary T and thus is not useful for the development of CAR-Ts.

In the present invention we have demonstrated that when a CAR is generated based on native CD64 the cytotoxicity is not affected but cytokine secretion and proliferation are strongly inhibited when T lymphocytes that express a native CD64 based-CAR are cocultured with a target cell in the presence of an specific antibody and human serum as a source of unrelated IgG that may saturate the CD64 and inhibit the specific reaction. Thus, native CD64 is not useful for the development of a CAR and then we have selected the hydrophobic pocket based on the crystal structure with PDB accession code 4W4O described by Kiyoshi M et al, 2015 and the FG loop described by Lu J et al, 2015 to develop CD64 mutants to reduce affinity for IgG and to mitigate inhibition by human serum.

For hydrophobic pocket several mutants have been developed in the present invention. We selected the amino acids described in Table 3 of Example 2 to generate mutants as those amino acids are very close to Leucine at position 235 in IgG1, an amino acid that is essential for high affinity binding between CD64 and IgG1.

**Table 3:** Amino acids that form the hydrophobic pocket of CD64 and that are near Leucine at 235 in IgG1.

| distance (nm) | Leu 235 atoms | Hydrophobic pocket |
|---|---|---|
| 0,49 | Leu 235 CD1 | Trp 104 CB |
| 0,57 | Leu 235 CG1 | Leu 105 CG1 |
| 0,53 | Leu 235 CD2 | Leu 105 CD1 |
| 0,76 | Leu 235 CB | Leu 105 CB |
| 0,40 | Leu 235 CD2 | Ala 126 CB |
| 0,43 | Leu 235 CD1 | Lys 130 CG |
| 0,36 | Leu 235 CD2 | Val 132 CG1 |
| 0,59 | Leu 235 CG1 | Val 132 CB |
| 0,51 | Leu 235 CB | Val 132 CB |
| 0,54 | Leu 235 CG2 | Tyr 176 CB |
| 0,45 | Leu 235 CD2 | Tyr 176 CB |

Amino acids selected from W104, L105, A126, K130, V132 and Y176 constitute the hydrophobic pocket that accommodate L235 of IgG1 and thus, those amino acids may be changed to generate mutants of CD64 with reduced affinity for IgG to mitigate inhibition by human serum.

Lysine at 130 in CD64 was selected to generate several mutants because lysine is a positively charged amino acid but containing a long lateral group with four -CH2- groups that may be considered hydrophobic and thus, the four lateral CH2 groups are part of the wall of the hydrophobic pocket while the positively charged amino group at the end of lysine is part of the top of this hydrophobic pocket. The hydrophobic lateral group of Leucine at position 235 of IgG1 is inserted into the hydrophobic pocket and contact with the wall of the hydrophobic pocket, that is, there are van der Waals interactions between K130 and L235.

Mutations in protein engineering may be designed using the so-called "knob-into-hole" technology that comprises a "knob" mutation into one polypeptide and a "hole" mutation into the other polypeptide. The "knob-into-hole" technology is very well known in the state of art (see for example US Patent 5731168 and US Patent 7695936). This technology is based on the introduction of a protuberance or "knob" at the interface of a first polypeptide and a cavity or "hole" to promote specific interaction between the mutated pair of polypeptides. This protuberance may be created by substitution of a small amino acid by a larger one while substitution of an interacting larger amino acid at the other polypeptide with a small amino acid. Thus, we predicted two ways to generate mutants of CD64 with reduced affinity for IgG: (a) if an amino acid with a larger lateral group is used instead of K130, then there will be a "knob" that creates a steric hindrance and reduced affinity; (b) if a very small amino acid is used instead of K130 then van der Waals interactions with L235 are eliminated and thus affinity would be reduced. Amino acids selected to partially fill the hydrophobic pocket were Tyrosine, Phenylalanine, Tryptophan and Histidine while amino acids with small lateral group were selected from Serine, Glycine or Alanine. We have found that the mutation K130H generated a CAR-T where as expected cytotoxicity is not affected and also cytokine secretion in a coculture with a target cell and an anti-CD20 specific antibody in the absence of human serum is at the expected level but in the presence of 20 percent human serum the cytokine secretion is inhibited a 68 percent, that is, the mutation K130H reduces inhibition from about 85-90 percent when native CD64 is used to about a 70 percent, that is, this mutation mitigates inhibition after reducing percent of inhibition a 15-20 percent, but still there is a strong serum inhibition and thus, this mutation is not considered useful for the methods of the present invention. When affinity was measured using Biacore this CD64 with K130H mutation had a dissociation constant of 53 nanomolar, that is, an affinity about 5-fold lower for IgG1 than native CD64 that had 10 nanomolar. In contrast, K130S mutation, using the same coculture with Raji as target cell and anti-CD20 antibody, had an inhibition by 20 percent human serum of 49 percent, respectively, that is, K130S mitigate serum inhibition from 85-90 percent to about 50 percent. Another mutant, CD64 with K130Y mutation, had a 24 percent inhibition by 20 percent human serum , that is, it mitigates serum inhibition from 85-90 percent (see Table 2) to about 25 percent as shown in Table 4

**Table 4:** Secretion of cytokines after co-culture with Raji and anti-CD antibody

| Secretion of cytokines after 24h of co-culture with Raji and anti-CD20 antibody | | | | | | | | | | |
|---|---|---|---|---|---|---|---|---|---|---|
| CAR-T | | Antibody | | 20% Human Serum (HS) | | 2,5% Human Serum (HS) | | 10% FBS | | percent inhibition 20%HS vs 10%FBS |
| Extracellular | Intracellular | Type | $\mu\beta$/mL | Mean | SD | Mean | SD | Mean | SD | |
| CD64K130Y | 4-1BB-CD3z | - | 0 | 336,4 | 7,9 | 409,0 | 8,3 | 273,4 | 9,4 | - |
| CD64K130Y | 4-1BB-CD3z | IgG1 anti-CD20 | 2 | 4.519,9 | 352,3 | 5.751,9 | 161,6 | 5.914,8 | 175,8 | 23,6% |
| CD64K130Y | 4-1BB-CD3z | IgG1 L235A anti-CD20 | 2 | 292,1 | 26,2 | 2.717,4 | 37,2 | 6.267,8 | 167,5 | 95,3% |

These results demonstrate that mutations in amino acids that contribute to the formation of the hydrophobic pocket like K130 generate mutants of CD64 whose serum inhibition is compatible with their use for the development of useful CD64-based CAR-T. If no antibody is used in the reaction then there is no cytokine secretion (see Figure 6, left), an anti-CD20 Mab induces cytokine secretion and the percent inhibition is below 25 percent (Figure 6, center) while a mutated antibody with Leucine at 235 mutated to Alanine is strongly inhibited by human serum at 20 percent (Figure 6, right). Leucine at 235 is the main contributor to affinity of IgG1 for CD64.

Above results demonstrate that both strategies, that is, to fill the hydrophobic pocket with a larger amino acid than Lysine is an useful strategy to reduce affinity and mitigate human serum inhibition but also mutation of Lysine to an amino acid with a short lateral group eliminates van der Waals interactions and is also a good strategy to generate useful CD64-based CAR-T, albeit the use of the former strategy mitigates better human serum inhibition than the use of a small amino acid. Affinity of CD64 with K130S measured using Biacore was 74 nanomolar, and thus, a reduction of about 7-fold the affinity of CD64 for IgG1 is considered a threshold that helps to mitigate human serum inhibition needed to generate useful CD64-based CAR-T. Above experiments were done using an anti-CD20 antibody and CD20 is a molecule highly expressed on the surface of B lymphocytes and with low internalization rate after bound to an antibody and thus we used CD22 as a second model antigen, because this antigen has a surface level about 10 times lower than CD20 and an internalization rate much higher than that of CD20. Using CD22, the CD64 K130Y mutation generates a CAR-T that is inhibited a 54 percent by human serum, that is CD22 is more sensitive to serum inhibition than CD20 and this may be explained by a lower surface expression and higher rate of internalization of CD22 versus CD20. Anyway, this result demonstrate that for antigens like CD20 with a high surface level and low rate of internalization after bound to antibody and also for antigens like CD22 with lower surface expression and higher internalization rate after bound to an antibody, mutants that occupy the hydrophobic pocket are valid strategies to mitigate inhibition by human serum and thus they are useful to generate CD64-based CAR-T of the present invention.

Above results using hydrophobic pocket also help to evaluate known mutants of FG loop. Among the seven mutants described by Lu J *et al*, 2015, only three had a dissociation constant higher than 70 nanomolar and thus the other four mutants were considered not useful for the methods of the present invention. The CD64 with R175A mutation was developed and serum inhibition of cytokine secretion in a coculture was measured either in absence of human serum and in the presence of human serum. In a coculture with an R175A CD64-based CAR-T and using Raji and anti-CD22 as a target antigen, we have found that as described in Table 10 of Example 6, there is a 52 percent of inhibition of cytokine secretion, an inhibition level equivalent to that of K130Y mutation when the same anti-CD22 was used.

Overall, all the above results demonstrate that using certain mutations in both the hydrophobic pocket or in FG loop of CD64 it is possible to mitigate inhibition of cytokine secretion by human serum, but it is important to demonstrate that remaining cytokine secretion still supports proliferation of T lymphocytes that express such CAR-T on their surface. It is important to underline that albeit mutations mitigate the inhibition of cytokine secretion by human serum, we have not identified any mutation that suppress inhibition at all, that is, best mutants have at least a 20-30 percent inhibition by human serum for an antigen of high surface expression like CD20 but for antigens like CD22 with lower surface expression the same mutants are inhibited about a 50 percent by human serum at 20 percent.

A question that remains is about the cytokine level that is needed to sustain proliferation of primary T lymphocytes in the presence of human serum. A repetitive proliferation experiment was designed to measure proliferation and cytokine secretion of a coculture of primary T lymphocytes transduced with a selected mutant of CD64-based CAR-T, Raji cells, an anti-CD20 antibody and either FBS or 20 percent of human serum as inhibitor of specific interaction between CD64 and the anti-CD20 antibody. As referred in Example 5, Figure 9 and Table 9 show the cytokine secretion of a proliferation experiment after 3 days and 7 days in absence or presence of human serum and Figure 11 shows the proliferation fold of those T lymphocytes. Those results demonstrate that in a repetitive proliferation assay the cytokine secretion of a wild-type CD64-based CAR-T is inhibited about a 90 percent after 3 days and about a 70 percent after 7 days. In contrast, the cytokine secretion of a mutated CAR-T with an insertion of Valine at 172 is inhibited about a 40 percent after 3 days and about a 50 percent after 7 days. In addition, those results demonstrate that a CAR-T containing both a mutated CD64 and CD28 intracellular co-stimulation domain has about 15-fold higher or 11-fold higher cytokine secretion after 3 days and 7 days, respectively, in the presence of 20 percent of human serum than a CAR-T that contains both a wild-type CD64 and 4-1BB intracellular co-stimulation domain. That is, a CAR-T based on mutated CD64 still has inhibition of cytokine secretion and proliferation by unrelated immunoglobulins present in human serum but including CD28 intracellular co-stimulation domain instead of 4-1 BB, generates a CAR-T with a cytokine secretion level that is about 2-fold higher than that of a wild-type CD64-based CAR-T that contains 4-1BB as intracellular co-stimulation domain. Thus, an important finding of the present invention is that inhibition by human serum may be further mitigated by the use of CD28 intracellular co-stimulation domain instead of 4-1BB co-stimulation domain because absolute cytokine secretion level of CD28 based CAR-T in the presence of human serum is two-fold higher than the level of an equivalent 4-1BB based CAR-T in the absence of human serum. This mitigation of the inhibition by human serum is even more important for antigens like CD22 with lower expression that as demonstrated in the present invention are more strongly inhibited by unrelated immunoglobulins present in human serum. Finally, results from proliferation experiment shown in Figure 11 describe that in the absence of human serum as inhibitor primary T lymphocytes that express a CD64-TMCD8-Bbz CAR-T proliferate 32 fold in 7 days in absence of human serum while a mutant CD64+V172-TMCD8-28z proliferate 38 fold in the same time also in absence of human serum. While the level of gamma interferon released by T lymphocytes in absence of serum is more than 7 fold higher for a CAR-T containing CD28 than for a 4-1BB containing CAR-T but there is no statistically different proliferation level, those results suggest that there is a threshold to support proliferation but there is no correlation between gamma interferon secretion and proliferation. When human serum is present, CD64-TMCD8-Bbz, proliferate just 5,5-fold in 7 days while a mutant CD64+V172-TMCD8-28z proliferate 24-fold, that is, a CAR-T that uses a combination of a mutant CD64 with lower inhibition by human serum and CD28 as a co-stimulator supports a proliferation that is 4,3-fold higher than that of a CAR-T with a native CD64 and 4-1BB as a co-stimulator. Those results also suggest that a gamma interferon secretion level 3 times lower than the level of that of a 4-1BB based CAR-T does not support proliferation, that is, there is probably a low threshold to support proliferation and a high threshold after which a higher cytokine secretion level does not increases proliferation further.

Above results demonstrate that cytokine secretion and proliferation are useful methods to test inhibition by unrelated IgGs present in human serum of CAR-T based on CD64. On the contrary, cytotoxicity is not a good method to test inhibition by human serum. Using cytokine secretion and proliferation we have demonstrated that some CD64 mutants are inhibited less than a 50 percent and such inhibition may be further mitigated by the use of CD28 as intracellular co-stimulation domain. In addition, we have tested such mutants for cytotoxicity using CD22 as a target antigen either in the absence or presence of human serum and results are shown in Figure 14 for a CAR-T based on CD64 with R175A, a mutation in FG loop and Raji as a target cell and as expected there are no statistically different values of cytotoxicity when human serum is used instead of FBS. In the same way, Figure 15 shows results for a CAR-T based on CD64 with K130Y, a mutation in the hydrophobic pocket, Raji as a target cell and anti-CD22 antibody and again there are no statistically different values of cytotoxicity when human serum is used instead of FBS. Overall, we have developed novel CAR-T based on mutants of CD64 that have an inhibition level compatible with their utility for development of novel CAR-T. Such CAR-T should contain the transmembrane of CD8 alpha and no hinge for efficient expression on the surface of primary T lymphocytes and using CD28 intracellular co-stimulation domain remaining inhibition by human serum is mitigated.

Another important property of a CAR-T is that it must be non-immunogenic.

Mutations introduced into CD64 must be designed for the lowest possible immunogenicity to increase CAR persistence in vivo.

Current knowledge about CAR-T is mainly derived from scFv-based CAR-T that are derived from the variable region of antibodies and thus it is important to know several structural properties of antibodies to understand their use for target recognition. Antibodies are heterodimeric molecules that comprise one light chain and one heavy chain and each chain comprise a variable region and a constant region and the variable region of an antibody is formed by one light variable region and one heavy variable region that together constitute the region that makes contact with antigen. Each variable region has 3 hypervariable regions called Complementarity Determining Regions (CDRs) embedded into more conserved regions that perform basic structural functions necessary for proper folding of the whole variable domain and those more conserved regions are called frameworks regions. Thus, the general structure of a variable region of either a light or a heavy variable chain is from amino terminal end of the polypeptide to carboxyl terminal end: a first framework (fw1), a first CDR (CDR1), a second framework (fw2), a second CDR (CDR2), a third framework (fw3), a third CDR (CDR3) and a fourth framework (fw4) and the last framework is fused to the constant region that may be a kappa or a lambda light chain or a heavy constant chain that may be selected from alpha, delta, epsilon, gamma or mu chains. Thus, each antibody has 3 CDRs of a variable light chain and 3 CDRs of a variable heavy chain surrounded by 8 frameworks that keep up the proper folding of the whole variable domain for contacting the antigen with enough affinity.

The main region of variability of antibodies is located within CDR3 of the heavy chain because at least 3 different gene segments participate in the formation of this CDR: the genes that code for variable region (V) that are about 100 different genes, the Joining minigenes (J) that are about 4 to 6 regions and the Diversity gene segments (D) that are about 30 different regions in the genome. In the heavy chain the CDR3 is the junction point of the V, D and J regions and thus it is highly variable, even within human antibodies because of above combinations but also due to somatic hypermutations that introduce more variation by altering residues throughout the variable region. As a consequence of this variability even fully human antibodies may contain sequences not found in any other human protein that are generated by recombination and by somatic hypermutations that is a key mechanism of generating antibody diversity and to increase affinity for antigens in the secondary immune response. As a result, there are sequences located within CDRs that might be recognized as foreign sequences by immune cells. Above properties of antibodies may be reviewed elsewhere (Frazer JK and Capra JD, 1999). Briefly, when a foreign protein enters into the body, B lymphocyte recognize such protein using surface immunoglobulins that recognize epitopes in the foreign protein and as a result, new antibodies are secreted into blood that recognize the foreign protein and macrophages eliminate it through, for example phagocytosis of antigen-antibody immune complexes. This is called a humoral immune response, that is, an immune response mediated by antibodies. When an antibody has an antigen-binding site or paratope that is also recognized as foreign by other antibodies, then a second antibody is formed that recognizes and eliminates the first antibody that has a paratope recognized as a foreign epitope. This may form a network of idiotype antibodies and anti-idiotype antibodies that may control immunogenic antibodies or autoreactive antibodies, but as the immune response is a polyclonal response, then elimination of a single or even few clones does not affect immune response as additional clones may still replace those eliminated.

Besides this humoral immune response, there is another form of immune response that is mediated by T lymphocytes that kill, for example infected cells and this is called cellular immune response. All molecular mechanisms of cell based immune response cannot be reviewed in the present invention but may be reviewed elsewhere (Margulies DH, 1999; Germain RN, 1999; Davis MM and Chien YH, 1999) for a review of the Major Histocompatibility Complex (MHC or HLA in humans), antigen processing and presentation and T-cell antigen receptors (TcR), respectively. Briefly, for almost every protein synthesized inside cells a fraction is degraded into peptides in intracellular organelles and peptides with affinity for a groove of HLA-I or HLA-II molecules are captured by HLA and displayed on the surface of the cell. Either T CD8 positive or T CD4 positive T lymphocytes with a T cell receptor protein complex on their surface recognize such peptides bound to an HLA and if the affinity is enough, then those T cell proliferate, secrete cytokines and/or become cytotoxic T lymphocytes that upon contact with a target cell that is producing the recognized HLA-peptide complex either secrete cytokines or have cytotoxic activity by secretion of toxic proteins like perforin and granzymes that kill the target cell.

The human body does not recognize self-proteins bound to HLA and the process to do so is called tolerance that may be central or peripheral. Most ubiquitous self-proteins are presented to HLA within thymus and T lymphocytes that recognize them are eliminated and thus, most of T lymphocytes that are produced into the thymus do not recognize peptides derived from self-proteins. For other self-proteins reactive T lymphocytes are produced into the thymus and released from primary lymphoid organs to become part of the recirculating pool of peripheral lymphocytes where they encounter antigens not found on thymus. Antigen presentation without co-stimulation renders those T lymphocytes non-responsive and this form of non-reactivity is called peripheral tolerance. Thus, the best method to predict if a peptide may be immunogenic is to search if its sequence is 100 percent shared by a known human protein because in this case the peptide is not immunogenic, but just a single change of amino acid may transform a non-immunogenic peptide into an immunogenic one. Also, for a peptide to be immunogenic it must bind with high affinity to any HLA of a subject. The process of antibody humanization consists of substitution of amino acids mainly of framework regions to use peptides that are shared between human and mouse antibodies to destroy both epitopes that may trigger an antibody response or peptides that are non-humans and may bind with high affinity to HLA to induce a cellular immune response. But antibody humanization has some limits because CDRs may not be humanized freely as they participate in antigen binding and thus not every amino acid within CDR may be changed. Even fully human antibodies may be immunogenic because the process of diversity generation results in antibodies that contain immunogenic epitopes mainly within CDRs that may have strong variability due to mutations incorporated in the normal affinity maturation.

There are two classes of Major Histocompatibility Complex (MHC), the MHC-I (MHC-I is called Human Leukocyte Antigen-I or HLA-I in humans) that binds intracellular peptides and the MHC-II or HLA-II that binds extracellular proteins. There are 3 HLA-I genes per haploid genome in each individual, and those genes are called HLA-A, HLA-B and HLA-C and also there are 3 HLA-II genes per haploid genome in each individual, and those genes are called HLA-DP, HLA-DQ and HLA-DR.

Class I (MHC-I or HLA-I) molecules bind short peptides mostly of 9 to 11 amino acids derived from intracellular processing of proteins and present them to T cells. The peptide repertoire of a specific molecule is, to a large extent, determined by the molecular structure accommodating so-called main anchor positions of the presented peptide. These HLA-I receptors are extremely polymorphic as there are about 945 different HLA-A and -B alleles, and much of the polymorphism influences the peptide-binding repertoire. Despite this polymorphism, class I molecules can be clustered into up to now 12 sets of molecules, denominated as supertypes, that bind largely overlapping peptide repertoires (for review see Sidney *et al,* 2008). There are available several in silico prediction tools useful for predicting HLA binding, for example NetMHC-4.0 (Nielsen and Andreatta, 2016; Hoof I *et al,* 2009; Nielsen *et al,* 2003) that uses A1, A2, A3, A24, A26, B7, B8, B27, B39, B44, B58 and B62 supertypes for prediction of epitopes of proteins. The frequency of each supertype is not homogeneous across human population and between Caucasians, Africans or Asiatic populations for example and may be searched in public databases for example in Allele Frequency Net Database at www.allelefrequencies.net. For example, A2 supertype is found in about 40 percent of Caucasian subjects while B39 is present on average on 5 percent of people. Thus, the probability of immunogenicity of a peptide in a Caucasian population should be 8-fold higher if the peptide is presented within A2 supertype than if presented by B39 supertype. In addition, the affinity of interaction between a peptide and HLA is important for immunogenicity but as all HLA supertypes do not have the same affinity for peptides, then for each HLA a rank of binding affinity has been determined and we have used a threshold below 2 percent of peptides as the level below which a peptide is considered a weak binder (WB) and a level below 0,5 percent as the level below which a peptide is considered a strong binder (SB). In order to develop a potential immunogenicity rank (PIR) we have assumed that each strong binder (SB) has a fourfold higher probability of being immunogenic than a weak binder (WB) and thus we have used a 25 percent probability of being immunogenic for each WB peptide. It is known that about 25 to 30 percent of in silico predicted peptides are really immunogenic but it is not easy to predict real immunogenicity using just in silico predictions as the only gold-standard is to test proteins into human subjects. That is, we know that in silico prediction of binding to HLA has many limitations because it does not predict if the peptide is processed, if it is protease-sensitive peptide and then is degraded inside granules, if it is transported to the surface of HLA molecules and more importantly if there is a potential reactive T lymphocyte to the HLA-bound peptide to elicit an immune response. Still in silico prediction is one of the best methods available today for HLA binding prediction. We have assumed average alleles frequencies described in Table 11 as shown in Example 9, for calculation of potential immunogenicity rank prediction (PIR). PIR was calculated as the sum of allele frequencies for each SB peptide plus 25 percent of the sum of allele frequencies for each WB peptide.

This PIR may be used to classify proteins according to their predicted immunogenicity. We used NetMHC-4.0 in silico prediction tool to predict SB and WB peptides for each supertype.

We then calculated PIR of different proteins for which there is known immunogenicity in humans, such as CMV pp65 that is a protein immunogenic for nearly 100 percent of human subjects. In addition, there is clinical experience with scFv based-CAR-T either using murine antibodies without humanization and humanized sequences, mainly sequences derived from anti-CD19 clone FMC63 that is used for example in Kymriah™ from Novartis. As a comparison we included epitopes that either partially or completely overlap with CDRs of FMC63 and also epitopes from tetracycline repressor, a bacterial protein that might be used for development of inducible CAR-T. PIR relative to that of CMV pp65 was calculated assuming that CMV pp65 is immunogenic for 100 percent of humans. Results of prediction indicate that PIR of CMV pp65 is of 1106, PIR of tetR is 610 and PIR of scFv anti-CD19 FMC63 clone is 397, that is a scFv has 36 percent of the immunogenicity of a highly immunogenic protein like CMV pp65. (Results are shown in Table 5 below)

But an important conclusion derived from this prediction is that while all immunogenicity derived from murine frameworks could be completely eliminated by antibody humanization, about 65 percent of potential PIR of an antibody comes from CDRs that cannot be humanized easily, that is, without destroying antigen recognition. In other words, the use of antibodies as chimeric antigen receptors has an important limitation that is due to the fact that most CDRs contain mutations that might generate HLA-I epitopes that should limit the persistence of immune cells that express CAR on their surfaces.

**Table 5:** Potential Immunogenicity Rank (PIR) of a fill scFv, CDRs on an antibody and two selected proteins of known immunogenicity

| Protein | Number of epitopes | SB epitopes | Sequence description | PIR | PIR relative to CMV pp65 |
|---|---|---|---|---|---|
| scFv-aCD19 (FMC63) | 80 | 13 | scFv (Vk-LK-Vh) | 397% | 36% |
| CDRs scFv-aCD19 (FMC63) | 47 | 10 | CDRs of FMC63 scFv (Vk-LK-Vh) | 259% | 23% |
| CDRs scFv-aCD19 (FMC63) CTL119 | 41 | 9 | CDRs of humanized FMC63 scFv (Vk-LK-Vh) | 210% | 19% |
| tetR | 79 | 27 | full protein | 610% | 55% |
| CMV pp65 | 179 | 43 | full protein | 1106% | 100% |

As there is clinical experience using scFv-based CAR-Ts in hematological malignancies and thus in order to interpret the impact of the above PIR predictions on clinical outcomes of patients, it is important to review such clinical experience using anti-CD19 scFv-based CAR-T

It is known that remissions are observed with CD19-targeted CAR T cell product like CTL019. For example, in 59 young patients with CD19 positive ALL that were treated with CTL019 CAR, 55/59 (93 percent) were in complete response at assessment 1 month after infusion and MRD negative status was achieved in 52. After a median follow-up of 12 months, 34 had ongoing complete response (Maude SL *et al*, 2016). Those patients received lymphodepleting chemotherapy with fludarabine and cyclophosphamide before CAR-T infusion and received just one infusion of CAR-T cells.

However, in a subset of patients there was limited persistence of infused CAR-T cells, which can increase the risk of relapse. Most CAR single chain variable fragment (scFv) domains, including that of CTL019, are of murine origin;

therefore, there is a possibility of immune-mediated rejection that may be mitigated by fully human or humanized CAR designs and thus CTL119 was developed, a CAR that uses a humanized version of FMC63 anti-CD19 scFv and patients were treated with such CTL119 (Maude SL et al, 2017). Two cohorts were infused with CTL119 CAR-T: 22 CAR-naive young adults with B-ALL and 16 young adults previously treated with CTL019 that relapsed. 100 percent of CAR-naive patients developed complete response (CR) at assessment after 1 month and all had minimal residual disease negative status and of those patients 2 relapsed with a CD19 positive tumor and 2 relapsed with a CD19 negative disease. In the retreatment cohort, 12 of 16 patients (75 percent) had an initial complete response and 9 of 16 (56 percent) had also B cell aplasia. Three non-responding patients had reduced CD19 expression.

Overall the above results may be interpreted in the following way: (i) the immune response against a murine CAR-T is an important cause of loss of persistence and relapse as patients in relapse when retreated with a humanized CAR-T have about a 60 percent of complete responses accompanied with B cell aplasia; (ii) the rate of initial complete responses in patients treated with humanized CAR-T may be even higher than that of patients treated with murine CAR-T as in a CAR-naïve population there was a 100 percent rate of CR while in murine CAR-T there was a 93 percent and thus it is possible that immune response against CAR-T is one of the main causes of early relapse; (iii) while humanization reduces the immunogenicity of the CAR-T there is still remaining immunogenicity because not all patients with an initial complete response after retreatment had B cell aplasia, a good marker of CAR-T activity. Thus, albeit the CAR has been humanized our PIR predicts that still CDRs may be immunogenic and thus retreatment of a patient may be considered a reinfusion of a protein that shares the non-humanized epitopes, that is, CDRs are almost shared between CTL019 and CTL119 and thus, for patients with an immune response to CDR-derived epitopes of CTL019 this immune response is amplified after reinfusion of CTL119. Thus, our PIR prediction support current clinical experience in scFv-based CAR-T were humanization helps but does not fully abrogates immunogenicity. In other words, CAR immunogenicity seems currently an underappreciated problem in CAR development in particular if multiple infusions are going to be done to at least some patients. Thus, an important finding of current invention is that CAR-T immunogenicity should be reduced at a minimum and thus, preferred mutants of CD64 of the present invention are those with the lowest possible PIR. Results are shown in Figure 16.

Those results indicate that there are three groups of CD64 mutants: those with a PIR below about 4%, those with a PIR around 10% and those with a PIR higher than about 30%, but there are no mutants with a level higher than 60 percent. Those values compare well with PIR of scFv for example, the PIR of FMC63 anti-CD19 scFv is of 397%, that is, values of mutants of CD64 are between 7 to 132-fold lower than those of scFv anti-CD19 FMC63 clone. Even humanized antibodies like scFv anti-CD19 clone FMC63 have a PIR of 210 percent, that is mutants of CD64 described in the present invention are between 4 to 70-fold less immunogenic than humanized scFv. In the methods of present invention, preferred mutants are those with the lowest PIR, that is, sequences with very low probability of being immunogenic. In particular, CAR-T based on CD64 mutants described in SEQ. ID. NO. 7 to 10 are preferred as they are probably of very low immunogenicity and such property is particularly important in several situations: (a) when the patient needs multiple infusions of the CAR-T product in order to minimize potential immune response that would limit persistence of therapy; (b) when complete lymphodepletion of patient is not recommended and thus either partial or no lymphodepletion are used before CAR-T infusion; (c) when CAR-T is integrated into CD34 positive hematological stem cells and thus and immune response against CAR-T protein may avoid long term persistence of CAR.

Even though a protein may be also immunogenic if it is recognized by antibodies, we have not considered antibody response as the most important cause of low persistence by immunogenicity and thus PIR is a rank that we have calculated using only cellular based cytotoxicity mediated by CD8 positive T lymphocytes. If an antibody recognizes a protein on the surface of a cell then CDC or ADCC may eliminate the cell that express this protein on cell membrane and in fact, this is a highly effective therapy based on monoclonal antibodies to eliminate unwanted cells like tumoral cells or B and T lymphocytes when the patient has an autoimmune response. But for an antibody-based therapy to be effective there is a threshold in both the number of surface molecules and in the serum concentration of the antibody (Beers SA *et al,* 2010). For example, Watanabe K *et al* (Watanabe K *et al,* 2015) studied the density of CD20 molecules needed for an anti-CD20 CAR-T killing activity and proliferation and compared those values with the antigen density needed for antibody-mediated lysis. They found that about 200 CD20 molecules are enough for lytic activity while about 2.000 are needed to support CAR-T proliferation and those low numbers are still 10-fold higher than those needed for a TcR and HLA-peptide interaction (Purbhoo MA *et al,* 2006) while for antibody-based cell cytotoxicity a low level should be about 10.000 molecules or higher (see for example Table II at page 2818 of Tang Y *et al,* 2007). Thus, high affinity antibodies and a target antigen density about 1000-fold higher are needed for an efficient lysis mediated by antibodies when compared with TcR and peptide-HLA-I mediated cytotoxicity and because of that, in the methods of the present invention cell-based cytotoxicity mediated by cytotoxic T CD8 positive lymphocytes that recognize peptides bound to HLA I is considered more relevant cell based than antibody mediated cytotoxicity.

One important finding of the present invention is that proteins that are designed to be expressed into cells of patients and where long-term persistence is needed, must be tested for immunogenicity, that is, PIR or equivalent ranks must be used to select best mutants in terms of immunogenicity potential. Novel CD64 mutants identified in the present invention with the lowest PIR are described in SEQ. ID. NO. 7 to 10.

Above mutants with low immunogenicity potential must be matched with best mutants identified to mitigate inhibition by unrelated immunoglobulins present in human serum that are selected from SEQ. ID. NO. 2 to 13. Mutants that match both criteria are SEQ. ID. NO. 7 to 10 and thus are the preferred mutants of CD64 identified in the present invention.

Combination of such preferred mutants of CD64 with a transmembrane of CD8 alpha without hinge and the intracellular region containing at least CD28 as a co-stimulatory domain to further mitigate inhibition by human serum and either full CD3z or a truncated form with two ITAMs as stimulation domain generates preferred CAR-T of the present invention.

This invention is best understood by the following examples.

**Example 1-.** Generation of lentiviral vectors that produce CAR-T, production of lentiviral supernatants, transduction into primary T lymphocytes and determination of expression by flow cytometry.

1-. Generation of lentiviral vectors: Lentiviral vectors 3'-LTRsyn containing MCSV Promoter were generated using G-Blocks from IDT from the backbone of pRRL-cPPT-PGK-GFP-Wsin as described by Jones *et al,* 2009 and Lizee *et al* 2003, but substituting PGK promoter by MCSV promoter and GFP for a CAR-T previously cloned and fully sequenced into pSpark™ vector using *Xho*I and *Mlu*I restriction enzymes located downstream MCSV promoter (PMCSV). The resultant vector containing pRRL-cPPT-PMCSV-Xhol-Mlul-WPRE-3'LTRsyn was called Lentigex vector and CAR sequences were designed and synthesized with only one *Xho*I site at 5'-end and one *Mlu*I site at 3'-end. Drawing of such vector is showed in Figure 3 but cPPT and WPRE are not showed for clarity.

2-. Production of lentiviral particles: To produce lentiviral supernatants 293T cells are plated for example in 175 square centimeters flasks, at 44.000 cells per square centimeter in 40 milliliters of DMEM containing 10 percent FBS medium, 24 hours before transfection and then transfected with 10 micrograms of a Lentigex vector that has inserted the CAR and 30 micrograms of a 1:1:1 mix of pLP1:pLP2:VSVG plasmid vectors using Canfast as transfection reagent, as recommended by manufacturer. After 48 hours supernatants are harvested and concentrated using Amicon centrifuge filters between 10 to 100-fold as recommended by manufacturer. Lentiviral titers are determined using Jurkat cell line. Jurkat cells were transduced, using 200.000 cells, with different dilutions of concentrated lentiviral supernatants and the percentage of positive cells is measured using specific antibodies and flow cytometry.

Titer was calculated using the following equation:

$$\text{Titer (IU/mL)} = \frac{[\text{Amount of cells}] * [\text{percent (+) cells}] * [\text{dilution fold}]}{[\text{volume (mL) of infection}]}$$

where [amount of cells] is 200.000; the [percentage of positive cells] is the percent of positive cells at a dilution of supernatant that produces about a 10 percent transduction measured by flow cytometry; [dilution fold] is the number of times that a supernatant is diluted to produce about a 10 percent transduction and [volume of transduction] is fixed to 0,5mL.

3-. Transduction of primary T lymphocytes with lentiviral particles: Transduction of T lymphocytes was made using 200.000 primary T lymphocytes isolated from peripheral blood of healthy people using human Pan T Cell Isolation Kit from Miltenyi Biotec. T lymphocytes that were activated with for example anti-CD3/anti-CD28 Dynabeads from ThermoFisher at 3:1 bead to cell ratio during 24-48 hours in X-VIVO™ 15 medium containing 10 percent of FBS and 50 units per milliliter of human interleukin-2 from Miltenyi Biotec and activated T cells are transduced by centrifugation at 1000g and 32 Celsius degrees during 60 minutes in 24-well plates containing 200.000 T lymphocytes and concentrated lentiviral supernatant with 5 micrograms per milliliter of polybrene. 24 hours after transduction medium is exchanged and cells are cultured in X-VIVO™ 15 medium containing 50 units per milliliter of human interleukin-2.

4-. Determination of expression by flow cytometry: Determination of surface expression of CAR-T was done after 4 days in culture using a primary antibody at 5 micrograms per milliliter against CD64 (Thermo Fisher) or anti-CD16 and then anti-mouse IgG-FITC at 1/60 (Sigma Aldrich).

T lymphocytes that expressed over 15 percent of CAR on their surface were used in experiments and were expanded between 9 to 11 days in X-VIVO™ 15 medium containing 50 units per milliliter of human interleukin-2 without addition of new anti-CD3/anti-CD28 Dynabeads. Beads were magnetically eliminated 24 hours before experiments.

In a first experiment as shown in Figure 4 and Tables 6A and 6B (below) we made a comparison of surface expression on primary T lymphocytes of a CD64-based CAR-T with a CD16-based CAR having each TM of CD8 alpha and 4-1BB and CD3z intracellular domains. CAR based on CD64 has no hinge while CD16 has a 14 amino acids-long hinge. Non-transduced T lymphocytes were included as controls in (A) and (C). (B) is the histogram of a CAR with CD64 extracellular domain fused to the transmembrane of CD8 alpha with 4-1BB and CD3z intracellular domains and; (D) is the histogram of a CAR with CD16 extracellular domain fused to a 14 amino acids-long hinge of human CD8 alpha and transmembrane of CD8 alpha with 4-1BB and CD3z intracellular domains. The percentage of T lymphocytes that express the CD64-based CAR on their surface is 31,6 percent in (B) while the percentage of T lymphocytes that express the CD16-based CAR on their surface is 9,8 percent in (D) (see Table 6A). If a 20 percent of transduction is selected as the criteria for a successful transduction then a 10-fold concentrated supernatant may be used for a CD64-based CAR while for a CD16-based CAR the supernatant must be more concentrated to reach the 20 percent of transduction with an increase in production costs of a CD16-based CAR relative to a CD16-based CAR.

Table 6A and 6B. Comparison of a CD16-based second generation CAR-T with an equivalent CD64-based CAR-T.

**Table 6A.** Comparison at 10-fold concentration for each CAR-T.

| CAR EC domain | Lentiviral fold concentration | Flow cytometry (+) cells | | IC domain | hinge+TM | eGFP+ |
|---|---|---|---|---|---|---|
| | | % | GeoMean | | | |
| CD64 | 10 | 31,6 | 69 | 4-1BBh+CD3zh | CD8a | 14% |
| CD16 | 10 | 9,8 | 59,4 | 4-1BBh+CD3zh | CD8a | 16% |

**Table 6B.** Comparison at 30-fold concentrated CAR (CD64-based CAR-T) and 100-fold concentrated CAR (CD16-based CAR-T)

| CAR EC domain | Lentiviral fold concentration | Flow cytometry (+) cells | | IC domain | hinge+TM | eGFP+ |
|---|---|---|---|---|---|---|
| | | % | GeoMean | | | |
| CD64 | 30 | 44 | 55,7 | 4-1BBh+CD3zh | CD8a | |
| CD16 | 100 | 36,8 | 84,1 | 4-1BBh+CD3zh | CD8a | 30% |

Above tables demonstrate that a CD64-based CAR-T expresses at higher level than an equivalent CD16-based CAR-T on the surface of primary T lymphocytes. This means that a lower amount of lentiviral supernatant is needed per patient and this reduces the overall production costs.

**Example 2:** Determination of potential amino acids of hydrophobic pocket of CD64 that are close to Leucine at 235 of IgG1.

The crystal structure deposited at Protein Data Bank under accession code 4W4O was used for estimation of distances that were measured using Jmol.

Results are showed in Table 3 below. Amino acids selected from W104, L105, A126, K130, V132 and Y176 constitute the hydrophobic pocket that accommodate L235 of IgG1 and thus, those amino acids may be changed to generate mutants of CD64 with reduced affinity for IgG to mitigate inhibition by human serum.

**Table 3:** Amino acids that form the hydrophobic pocket of CD64 and that are near Leucine at 235 in IgG1.

| distance (nm) | Leu 235 atoms | Hydrophobic pocket |
|---|---|---|
| 0,49 | Leu 235 CD1 | Trp 104 CB |
| 0,57 | Leu 235 CG1 | Leu 105 CG1 |
| 0,53 | Leu 235 CD2 | Leu 105 CD1 |

(continued)

| distance (nm) | Leu 235 atoms | Hydrophobic pocket |
|---|---|---|
| 0,76 | Leu 235 CB | Leu 105 CB |
| 0,40 | Leu 235 CD2 | Ala 126 CB |
| 0,43 | Leu 235 CD1 | Lys 130 CG |
| 0,36 | Leu 235 CD2 | Val 132 CG1 |
| 0,59 | Leu 235 CG1 | Val 132 CB |
| 0,51 | Leu 235 CB | Val 132 CB |
| 0,54 | Leu 235 CG2 | Tyr 176 CB |
| 0,45 | Leu 235 CD2 | Tyr 176 CB |

Amino acids selected from W104, L105, A126, K130, V132 and Y176 constitute the hydrophobic pocket that accommodate L235 of IgG1 and thus, those amino acids may be changed to generate mutants of CD64 with reduced affinity for IgG to mitigate inhibition by human serum.

**Example 3:** Secretion of cytokines after 24 hours of co-culture of a CD64-based CAR transduced into primary T lymphocytes and Raji cells using an anti-CD20 antibody.

Two CD64-based CAR-T were developed, transduced into primary T lymphocytes and expanded as described in Example 1. The first CAR used native CD64 as described in SEQ. ID. NO 1 fused to TMCD8 of SEQ. ID. NO. 17 and 4-1BB and CD3z as described in SEQ. ID. NO. 30 (CD64wt-Bbz) while the second CAR used CD64+V172 according to SEQ. ID. NO. 1 modified in FG loop as described in SEQ. ID. NO. 7, fused to TMCD8 of SEQ. ID. NO. 17 and CD28 and CD3z truncated with 2 ITAMs as described in SEQ. ID. NO. 21 (CD64+V172-28z).

Two compare those two CAR-T, the one with higher expression as determined by flow cytometry was adjusted to the level of the CAR with lower expression. 50.000 lymphocytes were incubated in a 96-well plate with round bottom with 50.000 Raji cells in 200 microliters of X-VIVO™15 medium with 10 percent FBS or 20 percent of human serum without IL-2 for 24 hours and using 5 micrograms per milliliter of an anti-CD20 (Rituximab clone) transiently expressed in ExpiCHO cells following recommendations of manufacturer and purified in AKTA by protein-G Sepharose (GE Healthcare). Human gamma interferon was quantified by ELISA using Mabtech kit (Cat. No. 3420). Results were normalized for 50.000 CAR-T positive cells.

Results are shown in Figure 5 and Table 7 below. Secretion of cytokines induced by a CAR comprising the extracellular region of human native (wild-type) CD64 is strongly inhibited by unrelated IgGs present in human serum and thus, a CAR based on native CD64 is not useful. But a mutated CD64 containing an insertion of Valine at 172 position results in a CAR-T that is inhibited a 20 percent after 24 hours and thus this CAR is useful for the methods of the present invention.

Table 7: Secretion of cytokines after 24h of co-culture with Raji and anti-CD20 antibody

| Secretion of cytokines after 24h of co-culture with Raji and anti-CD20 antibody | | | | | | |
|---|---|---|---|---|---|---|
| CAR-T | | 10% FBS | | 20% human serum | | % inhibition 20%HS vs 10%FBS |
| Extracellular | Intracellular | Mean | SD | Mean | SD | |
| CD64 wild type | 4-1BB-CD3z | 12.021,2 | 793,2 | 1.167,4 | 172,7 | 90,3% |
| CD64 with V172 | CD28-CD3z trunc | 11.475,2 | 593,9 | 9.131,9 | 273,8 | 20,4% |

**Example 4:** Secretion of cytokines after 24 hours of co-culture of a K130S mutant of a CD64-based CAR transduced into primary T lymphocytes and Raji cells using an anti-CD20 antibody.

This experiment was designed in the same way as Example 3 but also including no antibody and a third condition of 2,5 percent of human serum besides FBS at 10 percent and Human Serum at 20 percent.

Results are shown in Figure 8 and Table 8 below and demonstrate that an amino acid with a small lateral group like Serine when substitutes the Lysine at position 130, generates a CAR-T whose inhibition by serum may be reduced from about a 90 percent to about a 50 percent, and thus, this CAR-T is also useful for the methods of the present invention.

Table 8: Secretion of cytokines after 24h of co-culture with Raji and anti-CD20 antibody

| Secretion of cytokines after 24h of co-culture with Raji and anti-CD20 antibody | | | | | | | | | | |
|---|---|---|---|---|---|---|---|---|---|---|
| CAR-T | | Antibody | | 20% Human Serum (HS) | | 2,5% Human Serum (HS) | | 10% FBS | | percent inhibition 20%HS vs 10%FBS |
| Extracellular | Intracellular | Type | μg/mL | Mean | SD | Mean | SD | Mean | SD | |
| CD64K130S | 4-1BB-CD3z | - | 0 | 326,3 | 23,0 | 692,3 | 98,9 | 335,6 | 41,6 | - |
| CD64K130S | 4-1BB-CD3z | IgG1 anti-CD20 | 2 | 2.729,9 | 609,9 | 5.350,9 | 526,6 | 5.408,8 | 723,0 | 49,5% |

**Example 5:** Cytokine secretion induced in a proliferation assay at 3 days and 7 days of different CAR-T.

Proliferation experiments were designed in the same way of Example 3 also using the same two CAR-T, FBS at 10 percent or Human Serum at 20 percent but with the following modifications: 200.000 T lymphocytes were incubated with 200.000 Raji cells pretreated with mitomycin C in a flat-bottom of a 24 well plate in 1 mL of X-VIVO™-15 in the presence of 5 micrograms per milliliter and plate was incubated for 3 days after which viable cells were counted in a Neubauer chamber, adjusted again to 200.000 cells and new mitomycin C treated Raji and antibody was added and culture was incubated for further 4 days. After both 3 days and 7 days an aliquot of supernatant was collected and used to quantify gamma IFN and results were normalized for 200.000 CAR-T positive cells.

Results of this Example are shown in Figure 9 and Table 9 below. Those results demonstrate that in a repetitive proliferation assay the cytokine secretion of a wild-type CD64-based CAR-T is inhibited about a 90 percent after 3 days and about a 70 percent after 7 days. In contrast, the cytokine secretion of a mutated CAR-T with an insertion of Valine at 172 is inhibited about a 40 percent after 3 days and about a 50 percent after 7 days. In addition, those results demonstrate that a CAR-T containing both a mutated CD64 and CD28 intracellular co-stimulation domain has about 15-fold higher or 11-fold higher cytokine secretion after 3 days and 7 days, respectively, in the presence of 20 percent of human serum than a CAR-T that contains both a wild-type CD64 and 4-1BB intracellular co-stimulation domain. That is, CAR-T based on mutated CD64 still have inhibition by human serum but including CD28 intracellular co-stimulation domain instead of 4-1 BB, generates a CAR-T with a cytokine secretion level that is about 2-fold higher than that of a wild-type CD64-based CAR-T that contains 4-1BB as intracellular co-stimulation domain.

Table 9: Secretion of cytokines in a proliferation assay after 3 days and 7 days of co-culture with Raji and anti-CD20 antibody

| Secretion of cytokines in a proliferation assay after 3 days and 7 days of co-culture with Raji and anti- CD20 antibody | | | | | | | |
|---|---|---|---|---|---|---|---|
| CAR-T | | 10% FBS | | 20% human serum | | % inhibition 20%HS vs 10%FBS (3 days) | % inhibition 20%HS vs 10%FBS (7 days) |
| Extracellular | Intracellular | Mean (3 days) | Mean (7 days) | Mean (3 days) | Mean (7 days) | | |
| CD64 wild type | 4-1BB-CD3z | 203.284,2 | 107.109,0 | 16.902,9 | 32.648,0 | 91,7% | 69,5% |
| CD64 with V172 | CD28-CD3z trunc | 437.672,3 | 752.959,9 | 261.850,9 | 363.595,7 | 40,2% | 51,7% |

Proliferation fold was calculated after 3 days (P3d) and 7 days (P7d) by dividing the number of viable cells at the end of each cycle by 200.000. The final proliferation was P3d multiplied by P7d. Results of proliferation are shown in Figure 11.

Those results demonstrate that human serum has a profound inhibitory effect on proliferation of immune cells that express a CAR-T based on wild-type CD64 as proliferation is inhibited about 6-fold. In contrast a CAR-T based on a mutated CD64 with CD28 intracellular co-stimulation domain has 24-fold proliferation in the presence of human serum, that is, inhibition is a 38 percent when compared with the same CAR-T in absence of human serum. Proliferation is 4,3-fold higher in a mutated CD64 containing CD28 intracellular co-stimulation domain when compared with a wild type CD64 containing 4-1BB as intracellular co-stimulation domain. Those results demonstrate that CD64-based CAR-Ts are useful but they are still inhibited by human serum and such inhibition in cytokine secretion and proliferation in the presence of human serum may be mitigated and that a combination of certain mutations in CD64 extracellular domain with a CD28 intracellular co-stimulation domain generate potent CAR-Ts that proliferate and secrete cytokine under specific stimulation.

**Example 6:** Cytokine secretion induced in 24 hours coculture of either K130Y or R175A CD64-based CAR-T with different intracellular domains and using anti-CD22 in either absence or presence of Human Serum.

Two CD64-based CAR-T were developed, transduced into primary T lymphocytes and expanded as described as described in Example 1. The first CAR used CD64 as described in SEQ. ID. NO 1 modified with R175A as described in SEQ. ID. NO. 2 fused to TMCD8 of SEQ. ID. NO. 17 and CD28 and CD3z as described in SEQ. ID. NO. 20 while the second CAR used CD64 according to SEQ. ID. NO. 1 modified with K130Y in hydrophobic pocket as described in SEQ. ID. NO. 11, fused to TMCD8 of SEQ. ID. NO. 17 and 4-1BB and full CD3z as described in SEQ. ID. NO. 30.

Two compare those two CAR-T, the one with higher expression as determined by flow cytometry was adjusted to the level of the CAR with lower expression. 50.000 lymphocytes were incubated in a 96-well plate with round bottom with 50.000 Raji cells in 200 microliters of X-VIVO™15 medium with 10 percent FBS or 20 percent of human serum without IL-2 for 24 hours and using 2 micrograms per milliliter of an anti-CD22 (m971 clone) transiently expressed in ExpiCHO cells following recommendations of manufacturer and purified in AKTA by protein-G Sepharose (GE Healthcare). Human gamma interferon was quantified by ELISA using Mabtech kit (Cat. No. 3420). Results were normalized for 50.000 CAR-T positive cells.

Results of cytokine secretion after 24 hours of co-culture with Raji are shown in Figure 10 and Table 10 below using either a specific anti-CD22 antibody (left) at 2 micrograms per milliliter and no antibody (right). Fetal bovine serum at 10 percent (FBS10%) was used as a non-inhibitory serum and Human Serum at 20 percent (HS20%) that contains normal levels of IgG1, IgG3 and IgG4 were used as more physiological conditions, that is, to test if human normal serum inhibits binding of specific anti-CD22 antibody to mutated CD64-based CAR-T.

Those results demonstrate that cytokine secretion induced by a CAR-T mutated at either the FG loop (R175A) or at the hydrophobic pocket (K130Y) are still inhibited by human serum to about a 50 percent using an anti-CD22 antibody. Percent inhibition cannot not be compared with that of anti-CD20 antibody because a lower concentration of anti-CD22 was used here (2 micrograms per milliliter instead of 5 micrograms per milliliter for anti-CD20). Those results demonstrate that CAR-T developed in the methods of the present invention are also useful for antigens like CD22 with a surface density about 10 times lower than that of CD20, with a higher internalization rate than that of CD20 and using more stringent conditions like a lower amount of specific antibody at the same percent of human serum as inhibitor. Comparison of intracellular co-stimulation domains demonstrates that CD28 co-stimulation domain induces a higher cytokine secretion than 4-1BB co-stimulation domain and cytokine levels in the presence of human serum at 20 percent are about a 30 percent higher than those of 4-1BB in the absence of human serum, that is, a CAR-T based on certain mutated CD64 containing CD28 as intracellular co-stimulation domain in the presence of human serum has the same performance that a CAR-T containing 4-1BB in the absence of human serum. Those results underline the superior performance of CD28 intracellular co-stimulation domain for the development of CAR-T based on mutated CD64 that it is still inhibited by human serum.

Table 10: Secretion of cytokines after 24h of co-culture with Raji and anti-CD22 antibody

| Secretion of cytokines after 24h of co-culture with Raji and anti-CD22 antibody | | | | | | | | |
|---|---|---|---|---|---|---|---|---|
| CAR-T | | Antibody | | 20% Human Serum (HS) | | 10% FBS | | percent inhibition 20%HS vs 10%FBS |
| Extracellular | Intracellular | Type | µg/mL | Mean | SD | Mean | SD | |
| CD64R175A | CD28-CD3z | IgG1 anti-CD22 | 2 | 27.033,1 | 5.268,7 | 55.879,5 | 7.542,9 | 51,6% |
| CD64R175A | CD28-CD3z | IgG1 anti-CD22 | 0 | 1.957,4 | 39,0 | 1.317,8 | 311,5 | |
| CD64K130Y | 4-1BB-CD3z | IgG1 anti-CD22 | 2 | 9.581,5 | 1.543,7 | 20.924,3 | 992,0 | 54,2% |
| CD64K130Y | 4-1BB-CD3z | IgG1 anti-CD22 | 0 | 1.457,3 | 82,6 | 1.157,4 | 155,7 | |

**Example 7:** Cell based cytotoxicity of a native CD64-based CAR-T using a target cell and specific antibody in the presence or absence of human serum as inhibitor.

Target cells were transduced with Lentigex-PEF1a-FFLuc and a number of cells with a luminescence 5.000 to 100.000 units were used in experiments. To measure luminescence cells were dispensed in 96-well white microplates with round bottom, cells were centrifuged at 300rpm in a centrifuge for microplates for 1 minute and then 30 microliters of a mix containing NP40 at 40 percent and a Luciferase substrate mix 4-fold concentrated were dispensed in a total volume of 110 microliters, the plate was centrifuged again 1 minute at 300 rpm and incubated 2 minutes at room temperature protected from light. Luminescence was measured in an Envision (Perkin-Elmer).

A mix to measure cytotoxicity contained 50 microliters of primary T lymphocytes transduced with a CAR-T, 20 microliters of target cells and 10 microliters of antibody at a final concentration of 2 micrograms per milliliter. Control wells without effector lymphocytes were included to determine the maximal luminescence and control wells without cells were used as background. Wells without antibody were used to calculate non-specific lysis. Incubation time of the mix was selected in preliminary experiments for a non-specific lysis below 25 percent and incubation times from between 2 hours to 24 hours were tested. For each cell line the incubation time was fixed. The effector to target ratio were selected as 10 to 1; 5 to 1 and 2 to 1 and effector cells were total effector cells instead of CAR positive cells.

In a first cytotoxicity experiment described in Figure 12, 2.000 Raji-FFluc cells were used, a native-CD64-based CAR-T (CD64wt-Bbz) described in Example 3, incubation time was 24 hours and anti-CD20 (Rituximab clone) at either 5 or 20 micrograms per milliliter was included as specific antibody.

Fetal bovine serum at 10 percent (FBS10%) was used as a non-inhibitory serum and Human Serum at 20 percent (HS20%) that contains normal levels of IgG1, IgG3 and IgG4 was used as more physiological conditions, that is, human normal serum may inhibit binding of specific anti-CD20 antibody to a CD64-based CAR-T.

Lysis was calculated as a difference of 100 percent and the percent of viable Raji-FFluc cells surviving after 24 hours of coculture and non-specific lysis was calculated as a difference of 100 percent and the same coculture without the anti-CD20 antibody. Specific lysis was calculated as a difference between total lysis and non-specific lysis.

Results show that there was no difference between total lysis using a wild-type CD64-based CAR-T in absence or in the presence of human serum at 20 percent. However, in absence of human serum, non-specific lysis increased from about 17 percent to about 35 percent and as a result specific lysis was reduced in absence of human serum as inhibitor. Those results demonstrate that cytotoxicity is not a good method to discriminate between CAR-Ts like a CD64-based CAR-T that may be inhibited by human serum.

In another equivalent experiment but using anti-CD22 antibody at 2 micrograms per milliliter and either FBS at 10 percent or Human Serum at 10 percent we tested if for a target with a much lower surface density like CD22, still there is not a high inhibition of cytotoxicity by human serum.

Results are shown in Figure 13. Using this antibody against CD22 we demonstrate also that cytotoxicity is not a good method to discriminate between different CAR-T based on CD64 as there are no differences in specific lysis using a native CD64-based CAR-T in the presence or absence of human serum. When compared with cytokine secretion for example for a mutated CAR-T of Figure 10, cytotoxicity does not discriminate between CAR-T while cytokine secretion is a good method to discriminate between different CAR-T and this is true even for targets like CD22 with low expression.

**Example 8:** Cell based cytotoxicity of a mutated CD64-based CAR-T using a target cell and specific antibody in the presence or absence of human serum as inhibitor.

This experiment had the same design of Example 7 but CAR-T described in Example 6 were used. Results shown in Figure 14 are of a R175A mutated CD64-based CAR-T.

This experiment confirms that a mutated CD64-based CAR-T, in this example an R175A mutated CD64, may be used to kill tumoral cells that express CD22 on their surface. Those results confirm that also, using a mutated CD64-based CAR-T, the use of cytotoxicity is not a good method to discriminate between different CAR-T with differential inhibition by human serum. Thus, one important finding of current invention is that for development of CAR-T like a CD64-based CAR-T that may be inhibited by human serum, both proliferation and cytokine secretion may be used but cytotoxicity is not a good method to discriminate between different CAR-Ts.

Results shown in Figure 15 correspond to a K130Y mutated CD64-based CAR-T. This experiment is another example that confirms that a mutated CD64-based CAR-T, in this example an K130Y mutated CD64, may be used to kill tumoral cells that express CD22 on their surface. Those results confirm that also, using a mutated CD64-based CAR-T, the use of cytotoxicity is not a good method to discriminate between different CAR-T with differential inhibition by human serum. Thus, one important finding of current invention is that for development of CAR-T, like a CD64-based CAR-T, that may be inhibited by human serum, both proliferation and cytokine secretion may be used but cytotoxicity is not a good method to discriminate between different CAR-Ts.

**Example 9**: Prediction of immunogenic epitopes of mutated CD64 using NetMHC v4.0 and calculation of Potential Immunogenicity Rank (PIR). 12 supertypes of HLA-I were used and their frequency was assumed according to the following Table 11.

**Table 11:** Frequency of each HLA-I supertype used to calculate PIR.

| Supertype | Frequency (%) | Comment |
|---|---|---|
| A2 | 40 | Highly Frequent alleles |
| A1 | 30 | |
| B7 | 25 | |
| B8 | 22 | |
| A3 | 20 | |
| A24 | 15 | |
| B62 | 10 | Alleles of medium frequency |
| B27 | 9 | |
| B44 | 8 | |
| B58 | 7 | Alleles of low frequency |
| A26 | 6 | |
| B39 | 5 | |

Epitopes of mutated CD64 were predicted using NetMHC v4.0 in silico online prediction tool using a peptide length of 9 amino acids that is the most common length in over than 50 percent of peptides presented into HLA-I. A rank threshold for strong binding (SB) peptides has been set up at top 0,5 percent while the rank threshold for weak binding (WB) peptides has been set up at top 2,0 percent. Potential immunogenicity rank was calculated by the sum of the frequency in population of corresponding HLA I supertypes for strong binding peptides plus 25% of the sum of the frequency in population of corresponding HLA I supertypes for weak binding peptides.

Peptides with WB and SB derived from native CD64 are described in Table 12, below (FG loop peptides) and Table 21 below (Hydrophobic pocket) Peptides where the first or last amino acid is the mutated amino acid were considered as non-immunogenic if the same non-mutated peptide was presented within the same HLA-I with an affinity below 500 nanomolar. For example, peptide with SEQ. ID. NO. 35 MGKHRYTSA of wild-type CD64 is predicted to bind to HLA-B0801 with an affinity of 9,88 nanomolar (see Table 12), while mutated CD64 with insertion of Valine at 172 of SEQ. ID. NO. 7 generates a peptide with SEQ. ID. NO. 37 VGKHRYTSA, also predicted to bind to HLA-B0801 with an affinity of 109,98 nanomolar (see Table 13 below) and thus, if the peptide of native CD64 is processed and presented in HLA-B0801, as the first and last residues are considered anchoring amino acids to HLA-I, then both peptides are recognized by the same T cell receptor and thus this peptide is not immunogenic because humans have not such autoreactive T cell clones that are eliminated by either central or peripheral tolerance.

In contrast, the peptide SEQ. ID. NO. 56 KLVYNVLYY is predicted to bind to HLA-A2601 with an affinity of 5.972 nanomolar (see Table 21 below) while the mutant CD64 with K130S of SEQ. ID. NO. 15 generates a peptide SEQ. ID. NO.59 SLVYNVLYY that is also predicted to be presented within HLA-A2601 while with an affinity of 124,78 nanomolar, that is, the mutation generates an anchoring residue (a change of K to S) that increases 48-fold the affinity of peptide for HLA-A2601 and thus, this peptide is a potential immunogenic epitope. It means, that a mutation should increase immunogenicity in two ways: creating a new core region recognized by novel T lymphocytes or by increasing affinity for HLA-I and thus stabilizing antigen presentation. In general affinities over than 5.000 nanomolar were considered as non-immunogenic, but also native peptides with over than 5.000 nanomolar were considered as non-tolerogenic.

Predicted epitopes of peptides derived from mutated CD64 are described in Table 13 to 20 and Table 22 to 28.

Figure 17 describes a classification of different mutants of CD64 according to PIR. Mutants were classified into three main groups: those that have a very low PIR from 1 to 4 represented by SEQ. ID. NO. 7-10 and 16; those that have a medium PIR from about 10 to 12 represented by SEQ. ID. NO. 2, 4-6, 14 and 15 and those with a PIR of about 17 to 21 represented by SEQ. ID. NO. 3 and 11-13. Considering only PIR, mutants with the lowest value are preferred for the methods of the present invention in particular if multiple CAR-T infusions are used for a single patient.

Table 5 describes PIR of a scFv of antibody anti-CD19 FMC63, the CDRs of this antibody and of CMV pp65 protein and tetR protein. Those predictions indicate that mutants of CD64 developed in the present invention have PIRs from about 3 to 54 while a murine antibody used in current approved CAR-T anti-CD19 has a PIR of 397, that is, mutants developed in the present invention have between 7 to 132-fold lower potential immunogenicity than scFv, for example FMC63 clone anti-CD19.

Table 12. Epitopes of native FG loop of human CD64 predicted using NetMHC v4.0 with a peptide length of 9 amino acids for a peptide sequence SEQ. ID. No. 32 164-GTYHCSGMGKHRYTSAGISV-183

Description: FG loop of CD64 WT sequence

| HLA | peptide | Affinity(nM) | %Rank | BindLevel | Comment |
|---|---|---|---|---|---|
| HLA-A0101 | SEQ. ID. No. 33 | 474.08 | 0.40 | <= SB | native epitope |
| HLA-A2402 | SEQ. ID. No.34 | 824.90 | 1.00 | <= WB | native epitope |
| HLA-B0801 | SEQ. ID. No. 35 | 9.88 | 0.02 | <= SB | native epitope |

In the Table above the referred peptide sequences correspond to:

SEQ. ID. No. 33 CSGMGKHRY

SEQ. ID. No.34 RYTSAGISV

SEQ. ID. No. 35 MGKHRYTSA

Table 13. Epitopes of FG loop of human CD64 mutated with an insertion of Valine at 172 predicted using NetMHC v4.0 with a peptide length of 9 amino acids for a peptide sequence of SEQ. ID. NO. 7 164-GTYHCSGMVGKHRYTSAGISV-184

Description: insertion of V172

| HLA | peptide | Affinity(nM) | %Rank | BindLevel | Comment |
|---|---|---|---|---|---|
| HLA-B0801 | SEQ. ID. NO. 36 | 434.19 | 0.80 | <= WB | |
| HLA-B0801 | SEQ. ID. NO. 37 | 109.98 | 0.30 | <= SB | V potential anchor |

Potential Immunogenicity Rank (PIR)= 22%/4= 5,5 percent

In the Table above the referred peptide sequences correspond to:

SEQ. ID. NO. 36 MVGKHRYTS

SEQ. ID. NO. 37 VGKHRYTSA

Table 14. Epitopes of FG loop of human CD64 mutated with an insertion of Glycine at 172 predicted using NetMHC v4.0 with a peptide length of 9 amino acids for a peptide sequence of SEQ. ID. NO. 8 164-GTYHCSGMGGKHRYTSAGISV-184

Description: insertion of G172

| HLA | peptide | Affinity(nM) % | Rank | BindLevel | Comment |
|---|---|---|---|---|---|
| HLA-B0801 | SEQ. ID. NO. | 38 615.04 | 1.00 | <= WB | |
| HLA-B0801 | SEQ. ID. NO. | 39 227.39 | 0.50 | <= SB, | potential anchor |

Potential Immunogenicity Rank (PIR)= 22%/4= 5,5 percent

In the Table above the referred peptide sequence corresponds to:

SEQ. ID. NO. 38 MGGKHRYTS
SEQ. ID. NO. 39 GGKHRYTSA

**Table 15.** Epitopes of FG loop of human CD64 mutated with an insertion of Glycine at 171 predicted using NetMHC v4.0 with a peptide length of 9 amino acids for a SEQ. ID. NO. 9 164-GTYHCSGGMGKHRYTSAGISV-184

Description: insertion of G171

| HLA | peptide | Affinity(nM) | %Rank | BindLevel | Comment |
|---|---|---|---|---|---|
| HLA-A2601 | SEQ. ID. NO. 40 | 3250.74 | 1.10 | <= WB | |
| HLA-B3901 | SEQ. ID. NO. 41 | 4273.48 | 2.00 | <= WB | |

Potential Immunogenicity Rank (PIR)= (6%+5%)/4= 3,0%

In the Table above the referred peptide sequences correspond to:

SEQ. ID. NO. 40 GTYHCSGGM
SEQ. ID. NO. 41 YHCSGGMGK

**Table 16.** Epitopes of FG loop of human CD64 mutated with an insertion of Valine at 172 and Lysine at 173, Histidine at 174 and Arginine at 175 mutated to Glycine at 173, 174 and 175 predicted using NetMHC v4.0 with a peptide length of 9 amino acids for a SEQ. ID. NO. 10 164-GTYHCSGMVGGGGYTSAGISV-184

Description: insertion of V172 plus change of KHR to GGG

| HLA | peptide | Affinity(nM) | %Rank | BindLevel | Comment |
|---|---|---|---|---|---|
| HLA-B3901 | SEQ. ID. NO. 42 | 3590.04 | 1.70 | <= WB | |
| HLA-B1501 | SEQ. ID. NO. 43 | 397.58 | 1.90 | <= WB | |

Potential Immunogenicity Rank (PIR)= (5%+10%)/4=4 percent

In the Table above the referred peptide sequences correspond to:

SEQ. ID. NO. 42 YHCSGMVGG

SEQ. ID. NO. 43 SGMVGGGGY

**Table 17.** Epitopes of FG loop of human CD64 mutated with Histidine at 174 mutated to Glutamic Acid predicted using NetMHC v4.0 with a peptide length of 9 amino acids for a SEQ. ID. NO. 3 164-GTYHCSGMGKERYTSAGISV-184

Description: mutation at H174E

| HLA | peptide | Affinity(nM) | %Rank | BindLevel | Comment |
|---|---|---|---|---|---|
| HLA-A0101 | SEQ. ID. NO. 44 | 692.62 | 0.50 | <= SB | |
| HLA-B0801 | SEQ. ID. NO. 45 | 10.19 | 0.02 | <= SB | |
| HLA-B4001 | SEQ. ID. NO. 46 | 1476.87 | 1.50 | <= WB | |

Potential Immunogenicity Rank (PIR)= 30%+22%+8%/4= 54,0 percent

In the Table above the referred peptide sequences correspond to:

SEQ. ID. NO. 44 CSGMGKERY

SEQ. ID. NO. 45 MGKERYTSA

SEQ. ID. NO. 46 KERYTSAGI

**Table 18.** Epitopes of FG loop of human CD64 mutated with Arginine at 175 mutated to Alanine predicted using NetMHC v4.0 with a peptide length of 9 amino acids for a SEQ. ID. NO. 2 164-GTYHCSGMGKHAYTSAGISV-184

Description: mutation at R175A

| HLA | peptide | Affinity(nM) | %Rank | BindLevel | Comment |
|---|---|---|---|---|---|
| HLA-A0101 | SEQ. ID. NO. 47 | 311.18 | 0.30 | <= SB | |
| HLA-B0801 | SEQ. ID. NO. 48 | 971.33 | 1.40 | <= WB | |
| HLA-B3901 | SEQ. ID. NO. 49 | 684.79 | 0.60 | <= WB | |
| HLA-B3901 | SEQ. ID. NO. 50 | 704.55 | 0.60 | <= WB | |

Potential Immunogenicity Rank = 30%+(22%+5%+5%) /4= 38,0 percent

In the Table above the referred peptide sequences correspond to:

SEQ. ID. NO. 47 CSGMGKHAY
SEQ. ID. NO. 48 MGKHAYTSA
SEQ. ID. NO. 49 KHAYTSAGI
SEQ. ID. NO. 50 TSAGISVTV

**Table 19.** Epitopes of FG loop of human CD64 mutated with Lysine at 173, Histidine at 174 and Arginine at 175 mutated to Alanine at 173, 174 and 175 predicted using NetMHC v4.0 with a peptide length of 9 amino acids for a SEQ. ID. NO. 5 164-GTYHCSGMGAAAYTSAGISV-184

Description: mutation at KHR->AAA

| HLA | peptide | Affinity(nM) | %Rank | BindLevel | Comment |
|---|---|---|---|---|---|
| HLA-A0101 | SEQ. ID. NO. 51 | 59.19 | 0.10 | <= SB | |
| HLA-B3901 | SEQ. ID. NO. 52 | 332.62 | 0.40 | <= SB | |
| HLA-B1501 | SEQ. ID. NO. 51 | 241.82 | 1.40 | <= WB | |

Potential Immunogenicity Rank (PIR) = 30%+5%+10%/4= 37,0 percent

In the Table above the referred peptide sequences correspond to:

SEQ. ID. NO. 51 CSGMGAAAY

SEQ. ID. NO. 52 YHCSGMGAA

**Table** 20. Epitopes of FG loop of human CD64 mutated with Lysine at 173, Histidine at 174 and Arginine at 175 mutated to Glycine at 173, 174 and 175 predicted using NetMHC v4.0 with a peptide length of 9 amino acids for a SEQ. ID. NO. 6 164-GTYHCSGMGGGGYTSAGISV-184

Description: mutation at KHR->GGG

| HLA | peptide | Affinity(nM) | %Rank | BindLevel | Comment |
|---|---|---|---|---|---|
| HLA-A0101 | SEQ. ID. NO. 53 | 205.13 | 0.25 | <= SB | |
| HLA-B3901 | SEQ. ID. NO. 54 | 704.55 | 0.60 | <= WB | |

Potential Immunogenicity Rank (PIR)= 30%+10%/4= 32,0 percent

In the Table above the referred peptide sequences correspond to:

SEQ. ID. NO. 53 CSGMGGGGY

SEQ. ID. NO. 54 TSAGISVTV

**Table 21.** Epitopes of peptides around K130 that contributes to formation of the hydrophobic pocket of human CD64 predicted using NetMHC v4.0 with a peptide length of 9 amino acids for native CD64 with a peptide sequence SEQ. ID. NO. 55 121-ALRCHAWKDKLVYNVLYYRN-140.

Description: CD64K130 wild type

| HLA | peptide | Affinity(nM) | %Rank | BindLevel | Comment |
|---|---|---|---|---|---|
| HLA-A0101 | SEQ. ID. NO. 56 | 5910.62 | 1.80 | <= WB, | K potential anchor |
| HLA-A0301 | SEQ. ID. NO. 56 | 65.17 | 0.30 | <= SB, | K potential anchor |
| HLA-A2601 | SEQ. ID. NO. 56 | 5972.59 | 1.80 | <= WB, | K potential anchor |
| HLA-B5801 | SEQ. ID. NO. 56 | 296.14 | 0.70 | <= WB, | K potential anchor |
| HLA-B1501 | SEQ. ID. NO. 56 | 103.39 | 0.80 | <= WB, | K potential anchor |

In the Table above the referred peptide sequence corresponds to:
SEQ. ID. NO. 56 KLVYNVLYY

**Table 22.** Epitopes of mutated CD64 mutated at Lysine at 130 to Serine predicted using NetMHC v4.0 with a peptide length of 9 amino acids for SEQ. ID. NO. 15 121-ALRCHAWKDSLVYNVLYYRN-140.

Description: CD64K130S

| HLA | peptide | Affinity(nM) | %Rank | BindLevel | Comment |
|---|---|---|---|---|---|
| HLA-A0101 | SEQ. ID. NO. 57 | 6479.57 | 1.90 | <= WB | very high Kd |
| HLA-A0101 | SEQ. ID. NO. 58 | 1398.75 | 0.70 | <= WB | |
| HLA-A0101 | SEQ. ID. NO. 59 | 2868.67 | 1.10 | <= WB | |
| HLA-A0301 | SEQ. ID. NO. 59 | 211.45 | 0.80 | <= WB, | potential anchor |
| HLA-A2601 | SEQ. ID. NO. 58 | 6226.77 | 1.90 | <= WB | very high Kd |
| HLA-A2601 | SEQ. ID. NO. 59 | 124.78 | 0.12 | <= SB | |
| HLA-B3901 | SEQ. ID. NO. 60 | 287.25 | 0.40 | <= SB | |
| HLA-B5801 | SEQ. ID. NO. 57 | 86.75 | 0.40 | <= SB | |
| HLA-B1501 | SEQ. ID. NO. 57 | 99.32 | 0.80 | <= WB | |
| HLA-B1501 | SEQ. ID. NO. 59 | 115.40 | 0.90 | <= WB, | potential anchor |

Potential Immunogenicity Rank (PIR)= 6%+5%+7%+ (30%+30% +10%)/4= 35,5%

In the Table above the referred peptide sequences correspond to:

SEQ. ID. NO. 57 HAWKDSLVY

SEQ. ID. NO. 58 DSLVYNVLY

SEQ. ID. NO. 59 SLVYNVLYY

SEQ. ID. NO. 60 WKDSLVYNV

**Table 23.** Epitopes of mutated CD64 mutated at Lysine at 130 to Glycine predicted using NetMHC v4.0 with a peptide length of 9 amino acids for SEQ. ID. NO. 16 121-ALRCHAWKDGLVYNVLYYRN-140

Description: CD64K130G

| HLA | peptide | Affinity(nM) | %Rank | BindLevel | Comment |
|---|---|---|---|---|---|
| HLA-A0101 | SEQ. ID. NO. 61 | 4403.88 | 1.40 | <= WB, | potential anchor |
| HLA-A0301 | SEQ. ID. NO. 61 | 384.84 | 1.10 | <= WB, | potential anchor |
| HLA-A2601 | SEQ. ID. NO. 61 | 1300.40 | 0.60 | <= WB, | 6% |
| HLA-B3901 | SEQ. ID. NO. 62 | 212.72 | 0.30 | <= SB, | 5% |
| HLA-B5801 | SEQ. ID. NO. 63 | 265.52 | 0.70 | <= WB, | 7% |
| HLA-B1501 | SEQ. ID. NO. 63 | 102.77 | 0.80 | <= WB, | 10% |

(continued)

Description: CD64K130G

| HLA | peptide | Affinity(nM) | %Rank | BindLevel | Comment |
|---|---|---|---|---|---|
| HLA-B1501 | SEQ. ID. NO. 61 | 201.22 | 1.30 | <= WB, | potential anchor |

Potential Immunogenicity Rank (PIR)= 5%+ (6%+7%+10%)/4= 11 percent

In the Table above the referred peptide sequences correspond to:

SEQ. ID. NO. 61 GLVYNVLYY

SEQ. ID. NO. 62 WKDGLVYNV

SEQ. ID. NO. 63 HAWKDGLVY

**Table 24.** Epitopes of mutated CD64 mutated at Lysine at 130 to Alanine predicted using NetMHC v4.0 with a peptide length of 9 amino acids for SEQ. ID. NO. 14 121-ALRCHAWKDALVYNVLYYRN-140

Description: CD64K130A

| HLA | peptide | Affinity(nM) | %Rank | BindLevel | Comment |
|---|---|---|---|---|---|
| HLA-A0101 | SEQ. ID. NO. 64 | 2912.71 | 1.10 | <= WB, | 30% |
| HLA-A0301 | SEQ. ID. NO. 64 | 189.24 | 0.70 | <= WB, | potential anchor |
| HLA-A2601 | SEQ. ID. NO. 65 | 6594.29 | 2.00 | <= WB, | very high Kd |
| HLA-A2601 | SEQ. ID. NO. 64 | 798.46 | 0.40 | <= SB, | 6% |
| HLA-B3901 | SEQ. ID. NO. 66 | 2294.06 | 1.30 | <= WB, | 5% |
| HLA-B3901 | SEQ. ID. NO. 67 | 247.73 | 0.40 | <= SB, | 5% |
| HLA-B5801 | SEQ. ID. NO. 68 | 74.92 | 0.40 | <= SB, | 7% |
| HLA-B1501 | SEQ. ID. NO. 68 | 278.95 | 1.60 | <= WB, | 10% |
| HLA-B1501 | SEQ. ID. NO. 64 | 101.23 | 0.80 | <= WB, | potential anchor |

Potential Immunogenicity Rank (PIR)= 6%+5%+7%+ (30%+5%+10%)/4= 29 percent

In the Table above the referred peptide sequences correspond to:

SEQ. ID. NO. 64 ALVYNVLYY

SEQ. ID. NO. 65 DALVYNVLY

SEQ. ID. NO. 66 RCHAWKDAL

SEQ. ID. NO. 67 WKDALVYNV

SEQ. ID. NO. 68 HAWKDALVY

**Table 25.** Epitopes of mutated CD64 mutated at Lysine at 130 to Tryptophan predicted using NetMHC v4.0 with a peptide length of 9 amino acids for SEQ. ID. NO. 13 121-ALRCHAWKDWLVYNVLYYRN-140

Description: CD64K130W

| HLA | peptide | Affinity(nM) | %Rank | BindLevel | Comment |
|---|---|---|---|---|---|
| HLA-A0101 | SEQ. ID. NO. 69 | 5451.99 | 1.70 | <= WB | |
| HLA-A0101 | SEQ. ID. NO. 70 | 674.14 | 0.50 | <= SB | |
| HLA-A2601 | SEQ. ID. NO. 70 | 334.47 | 0.25 | <= SB | |
| HLA-B3901 | SEQ. ID. NO. 71 | 2372.08 | 1.30 | <= WB | |
| HLA-B3901 | SEQ. ID. NO. 72 | 156.36 | 0.25 | <= SB | |

(continued)

Description: CD64K130W

| HLA | peptide | Affinity(nM) | %Rank | BindLevel | Comment |
|---|---|---|---|---|---|
| HLA-B5801 | SEQ. ID. NO. 69 | 206.27 | 0.60 | <= WB | |
| HLA-B1501 | SEQ. ID. NO. 69 | 284.13 | 1.60 | <= WB | |
| HLA-B1501 | SEQ. ID. NO. 70 | 128.97 | 1.00 | <= WB, | potential anchor |

Potential Immunogenicity Rank (PIR)= 30%+6%+5%+ (30%+5%+ 7%+ 10%)/4= 54 percent

In the Table above the referred peptide sequences correspond to:

SEQ. ID. NO. 69 HAWKDWLVY

SEQ. ID. NO. 70 WLVYNVLYY

SEQ. ID. NO. 71 CHAWKDWLV

SEQ. ID. NO. 72 WKDWLVYNV

**Table 26.** Epitopes of mutated CD64 mutated at Lysine at 130 to Tyrosine predicted using NetMHC v4.0 with a peptide length of 9 amino acids for SEQ. ID. NO. 11 121-ALRCHAWKDYLVYNVLYYRN-140.

Description: CD64K130Y

| HLA | peptide | Affinity(nM) | %Rank | BindLevel | Comment |
|---|---|---|---|---|---|
| HLA-A0101 | SEQ. ID. NO. 73 | 433.00 | 0.40 | <= SB | |
| HLA-A0301 | SEQ. ID. NO. 73 | 582.80 | 1.50 | <= WB, | potential anchor |
| HLA-A2601 | SEQ. ID. NO. 74 | 6466.89 | 2.00 | <= WB, | very high Kd |
| HLA-A2601 | SEQ. ID. NO. 73 | 40.43 | 0.06 | <= SB | |
| HLA-B3901 | SEQ. ID. NO. 75 | 2075.71 | 1.20 | <= WB | |
| HLA-B3901 | SEQ. ID. NO. 76 | 405.87 | 0.50 | <= SB | |
| HLA-B5801 | SEQ. ID. NO. 74 | 121.83 | 0.50 | <= SB | |
| HLA-B1501 | SEQ. ID. NO. 74 | 234.13 | 1.40 | <= WB | |
| HLA-B1501 | SEQ. ID. NO. 73 | 42.75 | 0.40 | <= SB, | potential anchor |

Potential Immunogenicity Rank (PIR)= 30%+6%+5%+7%+ (5%+ 10%)/4= 52%

In the Table above the referred peptide sequences correspond to:

SEQ. ID. NO. 73 YLVYNVLYY

SEQ. ID. NO. 74 HAWKDYLVY

SEQ. ID. NO. 75 CHAWKDYLV

SEQ. ID. NO. 76 WKDYLVYNV

**Table 27.** Epitopes of mutated CD64 mutated at Arginine at 175 to Glycine predicted using NetMHC v4.0 with a peptide length of 9 amino acids for SEQ. ID. NO. 4 164-GTYHCSGMGKHGYTSAGISV-183

Description: mutation at R175G

| HLA | peptide | Affinity(nM) | %Rank | BindLevel | Comment |
|---|---|---|---|---|---|
| HLA-A0101 | SEQ. ID. NO. 77 | 788.54 | 0.50 | <= SB | |
| HLA-B0801 | SEQ. ID. NO. 78 | 662.94 | 1.10 | <= WB | |

Potential Immunogenicity Rank (PIR)= 30%+22%/4= 37 percent

In the Table above the referred peptide sequences correspond to:

SEQ. ID. NO. 77 CSGMGKHGY
SEQ. ID. NO. 78 MGKHGYTSA

**Table 28.** Epitopes of mutated CD64 mutated at Lysine at 130 to Phenylalanine predicted using NetMHC v4.0 with a peptide length of 9 amino acids for SEQ. ID. NO. 12 121- ALRCHAWKDFLVYNVLYYRN-140

Description: mutation at K130F

| HLA | peptide | Affinity(nM) | %Rank | BindLevel | Comment |
|---|---|---|---|---|---|
| HLA-A0101 | SEQ. ID. NO. 79 | 535.65 | 0.40 | <= SB, | 30% |
| HLA-A0301 | SEQ. ID. NO. 79 | 758.56 | 1.70 | <= WB | potential anchor |
| HLA-A0301 | SEQ. ID. NO. 80 | 54.36 | 0.25 | <= SB, | 20% |
| HLA-A2601 | SEQ. ID. NO. 79 | 44.21 | 0.06 | <= SB, | 6% |
| HLA-B3901 | SEQ. ID. NO. 81 | 4194.46 | 2.00 | <= WB, | 5% |
| HLA-B3901 | SEQ. ID. NO. 82 | 869.24 | 0.70 | <= WB, | 5% |
| HLA-B5801 | SEQ. ID. NO. 83 | 217.31 | 0.60 | <= WB, | 7% |
| HLA-B1501 | SEQ. ID. NO. 83 | 68.18 | 0.60 | <= WB, | 10% |
| HLA-B1501 | SEQ. ID. NO. 79 | 77.97 | 0.70 | <= WB | potential anchor |

Potential Immunogenicity Rank (PIR)= 30%+20%+6%+(5%+5%+ 7%+10%)/4=63 percent

In the Table above the referred peptide sequences correspond to:

SEQ. ID. NO. 79 FLVYNVLYY
SEQ. ID. NO. 80 LVYNVLYYR
SEQ. ID. NO. 81 CHAWKDFLV
SEQ. ID. NO. 82 WKDFLVYNV
SEQ. ID. NO. 83 HAWKDFLVY

REFERENCES

**1.** Eshhar Z et al (1993) PNAS 90: 720-724.
**2.** Brocker T & Karjalainen K (1995) J. Exp. Med. 181: 1653-1659.
**3.** Brocker T (2000) Blood 96: 1999-2001.
**4.** Smyth MJ et al (1994) J. Leukoc. Biol. 55: 514-522.
**5.** Kershaw MH et al (1996) J. Leukoc. Biol. 60: 721-728.
**6.** Teng MWL et al (2006) Human Gene Ther. 17: 1134-1143.
**7.** Clemenceau B et al (2006) Blood 107 (12): 4669-4677.
**8.** Cartron G et al (2002) Blood 99:754-758.
**9.** Alvarez-Vallina L & Hawkins RE (1996) Eur. J. Immunol. 26: 2304.
**10.** Finney HM et al (1998) J. Immunol. 161:2791-2797.
**11.** Hombach A et al (2001) J. Immunol. 167: 6123-6131.
**12.** Haynes NM et al (2001) J. Immunol. 166: 182-187.
**13.** Campana D & Kudo K (2017) European Patent EP3057986.
**14.** Imai C et al (2004) Leukemia 18: 676-684.
**15.** Guest RD et al (2005) J. Immunother. 28 (3):203-211.
**16.** Qin L et al, (2017) J. Hematol. Oncol. 10 (1): 68.
**17.** Ying Z et al (2019) Nat. Med. 25: 947-953.
**18.** Rodgers DT et al (2016) PNAS 113 (4): E459-E468.
**19.** Asaoka Y et al (2013) Mol. Immunol. 54: 403-407.
**20.** Jonnalagadda M et al (2015) Mol Ther. 23 (4): 757-768.
**21.** Watanabe N et al (2016) Oncoimmunol. 5 (12): e1253656.

22. Watanabe K et al (2015) J. Immunol. 194: 911-920.

23. Jager U et al (2012) Haematologica 97 (09): 1431-1438.

24. Schneider D et al (2017) J. Immunother. Cancer 5: 42.

25. Haynes NM et al (2002) Blood 100: 3155-3163.

26. Finney HM et al (2004) J. Immunol. 172: 104-113.

27. 27-. Milone MC et al (2009) Mol. Ther. 17 (8): 1453-1464.

28. Long AH et al (2015) Nat. Med. 21 (6): 581-590.

29. Zah E et al (2016) Cancer Immunol. Res. 4 (6); 498-508.

30. Grada Z et al (2013) Mol. Ther. Nucleic Acids 2: e105.

31. Bruhns P et al (2009) Blood 113: 3716-3725.

32. Van der Poel CE et al (2011) J. Immunol. 186: 2699-2704.

33. Lu J et al (2011) J. Biol. Chem. 286 (47): 40608-40613.

34. Hulett MD et al (1993) Eur J. Immunol. 23: 640-645.

35. Lu J et al (2015) PNAS 112 (3): 833-838.

36. Teng MWL et al (2006) Human Gene Ther. 17: 1134-1143.

37. Kiyoshi M et al (2015) Nat. Commun. 6: 6866.

38. Frazer JK & Capra JD (1999) Fundamental Immunology, Fourth Edition, ed. WE Paul, Lippincott-Raven Publishers, Chapter 3 Immunoglobulins: Structure and Function.

39. Margulies DH (1999) Fundamental Immunology, Fourth Edition, ed. WE Paul, Lippincott-Raven Publishers, Chapter 8.

40. Germain RN (1999) Fundamental Immunology, Fourth Edition, ed. WE Paul, Lippincott-Raven Publishers, Chapter 9.

41. Davis MM & Chien YH (1999) Fundamental Immunology, Fourth Edition, ed. WE Paul, Lippincott-Raven Publishers, Chapter 10.

42. Sidney J et al (2008) BMC Immunol. 9: 1

43. Nielsen M & Andreatta M (2016) Genome Medicine 8: 33

44. Nielsen M et al (2003) Protein Sci. 12: 1007-1017.

45. Maude SL et al. (2016) J. Clin. Oncol. 34(15): 3011-3011.

46. Maude SL et al (2017) Blood 130 (Supplement 1): 1319.

47. Purbhoo MA et al (2006) J. Immunol. 176: 7308-7316.

48. Tang Y et al (2007) J. Immunol. 179: 2815-2823.

49. Jones S et al (2009) Hum. Gene Ther. 20: 630-640.

50. Lizee G et al (2003) Hum. Gene Ther. 14 (6): 497-507.

51. Beers SA et al (2010) Blood 115 (25): 5191-5201.

52. Hoof I et al (2009) Immunogenetics 61: 1-13

**Claims**

1. A chimeric antigen receptor (CAR) that comprises:

   a) the mutated extracellular domain of a high affinity Fc gamma receptor (mutant CD64);
   b) a transmembrane domain
   c) an intracellular signal transduction domain,

   wherein the mutated extracellular domain of the high affinity Fc gamma receptor of (a) has reduced affinity of from about 7 fold to about 25 fold for native Fc of human IgG1 relative to native extracellular domain of the high affinity Fc gamma receptor and in a cytokine secretion assay the interferon-gamma secretion of the coculture is at least 40 percent of interferon-gamma secretion relative to the same co-culture when the 10 percent of fetal bovine serum is substituted by 20 percent of human serum, such coculture is of about 24 h and comprises: (1) a lymphocyte that express a chimeric antigen receptor (CAR) that uses such mutated extracellular domain of the high affinity Fc gamma receptor (CD64); (2) a target cell that expresses on the cell surface an antigen specifically recognized by an antibody; (3) a specific antibody for the target antigen of (2) used in the coculture at a concentration up to 5 micrograms per milliliter and (4) human serum at a final concentration in the coculture of 20 percent in volume used as an inhibitor of the reaction of the lymphocyte of (1) with a target cell of (2) using an antibody of (3).

2. A chimeric antigen receptor (CAR) according to claim 1 wherein the mutated extracellular domain of the high affinity Fc gamma receptor (CD64) comprises at least a mutation in the FG loop of the receptor that is located between Glycine at position 164 to Valine at position 183 according to CD64 described in SEQ. ID. NO. 1.

3. A chimeric antigen receptor (CAR) according to claim 2 wherein the mutation of CD64 at FG loop is made by substitutions of amino acids at positions between Cysteine at 168 to Glycine at 180 and/or by an insertion of an amino acid between Cysteine at 168 and Lysine at 173.

4. A chimeric antigen receptor (CAR) according to claim 3 wherein the mutated CD64 consist of CD64 mutated at FG loop selected from the amino acid sequences of SEQ. ID. NO. 2 to SEQ.ID. NO 10.

5. A chimeric antigen receptor (CAR) according to claim 1 wherein the mutated extracellular domain of the high affinity Fc gamma receptor (CD64) comprises at least a mutation in one of the amino acids that form part of the hydrophobic pocket that interacts with Leucine 235 of human IgG1.

6. A chimeric antigen receptor (CAR) according to claim 5 wherein the extracellular domain of the high affinity Fc gamma receptor (CD64) mutated in at least one amino acid that form part of the hydrophobic pocket that interacts with Leucine 235 of human IgG1 includes a mutation of amino acids that is selected from a group consisting of W104, L105, A126, W127, K130, V132 and Y176.

7. A chimeric antigen receptor (CAR) according to claim 6 wherein the mutated amino acid in the hydrophobic pocket of CD64 is K130.

8. A chimeric antigen receptor (CAR) according to claim 7 wherein mutated K130 amino acid in the hydrophobic pocket of CD64 is substituted with either an amino acid with a small lateral group selected from Serine, Glycine or Alanine with SEQ. ID. NO. 14 to SEQ. ID. NO. 16 or with a bulky amino acid selected from Y, F or W with SEQ. ID. NO. 11 to SEQ. ID. NO. 13.

9. A chimeric antigen receptor (CAR) according to claims 1 to 8 wherein the Fc fusion protein that binds to the CAR is selected from either a human IgG1 or a human IgG4 that has a mutation at F234L for improved binding to human CD64 and those IgG recognize a target antigen on the surface of a target cell.

10. A chimeric antigen receptor (CAR) according to claims 1 to 8 wherein the Fc fusion protein is selected from human Fc gamma 1 or human Fc gamma 4 mutated at F234L and the target recognition domain is selected from a group consisting of a scFv, an affibody and ankyrin, a fynomer, a nanobody, a ligand to a surface receptor or a scTCR.

11. A chimeric antigen receptor (CAR) according to claims 1 to 8 wherein such CAR has no hinge to join the mutated CD64 alpha chain to the transmembrane and intracellular stimulation domain and such transmembrane domain is selected from the group consisting of human CD28, human CD32 or human CD8 alpha.

**12.** A chimeric antigen receptor (CAR) according to claim 11 wherein preferred monomeric transmembrane domain is selected from human CD8 alpha with either SEQ. ID. NO. 17 or SEQ. ID. NO. 18.

**13.** A chimeric antigen receptor (CAR) according to claims 11 and 12 wherein such CAR has an intracellular co-stimulation domain that contains an intracellular region selected from a group consisting of that of CD28, 4-1BB, OX-40, DAP10 or CD27.

**14.** A chimeric antigen receptor (CAR) according to claims 11 or 12 wherein such CAR has an intracellular co-stimulation domain derived from human CD28 with SEQ. ID. NO. 19.

**15.** A chimeric antigen receptor (CAR) according to claims 13 or 14 a wherein such CAR has an intracellular stimulation domain selected from a group consisting of: (a) CD28 intracellular domain fused to either the whole CD3z intracellular region with amino acid sequence SEQ. ID. NO. 20 or a truncated CD3z with amino acid sequence SEQ. ID. NO. 21; (b) CD28 intracellular co-stimulation domain fused to a second intracellular co-stimulation domain selected from 4-1BB, OX-40 or ICOS and to either the whole CD3z intracellular region with amino acid sequence or a truncated CD3z with two ITAMs with amino acids sequences from SEQ. ID. NO. 22 to SEQ. ID. NO. 27.

**16.** A chimeric antigen receptor (CAR) such CAR has the following structure: CD64X-TMCD8-Z wherein

> i. CD64X is a human CD64 extracellular domain of SEQ. ID. NO. 1 that contains at least one mutation wherein such mutations are essentially non-immunogenic for HLA I, i.e. using NetMHC v4.0 in silico prediction tool, peptides containing such mutations have only weak binding peptides as defined by peptides that are above 0,5 percent of rank of binding for each HLA I, such mutation is made by an insertion of a single amino acid between Cysteine at 168 and Lysine at 173 of SEQ. ID. NO. 1 and amino acid insertion is selected from any of the following sequences: SEQ. ID. NO. 7 to SEQ. ID. NO. 10
> ii. TMCD8 is the transmembrane domain derived from human CD8 alpha with either sequence SEQ. ID. NO 17 or sequence SEQ. ID. NO. 18.
> iii. Z is selected from: (a) Human CD28 intracellular co-stimulation domain fused with a linker of two Glycine amino acids (GG), used to reduce the HLA I immunogenicity of the joining peptide, to either full or truncated human CD3z intracellular stimulation domain as described in sequences SEQ. ID. NO. 20 and SEQ. ID. NO. 21; (b) Human 4-1BB intracellular co-stimulation domain inserted between CD28 intracellular co-stimulation domain and CD3z intracellular stimulation domain as described in sequences SEQ. ID. NO. 22 and SEQ. ID. NO. 25; (c) Human OX40 intracellular co-stimulation domain inserted between CD28 intracellular co-stimulation domain and CD3z intracellular stimulation domain as described in sequences SEQ. ID. NO. 23 and SEQ. ID. NO. 26 or (d) Human ICOS intracellular co-stimulation domain inserted between CD28 intracellular co-stimulation domain and CD3z intracellular stimulation domain as described in sequences SEQ. ID. NO. 24 and SEQ. ID. NO. 27.

**17.** The nucleic acid sequences encoding chimeric antigen receptors according to any preceding claim for engineering of an immune cell.

**18.** Vectors comprising any of the nucleic acid sequences of claim 17 wherein such vector is selected from the group consisting of a retroviral vector, a lentiviral vector or a transposon and this vector is used for transduction or transfection or electroporation of an immune cell selected from a group consisting of an autologous T lymphocyte, an allogenic T lymphocyte, an autologous primary NK cell, an allogenic primary NK cell or an allogenic NK cell line.

**19.** An engineered immune cell that expresses at least one of the chimeric antigen receptors of claims 1 to 16 for use in the treatment of a disease selected from a tumor, an infectious disease or an autoimmune disease wherein such treatment comprises either a single infusion or multiple infusions of engineered immune cells that express the chimeric antigen receptors.

**20.** A pharmaceutical composition comprising an engineered immune cell according to claim 19 and a pharmaceutically acceptable carrier or excipient.

**21.** A chimeric antigen receptor (CAR) according to claims 1 to 16 for use with a target antigen, wherein such target antigen is selected from a group comprising: BCMA, CAIX, CA125, CD5, CD6, CD19, CD20, CD21, CD22, CD23, CD27L, CD30, CD33, CD34 CD38, CD40, CD44, CD47, CD54, CD55, CD56, CD70, CD74, CD79a, CD79b, CD80, CD117, CD123, CD138, CD151, CD171, CD200, CEA, CEACAM5, cG250, CLL1, CS1, CTLA4, CXCR4, cytokeratin, EGFR, EpCAM, EphA2, EphB4, FOLR1, FRalpha, fibronectin, GD2, GD3,GPC3, GPRC5D, HER2, IGF-1R, IL13Ra2 (CD213a2), kappa light chain, Lewis Y, LIV, L1CAM, LGR5, LPAR, melanin, mesothelin, MUC1, MUC16, nectin, PSMA, TAG72 and 5T4.

22. A kit of parts that comprises:

(a) an immune cell that expresses a chimeric antigen receptor according to any of claims 1 to 16 with an acceptable vehicle;
(b) a composition comprising a Fc fusion protein or a human native IgG1 or a human IgG4 mutated at F234L for binding to the mutated CD64 of the CAR expressed on the surface of the immune cell of (a), together with an acceptable vehicle, and
(c) instructions of use.

## FIG. 1

A

FcγRI (CD64)

Alpha chain (α)

Membrane

Gamma chain (γ)
(disulphide linked
homodimer)

B

CD16

Alpha chain (α)

Membrane

Gamma chain (γ)
(disulphide linked
homodimer)

## FIG. 2

CD64 Alpha
chain (α)

Membrane ⎫ transmembrane
⎬
⎭

Intracellular
CD28 region

Intracellular
CD3z region,
with 3 ITAMs

FIG.3

5´-LTR      (1)      (2)   (3) (4) (5)     3´-LTRsyn

FIG.4

## FIG. 5

## FIG. 6

## FIG. 7

## FIG. 8

## FIG. 9

## FIG. 10

## FIG. 11

## FIG. 12

## FIG. 13

## FIG. 14

**FIG. 15**

| Peptide or protein | SEQ ID NO | Total Binders | SB Binders | PEPTIDE SEQUENCE | PIR | PIR Relative to CMV pp65 | PIR relative to SEQ. ID. NO. 9 |
|---|---|---|---|---|---|---|---|
| CD64 R175A | 2 | 4 | 1 | 164-GTYHCSGMGKH<u>A</u> | 38,0% | 3,44% | 12,7 |
| CD64 H174E | 3 | 3 | 2 | 164-GTYHCSGMGK<u>E</u> | 54,0% | 4,88% | 18,0 |
| CD64 R175G | 4 | 4 | 1 | 164-GTYHCSGMGKHGYTSAGISV-183 | 38,0% | 3,44% | 12,7 |
| CD64 K130Y | 11 | 6 | 4 | 121-ALRCHAWKD<u>Y</u> | 52,0% | 4,70% | 17,3 |
| CD64 K130W | 13 | 7 | 3 | 121-ALRCHAWKD<u>Y</u> | 54,0% | 4,88% | 18,0 |
| CD64 K130S | 15 | 6 | 2 | 121-ALRCHAWKDSLVYNVLYYRN-140 | 35,5% | 3,21% | 11,8 |
| CD64 K130G | 16 | 4 | 1 | 121-ALRCHAWKD<u>Y</u>LVYNVLYYRN-140 | 11,0% | 0,99% | 3,7 |
| CD64 K130A | 14 | 6 | 3 | 121-ALRCHAWKDALVYNVLYYRN-140 | 35,5% | 3,21% | 11,8 |
| CD64 KHR->AAA | 5 | 3 | 2 | 164-GTYHCSGMG<u>AAA</u> | 29,0% | 2,62% | 9,7 |
| CD64 KHR->GGG | 6 | 2 | 1 | 164-GTYHCSGMG<u>GGG</u> | 32,0% | 2,89% | 10,7 |
| CD64+V172 | 7 | 1 | 0 | 164-GTYHCSGM<u>V</u> | 5,5% | 0,50% | 1,8 |
| CD64+V172 KHR->GGG | 10 | 1 | 0 | 164-GTYHCSGM<u>V</u> ___ | 3,0% | 0,27% | 1,0 |
| CD64+G171 | 9 | 2 | 0 | 164-GTYHCSG<u>G</u>MGKHRYTSAGISV-184 | 3,0% | 0,27% | 1,0 |
| CD64+G172 | 8 | 1 | 0 | 164-GTYHCSGMG<u>G</u>KHRYTSAGISV-184 | 5,5% | 0,50% | 1,8 |
| tetR | | 79 | 27 | full protein | 610% | 55,14% | 203,3 |
| CMV pp65 | | 179 | 43 | full protein | 1106% | 100% | 368,8 |

**FIG. 16**

| Peptide or protein | SEQ ID NO | Total Binders | SB Binders | PEPTIDE SEQUENCE | PIR | PIR Relative to CMV pp65 | PIR relative to SEQ. ID. NO. 9 |
|---|---|---|---|---|---|---|---|
| CD64 R175A | 2 | 4 | 1 | 164-GTYHCSGMGKHA | 38,0% | 3,44% | 12,7 |
| CD64 H174E | 3 | 3 | 2 | 164-GTYHCSGMGKE | 54,0% | 4,88% | 18,0 |
| CD64 R175G | 4 | 4 | 1 | 164-GTYHCSGMGKHGYTSAGISV-183 | 38,0% | 3,44% | 12,7 |
| CD64 K130Y | 11 | 6 | 4 | 121-ALRCHAWKDY | 52,0% | 4,70% | 17,3 |
| CD64 K130W | 13 | 7 | 3 | 121-ALRCHAWKDY | 54,0% | 4,88% | 18,0 |
| CD64 K130S | 15 | 6 | 2 | 121-ALRCHAWKDSLVYNVLYYRN-140 | 35,5% | 3,21% | 11,8 |
| CD64 K130G | 16 | 4 | 1 | 121-ALRCHAWKDYLVYNVLYYRN-140 | 11,0% | 0,99% | 3,7 |
| CD64 K130A | 14 | 6 | 3 | 121-ALRCHAWKDALVYNVLYYRN-140 | 35,5% | 3,21% | 11,8 |
| CD64 KHR->AAA | 5 | 3 | 2 | 164-GTYHCSGMGAAA | 29,0% | 2,62% | 9,7 |
| CD64 KHR->GGG | 6 | 2 | 1 | 164-GTYHCSGMGGGG | 32,0% | 2,89% | 10,7 |
| CD64+V172 | 7 | 1 | 0 | 164-GTYHCSGMV | 5,5% | 0,50% | 1,8 |
| CD64+V172 KHR->AAA | 10 | 1 | 0 | 164-GTYHCSGMV ___ | 3,0% | 0,27% | 1,0 |
| CD64+G171 | 9 | 2 | 0 | 164-GTYHCSGGMGKHRYTSAGISV-184 | 3,0% | 0,27% | 1,0 |
| CD64+G172 | 8 | 1 | 0 | 164-GTYHCSGMGGKHRYTSAGISV-184 | 5,5% | 0,50% | 1,8 |
| tetR | | 79 | 27 | full protein | 610% | 55,14% | 203,3 |
| CMV pp65 | | 179 | 43 | full protein | 1106% | 100% | 368,8 |

FIG. 17

EP 3 875 478 A1

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2016/040441 A1 (UNUM THERAPEUTICS [US])<br>17 March 2016 (2016-03-17)<br>* abstract *<br>* page 3, line 1 - line 7 *<br>* page 19, last line - page 20, line 1 *<br>* table 2 *<br>* page 22, line 18 - line 23 *<br>* page 24, line 10 - line 16 *<br>* page 3, line 17 - line 23 *<br>* page 3, line 24 - line 30 *<br>* page 4, line 21 - line 23 *<br>* First paragraph under Table 2;<br>page 20 *<br>* page 21, line 13 - line 16 *<br>----- | 1,9-15,<br>17-22<br>1-22 | INV.<br>C07K14/725<br>C07K14/705<br>C07K14/735 |
| X<br>Y | WO 2017/177217 A2 (UNUM THERAPEUTICS [US])<br>12 October 2017 (2017-10-12)<br>* page 2, line 28 - page 3, line 2 *<br>* page 3, line 3 - line 5 *<br>* page 10, line 6 - line 7 *<br>* table 2 *<br>* page 16, line 31 - line 32 *<br>* page 3, line 6 - line 13 *<br>* page 14, line 6 - line 32 *<br>* page 10, line 15 - line 18 *<br>* page 14, line 16 - line 32 *<br>* page 16, line 31 - page 17, line 10 *<br>* claims 46, 53, 56 *<br>* page 19, line 27 - line 29 *<br>* page 22, line 3 - line 26 *<br>* page 23, line 1 - line 25 *<br>* D. Cytoplasmic signaling domain;<br>page 24 - page 25 *<br>----- | 1,9-15,<br>17-22<br>1-22 | TECHNICAL FIELDS SEARCHED (IPC)<br><br>C07K |

-/--

The present search report has been drawn up for all claims

| Place of search | Date of completion of the search | Examiner |
|---|---|---|
| Munich | 22 April 2020 | Mundel, Christophe |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................
& : member of the same patent family, corresponding
    document

EPO FORM 1503 03.82 (P04C01)

page 1 of 3

Europäisches
Patentamt

European
Patent Office

Office européen
des brevets

**EUROPEAN SEARCH REPORT**

Application Number

EP 20 38 2160

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SARA CARATELLI ET AL: "FC[gamma] Chimeric Receptor-Engineered T Cells: Methodology, Advantages, Limitations, and Clinical Relevance", FRONTIERS IN IMMUNOLOGY, vol. 8, 27 April 2017 (2017-04-27), XP055688148, DOI: 10.3389/fimmu.2017.00457 * abstract * * page 3, right-hand column, line 15 - line 26 * * page 2, right-hand column, line 11 - line 18 * * page 4, right-hand column, line 15 - line 25 * * page 7, left-hand column, line 9 - line 13 *<br><br>-----<br><br>-/-- | 1-22 | |

| | | | TECHNICAL FIELDS SEARCHED (IPC) |
|---|---|---|---|

The present search report has been drawn up for all claims

| Place of search | Date of completion of the search | Examiner |
|---|---|---|
| Munich | 22 April 2020 | Mundel, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 38 2160

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JINGHUA LU ET AL: "Structure of Fc[gamma]RI in complex with Fc reveals the importance of glycan recognition for high-affinity IgG binding", PROCEEDINGS OF THE NATIONAL ACADEMY OF SCIENCES, vol. 112, no. 3, 5 January 2015 (2015-01-05), pages 833-838, XP055413240, US ISSN: 0027-8424, DOI: 10.1073/pnas.1418812112 * abstract * * Interface of FcgammaRI with the Lower Fc Region Is Conserved.; page 834 - page 835 * * figure 2 * * The FcgammaRI D2 Domain FG Loop Forms Unique Contacts with Fc Glycans.; page 835 - page 836 * * Mutations in the FG Loop of FcgammaRI Reduce Its IgG Binding Affinity; page 836 - page 837 * ----- | 1-22 | |
| A | ALEXANDRA KEGLER ET AL: "T cells engrafted with a UniCAR 28/z outperform UniCAR BB/z-transduced T cells in the face of regulatory T cell-mediated immunosuppression", ONCOIMMUNOLOGY, vol. 8, no. 9, 7 June 2019 (2019-06-07), page e1621676, XP055688367, DOI: 10.1080/2162402X.2019.1621676 * abstract * ----- | 1-22 | TECHNICAL FIELDS SEARCHED (IPC) |

The present search report has been drawn up for all claims

| Place of search | Date of completion of the search | Examiner |
|---|---|---|
| Munich | 22 April 2020 | Mundel, Christophe |

EPO FORM 1503 03.82 (P04C01)

page 3 of 3

## ANNEX TO THE EUROPEAN SEARCH REPORT
## ON EUROPEAN PATENT APPLICATION NO.

EP 20 38 2160

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2020

| Patent document cited in search report | | | Publication date | Patent family member(s) | | | Publication date |
|---|---|---|---|---|---|---|---|
| WO 2016040441 | A1 | | 17-03-2016 | AU | 2015315199 | A1 | 16-03-2017 |
| | | | | BR | 112017004675 | A2 | 05-12-2017 |
| | | | | CA | 2972714 | A1 | 17-03-2016 |
| | | | | CN | 107074969 | A | 18-08-2017 |
| | | | | EP | 3191507 | A1 | 19-07-2017 |
| | | | | JP | 2017527310 | A | 21-09-2017 |
| | | | | KR | 20170073593 | A | 28-06-2017 |
| | | | | SG | 10201902168P | A | 29-04-2019 |
| | | | | SG | 11201701775V | A | 27-04-2017 |
| | | | | US | 2017281682 | A1 | 05-10-2017 |
| | | | | WO | 2016040441 | A1 | 17-03-2016 |
| WO 2017177217 | A2 | | 12-10-2017 | US | 2019105348 | A1 | 11-04-2019 |
| | | | | WO | 2017177217 | A2 | 12-10-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3057986 A
- EP 3057986 B1
- WO 20171613333 A
- US 7446190 B
- US 5731168 A
- US 7695936 B

**Non-patent literature cited in the description**

- **KIYOSHI et al.** *Nature,* 2015
- **ESHHAR Z et al.** *PNAS,* 1993, vol. 90, 720-724
- **BROCKER T ; KARJALAINEN K.** *J. Exp. Med.,* 1995, vol. 181, 1653-1659
- **BROCKER T.** *Blood,* 2000, vol. 96, 1999-2001
- **SMYTH MJ et al.** *J. Leukoc. Biol.,* 1994, vol. 55, 514-522
- **KERSHAW MH et al.** *J. Leukoc. Biol.,* 1996, vol. 60, 721-728
- **TENG MWL et al.** *Human Gene Ther.,* 2006, vol. 17, 1134-1143
- **CLEMENCEAU B et al.** *Blood,* 2006, vol. 107 (12), 4669-4677
- **CARTRON G et al.** *Blood,* 2002, vol. 99, 754-758
- **ALVAREZ-VALLINA L ; HAWKINS RE.** *Eur. J. Immunol.,* 1996, vol. 26, 2304
- **FINNEY HM et al.** *J. Immunol.,* 1998, vol. 161, 2791-2797
- **HOMBACH A et al.** *J. Immunol.,* 2001, vol. 167, 6123-6131
- **HAYNES NM et al.** *J. Immunol.,* 2001, vol. 166, 182-187
- **IMAI C et al.** *Leukemia,* 2004, vol. 18, 676-684
- **GUEST RD et al.** *J. Immunother.,* 2005, vol. 28 (3), 203-211
- **QIN L et al.** *J. Hematol. Oncol.,* 2017, vol. 10 (1), 68
- **YING Z et al.** *Nat. Med.,* 2019, vol. 25, 947-953
- **RODGERS DT et al.** *PNAS,* 2016, vol. 113 (4), E459-E468
- **ASAOKA Y et al.** *Mol. Immunol.,* 2013, vol. 54, 403-407
- **JONNALAGADDA M et al.** *Mol Ther.,* 2015, vol. 23 (4), 757-768
- **WATANABE N et al.** *Oncoimmunol,* 2016, vol. 5 (12), e1253656
- **WATANABE K et al.** *J. Immunol.,* 2015, vol. 194, 911-920
- **JAGER U et al.** *Haematologica,* 2012, vol. 97 (09), 1431-1438
- **SCHNEIDER D et al.** *J. Immunother. Cancer,* 2017, vol. 5, 42
- **HAYNES NM et al.** *Blood,* 2002, vol. 100, 3155-3163
- **FINNEY HM et al.** *J. Immunol.,* 2004, vol. 172, 104-113
- **MILONE MC et al.** *Mol. Ther.,* 2009, vol. 17 (8), 1453-1464
- **LONG AH et al.** *Nat. Med.,* 2015, vol. 21 (6), 581-590
- **ZAH E et al.** *Cancer Immunol. Res.,* 2016, vol. 4 (6), 498-508
- **GRADA Z et al.** *Mol. Ther. Nucleic Acids,* 2013, vol. 2, e105
- **BRUHNS P et al.** *Blood,* 2009, vol. 113, 3716-3725
- **VAN DER POEL CE et al.** *J. Immunol.,* 2011, vol. 186, 2699-2704
- **LU J et al.** *J. Biol. Chem.,* 2011, vol. 286 (47), 40608-40613
- **HULETT MD et al.** *Eur J. Immunol.,* 1993, vol. 23, 640-645
- **LU J et al.** *PNAS,* 2015, vol. 112 (3), 833-838
- **KIYOSHI M et al.** *Nat. Commun.,* 2015, vol. 6, 6866
- Immunoglobulins: Structure and Function. **FRAZER JK ; CAPRA JD.** Fundamental Immunology. Lippincott-Raven Publishers, 1999
- **MARGULIES DH.** Fundamental Immunology. Lippincott-Raven Publishers, 1999
- **GERMAIN RN.** Fundamental Immunology. Lippincott-Raven Publishers, 1999
- **DAVIS MM ; CHIEN YH.** Fundamental Immunology. Lippincott-Raven Publishers, 1999
- **SIDNEY J et al.** *BMC Immunol.,* 2008, vol. 9, 1
- **NIELSEN M ; ANDREATTA M.** *Genome Medicine,* 2016, vol. 8, 33
- **NIELSEN M et al.** *Protein Sci,* 2003, vol. 12, 1007-1017

- **MAUDE SL et al.** *J. Clin. Oncol.,* 2016, vol. 34 (15), 3011-3011
- **MAUDE SL et al.** *Blood,* 2017, vol. 130 (1), 1319
- **PURBHOO MA et al.** *J. Immunol.,* 2006, vol. 176, 7308-7316
- **TANG Y et al.** *J. Immunol.,* 2007, vol. 179, 2815-2823
- **JONES S et al.** *Hum. Gene Ther.,* 2009, vol. 20, 630-640
- **LIZEE G et al.** *Hum. Gene Ther.,* 2003, vol. 14 (6), 497-507
- **BEERS SA et al.** *Blood,* 2010, vol. 115 (25), 5191-5201
- **HOOF I et al.** *Immunogenetics,* 2009, vol. 61, 1-13

SEQUENCE LISTING

<110> CANVAX BIOTECH SL

<120> Novel non-immunogenic chimeric antigen receptors and uses thereof

<130> AX200030EP

<160> 84

<170> BiSSAP 1.3.6

<210> 1
<211> 292
<212> PRT
<213> Homo sapiens

<220>
<223> human native CD64 extracellular domain

<400> 1
Met Trp Phe Leu Thr Thr Leu Leu Leu Trp Val Pro Val Asp Gly Gln
1               5                   10                  15
Val Asp Thr Thr Lys Ala Val Ile Thr Leu Gln Pro Pro Trp Val Ser
            20                  25                  30
Val Phe Gln Glu Glu Thr Val Thr Leu His Cys Glu Val Leu His Leu
        35                  40                  45
Pro Gly Ser Ser Ser Thr Gln Trp Phe Leu Asn Gly Thr Ala Thr Gln
        50                  55                  60
Thr Ser Thr Pro Ser Tyr Arg Ile Thr Ser Ala Ser Val Asn Asp Ser
65                  70                  75                  80
Gly Glu Tyr Arg Cys Gln Arg Gly Leu Ser Gly Arg Ser Asp Pro Ile
                85                  90                  95
Gln Leu Glu Ile His Arg Gly Trp Leu Leu Leu Gln Val Ser Ser Arg
            100                 105                 110
Val Phe Thr Glu Gly Glu Pro Leu Ala Leu Arg Cys His Ala Trp Lys
        115                 120                 125
Asp Lys Leu Val Tyr Asn Val Leu Tyr Tyr Arg Asn Gly Lys Ala Phe
        130                 135                 140
Lys Phe Phe His Trp Asn Ser Asn Leu Thr Ile Leu Lys Thr Asn Ile
145                 150                 155                 160
Ser His Asn Gly Thr Tyr His Cys Ser Gly Met Gly Lys His Arg Tyr
                165                 170                 175
Thr Ser Ala Gly Ile Ser Val Thr Val Lys Glu Leu Phe Pro Ala Pro
            180                 185                 190
Val Leu Asn Ala Ser Val Thr Ser Pro Leu Leu Glu Gly Asn Leu Val
        195                 200                 205
Thr Leu Ser Cys Glu Thr Lys Leu Leu Leu Gln Arg Pro Gly Leu Gln
        210                 215                 220
Leu Tyr Phe Ser Phe Tyr Met Gly Ser Lys Thr Leu Arg Gly Arg Asn
225                 230                 235                 240
Thr Ser Ser Glu Tyr Gln Ile Leu Thr Ala Arg Arg Glu Asp Ser Gly
                245                 250                 255
Leu Tyr Trp Cys Glu Ala Ala Thr Glu Asp Gly Asn Val Leu Lys Arg
            260                 265                 270
Ser Pro Glu Leu Glu Leu Gln Val Leu Gly Leu Gln Leu Pro Thr Pro
        275                 280                 285
Val Trp Phe His
        290

<210> 2
<211> 20

```
<212> PRT
<213> Homo sapiens


<220>
<223> PARTIAL SEQUENCE OF CD64 MUTATED IN FG LOOP REGION: CD64 R175A


<400> 2
Gly Thr Tyr His Cys Ser Gly Met Gly Lys His Ala Tyr Thr Ser Ala
1               5                   10                  15
Gly Ile Ser Val
            20


<210> 3
<211> 20
<212> PRT
<213> Homo sapiens


<220>
<223> PARTIAL SEQUENCE OF CD64 MUTATED IN FG LOOP REGION: CD64 H174E


<400> 3
Gly Thr Tyr His Cys Ser Gly Met Gly Lys Glu Arg Tyr Thr Ser Ala
1               5                   10                  15
Gly Ile Ser Val
            20


<210> 4
<211> 20
<212> PRT
<213> Homo sapiens


<220>
<223> PARTIAL SEQUENCE OF CD64 MUTATED IN FG LOOP REGION: CD64 R175G


<400> 4
Gly Thr Tyr His Cys Ser Gly Met Gly Lys His Gly Tyr Thr Ser Ala
1               5                   10                  15
Gly Ile Ser Val
            20


<210> 5
<211> 20
<212> PRT
<213> Homo sapiens


<220>
<223> PARTIAL SEQUENCE OF CD64 MUTATED IN FG LOOP REGION: CD64 KHR
        changed to AAA (KHR->AAA)


<400> 5
Gly Thr Tyr His Cys Ser Gly Met Gly Ala Ala Ala Tyr Thr Ser Ala
1               5                   10                  15
Gly Ile Ser Val
            20


<210> 6
<211> 20
<212> PRT
<213> Homo sapiens
```

<220>
<223> PARTIAL SEQUENCE OF CD64 MUTATED IN FG LOOP REGION: CD64 KHR
        changed to GGG (KHR->GGG)

<400> 6
Gly Thr Tyr His Cys Ser Gly Met Gly Gly Gly Gly Tyr Thr Ser Ala
1               5               10              15
Gly Ile Ser Val
            20

<210> 7
<211> 21
<212> PRT
<213> Homo sapiens


<220>
<223> PARTIAL SEQUENCE OF CD64 MUTATED IN FG LOOP REGION: CD64 with
        Valine inserted after Methionine at 171 (CD64+V172)

<400> 7
Gly Thr Tyr His Cys Ser Gly Met Val Gly Lys His Arg Tyr Thr Ser
1               5               10              15
Ala Gly Ile Ser Val
            20

<210> 8
<211> 21
<212> PRT
<213> Homo sapiens


<220>
<223> PARTIAL SEQUENCE OF CD64 MUTATED IN FG LOOP REGION: CD64 with
        Glycine inserted after Methionine at 171 (CD64+G172)

<400> 8
Gly Thr Tyr His Cys Ser Gly Met Gly Gly Lys His Arg Tyr Thr Ser
1               5               10              15
Ala Gly Ile Ser Val
            20

<210> 9
<211> 21
<212> PRT
<213> Homo sapiens


<220>
<223> PARTIAL SEQUENCE OF CD64 MUTATED IN FG LOOP REGION: CD64 with
        Glycine inserted after Glycine at 170 (CD64+G171)

<400> 9
Gly Thr Tyr His Cys Ser Gly Gly Met Gly Lys His Arg Tyr Thr Ser
1               5               10              15
Ala Gly Ile Ser Val
            20

<210> 10
<211> 21
<212> PRT

<213> Homo sapiens


<220>
<223> PARTIAL SEQUENCE OF CD64 MUTATED IN FG LOOP REGION: CD64 with
      Valine inserted after Methionine at 171 and KHR changed to AAA
      (CD64+V172 KHR->GGG)

<400> 10
Gly Thr Tyr His Cys Ser Gly Met Val Gly Gly Gly Gly Tyr Thr Ser
1               5                   10                  15
Ala Gly Ile Ser Val
                20


<210> 11
<211> 20
<212> PRT
<213> Homo sapiens


<220>
<223> PARTIAL SEQUENCE OF CD64 MUTATED IN HYDROPHOBIC POCKET: CD64
      K130Y

<400> 11
Ala Leu Arg Cys His Ala Trp Lys Asp Tyr Leu Val Tyr Asn Val Leu
1               5                   10                  15
Tyr Tyr Arg Asn
                20


<210> 12
<211> 20
<212> PRT
<213> Homo sapiens


<220>
<223> PARTIAL SEQUENCE OF CD64 MUTATED IN HYDROPHOBIC POCKET: CD64
      K130F

<400> 12
Ala Leu Arg Cys His Ala Trp Lys Asp Phe Leu Val Tyr Asn Val Leu
1               5                   10                  15
Tyr Tyr Arg Asn
                20


<210> 13
<211> 20
<212> PRT
<213> Homo sapiens


<220>
<223> PARTIAL SEQUENCE OF CD64 MUTATED IN HYDROPHOBIC POCKET: CD64
      K130F

<400> 13
Ala Leu Arg Cys His Ala Trp Lys Asp Trp Leu Val Tyr Asn Val Leu
1               5                   10                  15
Tyr Tyr Arg Asn
                20


<210> 14

<211> 20
<212> PRT
<213> Homo sapiens

<220>
<223> PARTIAL SEQUENCE OF CD64 MUTATED IN HYDROPHOBIC POCKET: CD64 K130A

<400> 14
Ala Leu Arg Cys His Ala Trp Lys Asp Ala Leu Val Tyr Asn Val Leu
1               5                   10                  15
Tyr Tyr Arg Asn
            20

<210> 15
<211> 20
<212> PRT
<213> Homo sapiens

<220>
<223> PARTIAL SEQUENCE OF CD64 MUTATED IN HYDROPHOBIC POCKET: CD64 K130S

<400> 15
Ala Leu Arg Cys His Ala Trp Lys Asp Ser Leu Val Tyr Asn Val Leu
1               5                   10                  15
Tyr Tyr Arg Asn
            20

<210> 16
<211> 20
<212> PRT
<213> Homo sapiens

<220>
<223> PARTIAL SEQUENCE OF CD64 MUTATED IN HYDROPHOBIC POCKET: CD64 K130G

<400> 16
Ala Leu Arg Cys His Ala Trp Lys Asp Gly Leu Val Tyr Asn Val Leu
1               5                   10                  15
Tyr Tyr Arg Asn
            20

<210> 17
<211> 21
<212> PRT
<213> Homo sapiens

<220>
<223> SEQUENCE OF CD8 ALPHA TRANSMEMBRANE REGION

<400> 17
Ile Tyr Ile Trp Ala Pro Leu Ala Gly Thr Cys Gly Val Leu Leu Leu
1               5                   10                  15
Ser Leu Val Ile Thr
                20

<210> 18

<211> 27
<212> PRT
<213> Homo sapiens

<220>
<223> SEQUENCE OF CD8 ALPHA TRANSMEMBRANE REGION WITH PALMITOYLATION
      SEQUENCE (LYC)

<400> 18
Ile Tyr Ile Trp Ala Pro Leu Ala Gly Thr Cys Gly Val Leu Leu Leu
1               5                   10                  15
Ser Leu Val Ile Thr Leu Tyr Cys Asn His Arg
            20                  25

<210> 19
<211> 41
<212> PRT
<213> Homo sapiens

<220>
<223> SEQUENCE OF HUMAN CD28 INTRACELLULAR CO-STIMULATION DOMAIN WITH
      DILEUCINE INTERNALIZATION MOTIF CHANGED TO DIGLYCINE

<400> 19
Arg Ser Lys Arg Ser Arg Gly Gly His Ser Asp Tyr Met Asn Met Thr
1               5                   10                  15
Pro Arg Arg Pro Gly Pro Thr Arg Lys His Tyr Gln Pro Tyr Ala Pro
            20                  25                  30
Pro Arg Asp Phe Ala Ala Tyr Arg Ser
            35                  40

<210> 20
<211> 156
<212> PRT
<213> Homo sapiens

<220>
<223> SEQUENCE OF HUMAN CD28 INTRACELLULAR CO-STIMULATION DOMAIN WITH
      DILEUCINE INTERNALIZATION MOTIF CHANGED TO DIGLYCINE FUSED USING
      A DIGLYCINE LINKER TO FULL CD3 ZETA INTRACELLULAR STIMULATION
      DOMAIN - (CD28 con CD3z complete)

<400> 20
Arg Ser Lys Arg Ser Arg Gly Gly His Ser Asp Tyr Met Asn Met Thr
1               5                   10                  15
Pro Arg Arg Pro Gly Pro Thr Arg Lys His Tyr Gln Pro Tyr Ala Pro
            20                  25                  30
Pro Arg Asp Phe Ala Ala Tyr Arg Ser Gly Gly Arg Val Lys Phe Ser
            35                  40                  45
Arg Ser Ala Asp Ala Pro Ala Tyr Gln Gln Gly Gln Asn Gln Leu Tyr
      50                  55                  60
Asn Glu Leu Asn Leu Gly Arg Arg Glu Glu Tyr Asp Val Leu Asp Lys
65                  70                  75                  80
Arg Arg Gly Arg Asp Pro Glu Met Gly Gly Lys Pro Gln Arg Arg Lys
                  85                  90                  95
Asn Pro Gln Glu Gly Leu Tyr Asn Glu Leu Gln Lys Asp Lys Met Ala
            100                 105                 110
Glu Ala Tyr Ser Glu Ile Gly Met Lys Gly Glu Arg Arg Arg Gly Lys
            115                 120                 125
Gly His Asp Gly Leu Tyr Gln Gly Leu Ser Thr Ala Thr Lys Asp Thr

```
                    130                   135                      140
         Tyr Asp Ala Leu His Met Gln Ala Leu Pro Pro Arg
         145                   150                      155


<210> 21
<211> 124
<212> PRT
<213> Homo sapiens


<220>
<223> SEQUENCE OF HUMAN CD28 INTRACELLULAR CO-STIMULATION DOMAIN WITH
      DILEUCINE INTERNALIZATION MOTIF CHANGED TO DIGLYCINE FUSED USING
      A DIGLYCINE LINKER TO TRUNCATED CD3 ZETA INTRACELLULAR
      STIMULATION DOMAIN COMPRISING TWO ITAMS - (CD28 con CD3z 2ITAMs)


<400> 21
Arg Ser Lys Arg Ser Arg Gly Gly His Ser Asp Tyr Met Asn Met Thr
1                   5                   10                      15
Pro Arg Arg Pro Gly Pro Thr Arg Lys His Tyr Gln Pro Tyr Ala Pro
            20                  25                  30
Pro Arg Asp Phe Ala Ala Tyr Arg Ser Gly Gly Arg Val Lys Phe Ser
            35                  40                  45
Arg Ser Ala Asp Ala Pro Ala Tyr Gln Gln Gly Gln Asn Gln Leu Tyr
        50                  55                  60
Asn Glu Leu Asn Leu Gly Arg Arg Glu Glu Tyr Asp Val Leu Asp Lys
65                  70                  75                      80
Arg Arg Gly Arg Asp Pro Glu Met Gly Gly Lys Pro Gln Arg Arg Lys
            85                  90                  95
Asn Pro Gln Glu Gly Leu Tyr Asn Glu Leu Gln Lys Asp Lys Met Ala
            100                 105                 110
Glu Ala Tyr Ser Glu Ile Gly Met Lys Gly Glu Arg
            115                 120


<210> 22
<211> 200
<212> PRT
<213> Homo sapiens


<220>
<223> HUMAN CD28 INTRACELLULAR CO-STIMULATION DOMAIN WITH DILEUCINE INT
      ERNALIZATION MOTIF CHANGED TO DIGLYCINE FUSED USING A TRIGLYCINE
      LINKER TO FULL INTRACELLULAR CO-STIMULATION DOMAIN OF HUMAN 4-1BB
      FUSED USING A DIGLYCINE LINKER TO FULL CD3 ZETA INTRACELLULAR


<400> 22
Arg Ser Lys Arg Ser Arg Gly Gly His Ser Asp Tyr Met Asn Met Thr
1                   5                   10                      15
Pro Arg Arg Pro Gly Pro Thr Arg Lys His Tyr Gln Pro Tyr Ala Pro
            20                  25                  30
Pro Arg Asp Phe Ala Ala Tyr Arg Gly Gly Gly Lys Arg Gly Arg Lys
            35                  40                  45
Lys Gly Gly Tyr Ile Phe Lys Gln Pro Phe Met Arg Pro Val Gln Thr
        50                  55                  60
Thr Gln Glu Glu Asp Gly Cys Ser Cys Arg Phe Pro Glu Glu Glu Glu
65                  70                  75                      80
Gly Gly Cys Glu Leu Gly Gly Arg Val Lys Phe Ser Arg Ser Ala Asp
            85                  90                  95
Ala Pro Ala Tyr Gln Gln Gly Gln Asn Gln Leu Tyr Asn Glu Leu Asn
            100                 105                 110
Leu Gly Arg Arg Glu Glu Tyr Asp Val Leu Asp Lys Arg Arg Gly Arg
            115                 120                 125
```

```
Asp Pro Glu Met Gly Gly Lys Pro Gln Arg Arg Lys Asn Pro Gln Glu
    130             135                 140
Gly Leu Tyr Asn Glu Leu Gln Lys Asp Lys Met Ala Glu Ala Tyr Ser
145             150                 155                 160
Glu Ile Gly Met Lys Gly Glu Arg Arg Arg Gly Lys Gly His Asp Gly
            165                 170                 175
Leu Tyr Gln Gly Leu Ser Thr Ala Thr Lys Asp Thr Tyr Asp Ala Leu
            180                 185                 190
His Met Gln Ala Leu Pro Pro Arg
            195                 200
```

```
<210> 23
<211> 195
<212> PRT
<213> Homo sapiens


<220>
<223> SEQUENCE OF HUMAN CD28 INTRACELLULAR CO-STIMULATION DOMAIN WITH
      DILEUCINE INTERNALIZATION MOTIF CHANGED TO DIGLYCINE FUSED USING
      A TRIGLYCINE LINKER TO FULL INTRACELLULAR CO-STIMULATION DOMAIN
      OF HUMAN OX40 FUSED USING A DIGLYCINE LINKER TO FULL CD3 ZETA


<400> 23
Arg Ser Lys Arg Ser Arg Gly Gly His Ser Asp Tyr Met Asn Met Thr
1               5               10                  15
Pro Arg Arg Pro Gly Pro Thr Arg Lys His Tyr Gln Pro Tyr Ala Pro
            20                  25                  30
Pro Arg Asp Phe Ala Ala Tyr Arg Gly Gly Gly Arg Arg Asp Gln Arg
            35                  40                  45
Leu Pro Pro Asp Ala His Lys Pro Pro Gly Gly Gly Ser Phe Arg Thr
    50                  55                  60
Pro Ile Gln Glu Glu Gln Ala Asp Ala His Ser Thr Leu Ala Lys Ile
65                  70                  75                  80
Gly Gly Arg Val Lys Phe Ser Arg Ser Ala Asp Ala Pro Ala Tyr Gln
            85                  90                  95
Gln Gly Gln Asn Gln Leu Tyr Asn Glu Leu Asn Leu Gly Arg Arg Glu
            100                 105                 110
Glu Tyr Asp Val Leu Asp Lys Arg Arg Gly Arg Asp Pro Glu Met Gly
            115                 120                 125
Gly Lys Pro Gln Arg Arg Lys Asn Pro Gln Glu Gly Leu Tyr Asn Glu
            130                 135                 140
Leu Gln Lys Asp Lys Met Ala Glu Ala Tyr Ser Glu Ile Gly Met Lys
145                 150                 155                 160
Gly Glu Arg Arg Arg Gly Lys Gly His Asp Gly Leu Tyr Gln Gly Leu
            165                 170                 175
Ser Thr Ala Thr Lys Asp Thr Tyr Asp Ala Leu His Met Gln Ala Leu
            180                 185                 190
Pro Pro Arg
            195


<210> 24
<211> 204
<212> PRT
<213> Homo sapiens


<220>
<223> SEQUENCE OF HUMAN CD28 INTRACELLULAR CO-STIMULATION DOMAIN WITH
      DILEUCINE INTERNALIZATION MOTIF CHANGED TO DIGLYCINE FUSED USING
      A TRIGLYCINE LINKER TO FULL INTRACELLULAR CO-STIMULATION DOMAIN
      OF HUMAN ICOS FUSED USING A DIGLYCINE LINKER TO FULL CD3 ZETA
```

<400> 24

```
Cys Asp Ile Cys Ser Cys Asp Glx Arg Ser Lys Arg Ser Arg Gly Gly
1               5                   10                  15
His Ser Asp Tyr Met Asn Met Thr Pro Arg Arg Pro Gly Pro Thr Arg
            20                  25                  30
Lys His Tyr Gln Pro Tyr Ala Pro Pro Arg Asp Phe Ala Ala Tyr Arg
            35                  40                  45
Gly Gly Gly Cys Trp Leu Thr Lys Lys Lys Tyr Ser Ser Ser Val His
        50                  55                  60
Asp Pro Asn Gly Glu Tyr Met Phe Met Arg Ala Val Asn Thr Ala Lys
65                  70                  75                  80
Lys Ser Arg Leu Thr Asp Val Thr Leu Gly Gly Arg Val Lys Phe Ser
                85                  90                  95
Arg Ser Ala Asp Ala Pro Ala Tyr Gln Gln Gly Gln Asn Gln Leu Tyr
            100                 105                 110
Asn Glu Leu Asn Leu Gly Arg Arg Glu Glu Tyr Asp Val Leu Asp Lys
            115                 120                 125
Arg Arg Gly Arg Asp Pro Glu Met Gly Gly Lys Pro Gln Arg Arg Lys
        130                 135                 140
Asn Pro Gln Glu Gly Leu Tyr Asn Glu Leu Gln Lys Asp Lys Met Ala
145                 150                 155                 160
Glu Ala Tyr Ser Glu Ile Gly Met Lys Gly Glu Arg Arg Arg Gly Lys
                165                 170                 175
Gly His Asp Gly Leu Tyr Gln Gly Leu Ser Thr Ala Thr Lys Asp Thr
                180                 185                 190
Tyr Asp Ala Leu His Met Gln Ala Leu Pro Pro Arg
            195                 200
```

<210> 25
<211> 168
<212> PRT
<213> Homo sapiens

<220>
<223> SEQUENCE OF HUMAN CD28 INTRACELLULAR CO-STIMULATION DOMAIN WITH D
      ILEUCINE INTERNALIZATION MOTIF CHANGED TO DIGLYCINE FUSED USING A
      TRIGLYCINE LINKER TO FULL INTRACELLULAR CO-STIMULATION DOMAIN OF
      HUMAN 4-1BB FUSED USING A DIGLYCINE LINKER TO TRUNCATED CD3 ZETA

<400> 25

```
Arg Ser Lys Arg Ser Arg Gly Gly His Ser Asp Tyr Met Asn Met Thr
1               5                   10                  15
Pro Arg Arg Pro Gly Pro Thr Arg Lys His Tyr Gln Pro Tyr Ala Pro
            20                  25                  30
Pro Arg Asp Phe Ala Ala Tyr Arg Gly Gly Gly Lys Arg Gly Arg Lys
            35                  40                  45
Lys Gly Gly Tyr Ile Phe Lys Gln Pro Phe Met Arg Pro Val Gln Thr
        50                  55                  60
Thr Gln Glu Glu Asp Gly Cys Ser Cys Arg Phe Pro Glu Glu Glu Glu
65                  70                  75                  80
Gly Gly Cys Glu Leu Gly Gly Arg Val Lys Phe Ser Arg Ser Ala Asp
                85                  90                  95
Ala Pro Ala Tyr Gln Gln Gly Gln Asn Gln Leu Tyr Asn Glu Leu Asn
            100                 105                 110
Leu Gly Arg Arg Glu Glu Tyr Asp Val Leu Asp Lys Arg Arg Gly Arg
            115                 120                 125
Asp Pro Glu Met Gly Gly Lys Pro Gln Arg Arg Lys Asn Pro Gln Glu
        130                 135                 140
Gly Leu Tyr Asn Glu Leu Gln Lys Asp Lys Met Ala Glu Ala Tyr Ser
145                 150                 155                 160
Glu Ile Gly Met Lys Gly Glu Arg
                165
```

<210> 26
<211> 163
<212> PRT
<213> Homo sapiens

<220>
<223> SEQUENCE OF HUMAN CD28 INTRACELLULAR CO-STIMULATION DOMAIN WITH
DILEUCINE INTERNALIZATION MOTIF CHANGED TO DIGLYCINE FUSED USING
A TRIGLYCINE

<400> 26
Arg Ser Lys Arg Ser Arg Gly Gly His Ser Asp Tyr Met Asn Met Thr
1               5                   10                  15
Pro Arg Arg Pro Gly Pro Thr Arg Lys His Tyr Gln Pro Tyr Ala Pro
            20                  25                  30
Pro Arg Asp Phe Ala Ala Tyr Arg Gly Gly Gly Arg Arg Asp Gln Arg
            35                  40                  45
Leu Pro Pro Asp Ala His Lys Pro Pro Gly Gly Gly Ser Phe Arg Thr
        50                  55                  60
Pro Ile Gln Glu Glu Gln Ala Asp Ala His Ser Thr Leu Ala Lys Ile
65                  70                  75                  80
Gly Gly Arg Val Lys Phe Ser Arg Ser Ala Asp Ala Pro Ala Tyr Gln
                85                  90                  95
Gln Gly Gln Asn Gln Leu Tyr Asn Glu Leu Asn Leu Gly Arg Arg Glu
            100                 105                 110
Glu Tyr Asp Val Leu Asp Lys Arg Arg Gly Arg Asp Pro Glu Met Gly
            115                 120                 125
Gly Lys Pro Gln Arg Arg Lys Asn Pro Gln Glu Gly Leu Tyr Asn Glu
        130                 135                 140
Leu Gln Lys Asp Lys Met Ala Glu Ala Tyr Ser Glu Ile Gly Met Lys
145                 150                 155                 160
Gly Glu Arg

<210> 27
<211> 164
<212> PRT
<213> Homo sapiens

<220>
<223> SEQUENCE OF HUMAN CD28 INTRACELLULAR CO-STIMULATION DOMAIN WITH D
ILEUCINE INTERNALIZATION MOTIF CHANGED TO DIGLYCINE FUSED USING A
TRIGLYCINE LINKER TO FULL INTRACELLULAR CO-STIMULATION DOMAIN OF
HUMAN ICOS FUSED USING A DIGLYCINE LINKER TO TRUNCATED CD3 ZETA

<400> 27
Arg Ser Lys Arg Ser Arg Gly Gly His Ser Asp Tyr Met Asn Met Thr
1               5                   10                  15
Pro Arg Arg Pro Gly Pro Thr Arg Lys His Tyr Gln Pro Tyr Ala Pro
            20                  25                  30
Pro Arg Asp Phe Ala Ala Tyr Arg Gly Gly Gly Cys Trp Leu Thr Lys
            35                  40                  45
Lys Lys Tyr Ser Ser Ser Val His Asp Pro Asn Gly Glu Tyr Met Phe
        50                  55                  60
Met Arg Ala Val Asn Thr Ala Lys Lys Ser Arg Leu Thr Asp Val Thr
65                  70                  75                  80
Leu Gly Gly Arg Val Lys Phe Ser Arg Ser Ala Asp Ala Pro Ala Tyr
                85                  90                  95
Gln Gln Gly Gln Asn Gln Leu Tyr Asn Glu Leu Asn Leu Gly Arg Arg
            100                 105                 110

```
Glu Glu Tyr Asp Val Leu Asp Lys Arg Arg Gly Arg Asp Pro Glu Met
        115                 120                 125
Gly Gly Lys Pro Gln Arg Arg Lys Asn Pro Gln Glu Gly Leu Tyr Asn
        130                 135                 140
Glu Leu Gln Lys Asp Lys Met Ala Glu Ala Tyr Ser Glu Ile Gly Met
145                 150                 155                 160
Lys Gly Glu Arg
```

```
<210> 28
<211> 113
<212> PRT
<213> Homo sapiens


<220>
<223> SEQUENCE OF HUMAN CD28 INTRACELLULAR CO-STIMULATION DOMAIN WITH
      DILEUCINE INTERNALIZATION MOTIF CHANGED TO DIGLYCINE FUSED USING
      A TRIGLYCINE LINKER TO FULL INTRACELLULAR CO-STIMULATION DOMAIN
      OF HUMAN ICOS FUSED USING A DIGLYCINE LINKER TO TRUNCATED

<400> 28
Arg Val Lys Phe Ser Arg Ser Ala Asp Ala Pro Ala Tyr Gln Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Gln Arg Arg Lys Asn Pro Gln Glu Gly Leu Tyr Asn Glu Leu Gln
    50                  55                  60
Lys Asp Lys Met Ala Glu Ala Tyr Ser Glu Ile Gly Met Lys Gly Glu
65                  70                  75                  80
Arg Arg Arg Gly Lys Gly His Asp Gly Leu Tyr Gln Gly Leu Ser Thr
                85                  90                  95
Ala Thr Lys Asp Thr Tyr Asp Ala Leu His Met Gln Ala Leu Pro Pro
            100                 105                 110
Arg


<210> 29
<211> 81
<212> PRT
<213> Homo sapiens


<220>
<223> SEQUENCE OF TRUNCATED HUMAN CD3 ZETA INTRACELLULAR STIMULATION
      DOMAIN COMPRISING TWO ITAMS

<400> 29
Arg Val Lys Phe Ser Arg Ser Ala Asp Ala Pro Ala Tyr Gln Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Gln Arg Arg Lys Asn Pro Gln Glu Gly Leu Tyr Asn Glu Leu Gln
    50                  55                  60
Lys Asp Lys Met Ala Glu Ala Tyr Ser Glu Ile Gly Met Lys Gly Glu
65                  70                  75                  80
Arg
```

<210> 30
<211> 157
<212> PRT
<213> Homo sapiens

<220>
<223> SEQUENCE OF FULL INTRACELLULAR CO-STIMULATION DOMAIN OF HUMAN
      4-1BB FUSED USING A DIGLYCINE LINKER TO FULL CD3 ZETA
      INTRACELLULAR STIMULATION DOMAIN

<400> 30

```
Lys Arg Gly Arg Lys Lys Gly Gly Tyr Ile Phe Lys Gln Pro Phe Met
1               5                   10                  15
Arg Pro Val Gln Thr Thr Gln Glu Glu Asp Gly Cys Ser Cys Arg Phe
            20                  25                  30
Pro Glu Glu Glu Glu Gly Gly Cys Glu Leu Gly Gly Arg Val Lys Phe
        35                  40                  45
Ser Arg Ser Ala Asp Ala Pro Ala Tyr Gln Gln Gly Gln Asn Gln Leu
        50                  55                  60
Tyr Asn Glu Leu Asn Leu Gly Arg Arg Glu Glu Tyr Asp Val Leu Asp
65                  70                  75                  80
Lys Arg Arg Gly Arg Asp Pro Glu Met Gly Gly Lys Pro Gln Arg Arg
                85                  90                  95
Lys Asn Pro Gln Glu Gly Leu Tyr Asn Glu Leu Gln Lys Asp Lys Met
            100                 105                 110
Ala Glu Ala Tyr Ser Glu Ile Gly Met Lys Gly Glu Arg Arg Arg Gly
            115                 120                 125
Lys Gly His Asp Gly Leu Tyr Gln Gly Leu Ser Thr Ala Thr Lys Asp
        130                 135                 140
Thr Tyr Asp Ala Leu His Met Gln Ala Leu Pro Pro Arg
145                 150                 155
```

<210> 31
<211> 489
<212> DNA
<213> Homo sapiens

<220>
<223> NUCLEOTIDE SEQUENCE OF TCRVB6.7 PROMOTER THAT MAY BE USED FOR
      EXPRESSION OF CAR-T

<400> 31

```
gagggaaccc atgtgatggg acaatcccgt tgggcacatg tgagtggggg aatggaggag      60

gctggggcat gaatggggat ggcagagggg accctgactt ggaggaaaga caatgagctc     120

atcccatggt gcctggtgtt gggggcactg ttggcacatc ctacagaacg tgctcagcag     180

acagaggagc ggctgtggga tgagaaggtg aactcggaga tgcagcgtga ggcctccaag     240

tccagacagc atgggagccc aaagcgatgt cccatgcaat aacgttgttt acaaggagct     300

tataaatatt taaagtagtc acccaagtgt ggtctaatat aaatcctgtg ttcctgaggt     360

catgcagatt gagagaggaa gtgatgtcac tgtgggaact tccgtgtaag gacggggcgt     420

ccctcctcct ctgctcctgc tcacagtgat cctgatctgg taaagctccc atcctgccct     480

gaccctgcc                                                            489
```

<210> 32
<211> 20
<212> PRT
<213> Homo sapiens


<220>
<223> Epitopes of mutated human CD64


<400> 32
Gly Thr Tyr His Cys Ser Gly Met Gly Lys His Arg Tyr Thr Ser Ala
1               5                   10                  15
Gly Ile Ser Val
            20

<210> 33
<211> 9
<212> PRT
<213> Homo sapiens


<220>
<223> Epitopes of mutated human CD64


<400> 33
Cys Ser Gly Met Gly Lys His Arg Tyr
1               5

<210> 34
<211> 9
<212> PRT
<213> Homo sapiens


<220>
<223> Epitopes of mutated human CD64


<400> 34
Arg Tyr Thr Ser Ala Gly Ile Ser Val
1               5

<210> 35
<211> 9
<212> PRT
<213> Homo sapiens


<220>
<223> Epitopes of mutated human CD64


<400> 35
Met Gly Lys His Arg Tyr Thr Ser Ala
1               5

<210> 36
<211> 9
<212> PRT
<213> Homo sapiens


<220>
<223> Epitopes of mutated human CD64

```
<400> 36
Met Val Gly Lys His Arg Tyr Thr Ser
1               5


<210> 37
<211> 9
<212> PRT
<213> Homo sapiens


<220>
<223> Epitopes of mutated human CD64

<400> 37
Val Gly Lys His Arg Tyr Thr Ser Ala
1               5

<210> 38
<211> 9
<212> PRT
<213> Homo sapiens


<220>
<223> Epitopes of mutated human CD64

<400> 38
Met Gly Gly Lys His Arg Tyr Thr Ser
1               5

<210> 39
<211> 9
<212> PRT
<213> Homo sapiens


<220>
<223> Epitopes of mutated human CD64

<400> 39
Gly Gly Lys His Arg Tyr Thr Ser Ala
1               5

<210> 40
<211> 9
<212> PRT
<213> Homo sapiens


<220>
<223> Epitopes of mutated human CD64

<400> 40
Gly Thr Tyr His Cys Ser Gly Gly Met
1               5

<210> 41
<211> 9
<212> PRT
<213> Homo sapiens


<220>
```

<223> Epitopes of mutated human CD64

<400> 41
Tyr His Cys Ser Gly Gly Met Gly Lys
1               5

<210> 42
<211> 9
<212> PRT
<213> Homo sapiens


<220>
<223> Epitopes of mutated human CD64

<400> 42
Tyr His Cys Ser Gly Met Val Gly Gly
1               5

<210> 43
<211> 9
<212> PRT
<213> Homo sapiens


<220>
<223> Epitopes of mutated human CD64

<400> 43
Ser Gly Met Val Gly Gly Gly Gly Tyr
1               5

<210> 44
<211> 9
<212> PRT
<213> Homo sapiens


<220>
<223> Epitopes of mutated human CD64

<400> 44
Cys Ser Gly Met Gly Lys Glu Arg Tyr
1               5

<210> 45
<211> 9
<212> PRT
<213> Homo sapiens


<220>
<223> Epitopes of mutated human CD64

<400> 45
Met Gly Lys Glu Arg Tyr Thr Ser Ala
1               5

<210> 46
<211> 9
<212> PRT
<213> Homo sapiens

<220>
<223> Epitopes of mutated human CD64

<400> 46
Lys Glu Arg Tyr Thr Ser Ala Gly Ile
1               5


<210> 47
<211> 9
<212> PRT
<213> Homo sapiens


<220>
<223> Epitopes of mutated human CD64

<400> 47
Cys Ser Gly Met Gly Lys His Ala Tyr
1               5

<210> 48
<211> 9
<212> PRT
<213> Homo sapiens


<220>
<223> Epitopes of mutated human CD64

<400> 48
Met Gly Lys His Ala Tyr Thr Ser Ala
1               5

<210> 49
<211> 9
<212> PRT
<213> Homo sapiens


<220>
<223> Epitopes of mutated human CD64

<400> 49
Lys His Ala Tyr Thr Ser Ala Gly Ile
1               5

<210> 50
<211> 9
<212> PRT
<213> Homo sapiens


<220>
<223> Epitopes of mutated human CD64

<400> 50
Thr Ser Ala Gly Ile Ser Val Thr Val
1               5

<210> 51
<211> 9
<212> PRT

<213> Homo sapiens


<220>
<223> Epitopes of mutated human CD64

<400> 51
Cys Ser Gly Met Gly Ala Ala Ala Tyr
1               5


<210> 52
<211> 9
<212> PRT
<213> Homo sapiens


<220>
<223> Epitopes of mutated human CD64

<400> 52
Tyr His Cys Ser Gly Met Gly Ala Ala
1               5


<210> 53
<211> 9
<212> PRT
<213> Homo sapiens


<220>
<223> Epitopes of mutated human CD64

<400> 53
Cys Ser Gly Met Gly Gly Gly Gly Tyr
1               5


<210> 54
<211> 9
<212> PRT
<213> Homo sapiens


<220>
<223> Epitopes of mutated human CD64

<400> 54
Thr Ser Ala Gly Ile Ser Val Thr Val
1               5


<210> 55
<211> 20
<212> PRT
<213> Homo sapiens


<220>
<223> Epitopes of mutated human CD64

<400> 55
Ala Leu Arg Cys His Ala Trp Lys Asp Lys Leu Val Tyr Asn Val Leu
1               5                   10                  15
Tyr Tyr Arg Asn
            20

```
<210> 56
<211> 9
<212> PRT
<213> Homo sapiens


<220>
<223> Epitopes of mutated human CD64

<400> 56
Lys Leu Val Tyr Asn Val Leu Tyr Tyr
1                   5

<210> 57
<211> 9
<212> PRT
<213> Homo sapiens


<220>
<223> Epitopes of mutated human CD64

<400> 57
His Ala Trp Lys Asp Ser Leu Val Tyr
1                   5

<210> 58
<211> 9
<212> PRT
<213> Homo sapiens


<220>
<223> Epitopes of mutated human CD64

<400> 58
Asp Ser Leu Val Tyr Asn Val Leu Tyr
1                   5

<210> 59
<211> 9
<212> PRT
<213> Homo sapiens


<220>
<223> Epitopes of mutated human CD64

<400> 59
Ser Leu Val Tyr Asn Val Leu Tyr Tyr
1                   5

<210> 60
<211> 9
<212> PRT
<213> Homo sapiens


<220>
<223> Epitopes of mutated human CD64

<400> 60
```

```
Trp Lys Asp Ser Leu Val Tyr Asn Val
1               5


<210> 61
<211> 9
<212> PRT
<213> Homo sapiens


<220>
<223> Epitopes of mutated human CD64

<400> 61
Gly Leu Val Tyr Asn Val Leu Tyr Tyr
1               5

<210> 62
<211> 9
<212> PRT
<213> Homo sapiens


<220>
<223> Epitopes of mutated human CD64

<400> 62
Trp Lys Asp Gly Leu Val Tyr Asn Val
1               5

<210> 63
<211> 9
<212> PRT
<213> Homo sapiens


<220>
<223> Epitopes of mutated human CD64

<400> 63
His Ala Trp Lys Asp Gly Leu Val Tyr
1               5

<210> 64
<211> 9
<212> PRT
<213> Homo sapiens


<220>
<223> Epitopes of mutated human CD64

<400> 64
Ala Leu Val Tyr Asn Val Leu Tyr Tyr
1               5

<210> 65
<211> 9
<212> PRT
<213> Homo sapiens


<220>
<223> Epitopes of mutated human CD64
```

```
<400> 65
Asp Ala Leu Val Tyr Asn Val Leu Tyr
1               5


<210> 66
<211> 9
<212> PRT
<213> Homo sapiens


<220>
<223> Epitopes of mutated human CD64

<400> 66
Arg Cys His Ala Trp Lys Asp Ala Leu
1               5

<210> 67
<211> 9
<212> PRT
<213> Homo sapiens


<220>
<223> Epitopes of mutated human CD64

<400> 67
Trp Lys Asp Ala Leu Val Tyr Asn Val
1               5

<210> 68
<211> 9
<212> PRT
<213> Homo sapiens


<220>
<223> Epitopes of mutated human CD64

<400> 68
His Ala Trp Lys Asp Ala Leu Val Tyr
1               5

<210> 69
<211> 9
<212> PRT
<213> Homo sapiens


<220>
<223> Epitopes of mutated human CD64

<400> 69
His Ala Trp Lys Asp Trp Leu Val Tyr
1               5

<210> 70
<211> 9
<212> PRT
<213> Homo sapiens
```

```
<220>
<223> Epitopes of mutated human CD64

<400> 70
Trp Leu Val Tyr Asn Val Leu Tyr Tyr
1               5


<210> 71
<211> 9
<212> PRT
<213> Homo sapiens


<220>
<223> Epitopes of mutated human CD64

<400> 71
Cys His Ala Trp Lys Asp Trp Leu Val
1               5


<210> 72
<211> 9
<212> PRT
<213> Homo sapiens


<220>
<223> Epitopes of mutated human CD64

<400> 72
Trp Lys Asp Trp Leu Val Tyr Asn Val
1               5


<210> 73
<211> 9
<212> PRT
<213> Homo sapiens


<220>
<223> Epitopes of mutated human CD64

<400> 73
Tyr Leu Val Tyr Asn Val Leu Tyr Tyr
1               5


<210> 74
<211> 9
<212> PRT
<213> Homo sapiens


<220>
<223> Epitopes of mutated human CD64

<400> 74
His Ala Trp Lys Asp Tyr Leu Val Tyr
1               5


<210> 75
<211> 9
<212> PRT
<213> Homo sapiens
```

```
<220>
<223> Epitopes of mutated human CD64

<400> 75
Cys His Ala Trp Lys Asp Tyr Leu Val
1               5


<210> 76
<211> 9
<212> PRT
<213> Homo sapiens


<220>
<223> Epitopes of mutated human CD64

<400> 76
Trp Lys Asp Tyr Leu Val Tyr Asn Val
1               5


<210> 77
<211> 9
<212> PRT
<213> Homo sapiens


<220>
<223> Epitopes of mutated human CD64

<400> 77
Cys Ser Gly Met Gly Lys His Gly Tyr
1               5


<210> 78
<211> 9
<212> PRT
<213> Homo sapiens


<220>
<223> Epitopes of mutated human CD64

<400> 78
Met Gly Lys His Gly Tyr Thr Ser Ala
1               5


<210> 79
<211> 9
<212> PRT
<213> Homo sapiens


<220>
<223> Epitopes of mutated human CD64

<400> 79
Phe Leu Val Tyr Asn Val Leu Tyr Tyr
1               5


<210> 80
<211> 9
```

```
<212> PRT
<213> Homo sapiens


<220>
<223> Epitopes of mutated human CD64

<400> 80
Leu Val Tyr Asn Val Leu Tyr Tyr Arg
1               5

<210> 81
<211> 9
<212> PRT
<213> Homo sapiens


<220>
<223> Epitopes of mutated human CD64

<400> 81
Cys His Ala Trp Lys Asp Phe Leu Val
1               5

<210> 82
<211> 9
<212> PRT
<213> Homo sapiens


<220>
<223> Epitopes of mutated human CD64

<400> 82
Trp Lys Asp Phe Leu Val Tyr Asn Val
1               5

<210> 83
<211> 9
<212> PRT
<213> Homo sapiens


<220>
<223> Epitopes of mutated human CD64

<400> 83
His Ala Trp Lys Asp Phe Leu Val Tyr
1               5

<210> 84
<211> 6
<212> PRT
<213> Homo sapiens


<220>
<223> Epitopes of mutated human CD64

<400> 84
Met Gly Lys His Arg Tyr
1               5
```